(19) Europäisches Patentamt
European Patent Office
Office européen des brevets

(11) EP 0 618 914 B1

(12) **EUROPÄISCHE PATENTSCHRIFT**

(45) Veröffentlichungstag und Bekanntmachung des Hinweises auf die Patenterteilung:
**17.10.2001 Patentblatt 2001/42**

(21) Anmeldenummer: **92924707.0**

(22) Anmeldetag: **10.12.1992**

(51) Int Cl.[7]: **C07D 405/12**, C09K 19/58, C07D 405/14, C07D 303/22, G02F 1/13

(86) Internationale Anmeldenummer:
**PCT/EP92/02855**

(87) Internationale Veröffentlichungsnummer:
**WO 93/13093 (08.07.1993 Gazette 1993/16)**

(54) **CHIRALE OXIRANYLMETHYL-ETHER UND IHRE VERWENDUNG ALS DOTIERSTOFFE IN FLÜSSIGKRISTALLMISCHUNGEN**

OXIRANYL METHYL ETHERS EXHIBITING CHIRALITY, AND THEIR USE AS DOPANTS IN LIQUID-CRYSTAL MIXTURES

OXIRANNYLMETHYLETHERS ET LEUR UTILISATION COMME AGENTS DOPANTS DANS DES MELANGES DE CRISTAUX LIQUIDES

(84) Benannte Vertragsstaaten:
**DE FR GB**

(30) Priorität: **28.12.1991 DE 4143139**

(43) Veröffentlichungstag der Anmeldung:
**12.10.1994 Patentblatt 1994/41**

(73) Patentinhaber: **Aventis Research & Technologies GmbH & Co. KG**
**65926 Frankfurt am Main (DE)**

(72) Erfinder:
- **SCHLOSSER, Hubert**
  **D-6246 Glashütten (DE)**
- **WINGEN, Rainer**
  **D-6234 Hattersheim am Main (DE)**
- **KALTBEITZEL, Anke**
  **D-6090 Rüsselsheim (DE)**
- **MANERO, Javier**
  **D-6230 Frankfurt am Main 80 (DE)**

(74) Vertreter: **Bardehle, Heinz, Dipl.-Ing. et al**
**Patent- und Rechtsanwälte**
**Bardehle . Pagenberg . Dost . Altenburg . Geissler . Isenbruck**
**Theodor-Heuss-Anlage 12**
**68165 Mannheim (DE)**

(56) Entgegenhaltungen:

| | |
|---|---|
| **EP-A- 0 263 437** | **EP-A- 0 306 919** |
| **EP-A- 0 404 081** | **WO-A-90/11335** |
| **WO-A-92/11241** | **WO-A-92/12974** |
| **US-A- 5 061 814** | |

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 618 914 B1

## Beschreibung

Flüssigkristalle haben insbesondere im letzten Jahrzehnt Eingang in verschiedene technische Gebiete gefunden, in denen elektrooptische und Anzeigevorrichtungs-Eigenschaften gefragt sind (z.B. in Uhren-, Taschenrechner- und Schreibmaschinenanzeigen). Diese Anzeigevorrichtungen beruhen auf den dielektrischen Aussrichtungseffekten in den nematischen, cholesterischen und/oder smektischen Phasen der flüssigkristallinen Verbindungen, wobei - verursacht durch die elektrische Anisotropie - die molekulare Längsachse der Verbindungen eine bevorzugte Ausrichtung in einem angelegten elektrischen Feld einnimmt. Die üblichen Schaltzeiten bei diesen Anzeigevorrichtungen sind für viele andere potentielle Anwendungsgebiete von Flüssigkristallen zu lang. Dieser Nachteil macht sich insbesondere dann bemerkbar, wenn eine große Anzahl von Bildpunkten angesteuert werden muß. Die Herstellungskosten von Geräten, die größere Bildschirmflächen enthalten, wie z.B. von Videogeräten sind dann im allgemeinen zu hoch.

Neben den nematischen und cholesterischen Flüssigkristallen haben seit einigen wenigen Jahren in zunehmendem Maße auch optisch aktive smektische Flüssigkristall-Phasen an Bedeutung gewonnen.

Clark und Lagerwall konnten zeigen, daß die Verwendung ferroelektrischer Flüssigkristallsysteme in sehr dünnen Zellen zu optoelektrischen Schalt- oder Anzeigeelementen führt, die im Vergleich zu den herkömmlichen TN ("twisted nematic")-Zellen um bis zu einem Faktor 1000 schnellere Schaltzeiten haben (vgl. z.B. Lagerwall et al. "Ferroelectric Liquid Crystals for Displays", SID Symposium, October Meeting 1985, San Diego, Ca., USA). Aufgrund dieser und anderer günstiger Eigenschaften, z.B. der bistabilen Schaltmöglichkeit und des nahezu blickwinkelunabhängigen Kontrasts, sind FLCs grundsätzlich für die obengenannten Anwendungsgebiete, z.B. über eine Matrixansteuerung, gut geeignet.

Für elektrooptische oder vollständig optische Bauelemente benötigt man entweder Verbindungen, die geneigte bzw. orthogonale smektische Phasen ausbilden und selbst optisch aktiv sind, oder man kann durch Dotierung von Verbindungen, die zwar solche smektischen Phasen ausbilden, selbst aber nicht optisch aktiv sind, mit optisch aktiven Verbindungen ferroelektrische smektische Phasen induzieren. Die gewünschte Phase soll dabei über einen möglichst großen Temperaturbereich stabil sein.

Zur Erzielung eines guten Kontrastverhältnisses in elektrooptischen Bauelementen ist eine einheitliche planare Orientierung der Flüssigkristalle nötig. Eine gute Orientierung in der $S^*_A$ und $S^*_C$-Phase läßt sich erreichen, wenn die Phasenfolge der Flüssigkristallmischung mit abnehmender Temperatur lautet:

$$\text{Isotrop} \rightarrow N^* \rightarrow S^*_A \rightarrow S^*_C$$

Vorraussetzung ist, daß der Pitch (Ganghöhe der Helix) in der N*-Phase sehr groß (größer 10 μm) oder noch besser völlig kompensiert ist (siehe z.B. T. Matsumoto et al., p. 468-470, Proc. of the 6th Int. Display Research Conf., Japan Display, Sept. 30 - Okt. 2, 1986, Tokyo, Japan; M. Murakami et al., ibid. S. 344 - S. 347). Dies erreicht man, indem man zu der chiralen Flüssigkristallmischung, die in der N*-Phase z.B. eine linksdrehende Helix aufweist, einen weiteren optisch aktiven Dotierstoff, der eine rechtsdrehende Helix induziert, in solchen Mengen hinzugibt, daß die Helix gerade kompensiert wird.

Für die Verwendung des SSFLCD-Effektes (Surface Stabilized Ferroelectric Liquid Crystal Display) von Clark und Lagerwall zur einheitlichen, planaren Orientierung ist ferner Vorraussetzung, daß der Pitch in der smektischen C* Phase wesentlich größer ist als die Dicke des Anzeigeelementes (Mol. Cryst. Liq. Cryst. 94 (1983), 213-134 und 114 (1984), 151-187). Dies erreicht man wie im Fall des cholesterischen Pitches durch Verwendung von Dotierstoffen mit entgegengesetztem Drehsinn der Helix.

Ferroelektrische Flüssigkristallanzeigen lassen sich auch durch Nutzung des DHF-Effektes oder des PSFLCD-Effektes (Pitch Stabilized Ferroelectric Liquid Crystal Display, auch SBF = Short Pitch Bistable Ferroelectric Effect genannt) betreiben. Der DHF-Effekt wurde von B.I. Ostrovski in Advances in Liquid Crystal Research and Applications, Oxford/Budapest, 1980, 469 ff. beschrieben, der PSFLCD-Effekt wurde in DE 3 920 625 bzw. EP 0 405 346 beschrieben. Zur Nutzung dieser Effekte wird im Gegensatz zum SSFLCD-Effekt ein flüssigkristallines Material mit einem kurzen $S_C$-Pitch benötigt.

Die optische Schaltzeit $\tau$ [μs] ferroelektrischer Flüssigkristallsysteme, die möglichst kurz sein soll, hängt von der Rotationsviskosität des Systems $\gamma$ [mPas], der spontanen Polarisation $P_s$[nC/cm$^2$] und der elektrischen Feldstärke E[V/m] ab nach der Beziehung

$$\tau \sim \frac{\gamma}{P_s \cdot E}$$

Da die Feldstärke E durch den Elektrodenabstand im elektrooptischen Bauteil und durch die angelegte Spannung festgelegt ist, muß das ferroelektrische Anzeigemedium niedrigviskos sein und eine hohe spontane Polarisation aufweisen, damit eine kurze Schaltzeit erreicht wird.

Schließlich wird neben thermischer, chemischer und photochemischer Stabilität eine kleine optische Anisotropie $\Delta n$, vorzugsweise $\approx 0{,}13$, und eine geringe positive oder vorzugsweise negative dielektrische Anisotropie $\Delta\in$ verlangt (siehe S.T. Lagerwall et al., "Ferroelectric Liquid Crystals for Displays" SID Symposium, Oct. Meeting 1985, San Diego, Ca, USA).

Die Gesamtheit dieser Forderungen ist nur mit Mischungen aus mehreren Komponenten zu erfüllen. Als Basis (oder Matrix) dienen dabei bevorzugt Verbindungen, die möglichst selbst bereits die gewünschte Phasenfolge $I \rightarrow N \rightarrow S_A \rightarrow S_C$ aufweisen. Weitere Komponenten der Mischung werden oftmals zur Schmelzpunktserniedrigung und zur Verbreiterung der $S_C$- und meist auch N-Phase, zum Induzieren der optischen Aktivität, zur Pitch-Kompensation und zur Anpassung der optischen und dielektrischen Anisotropie zugesetzt, wobei aber beispielsweise die Rotationsviskosität möglichst nicht vergrößert werden soll.

Einzelne dieser Komponenten und auch bestimmte Mischungen sind bereits aus dem Stand der Technik bekannt. Da aber die Entwicklung, insbesondere von ferroelektrischen Flüssigkristallmischungen, noch in keiner Weise als abgeschlossen betrachtet werden kann, sind die Hersteller von Displays an unterschiedlichen Mischungen interessiert. Dieses u.a auch deshalb, weil erst das Zusammenwirken der flüssigkristallinen Mischungen mit den einzelnen Bauteilen der Anzeigevorrichtung bzw. der Zellen (z.B. der Orientierungsschicht) Rückschlüsse auf die Qualität auch der flüssigkristallinen Mischungen zuläßt.

In US 4,705,874 und US 4,638,073 werden flüssigkristalline Phenylbenzoate und Biphenyle mit chiralen Oxiranylmethoxy-Substituenten beschrieben.

Desweiteren ist bekannt, daß chirale Oxiranylmethyl-ether von Phenyl-diazin-2,5-diylen bzw. Phenyldiazin-3,6-diylen (EP 0 263 437 und DE 39 09 355) und Organosilylalkyl- bzw. alkenyl-Verbindungen (EP 0 263 437) als Dotierstoffe in Flüssigkristall-Phasen verwendet werden können.

In der EP-A 0 306 919, der EP-A 0 404 081 und der US 5,061,814 werden weitere Flüssigkristallzusammensetzungen beschrieben, die Oxiranderivate als Dotierstoffe oder als Zwischenprodukte zur Herstellung von Mischungskomponenten enthalten.

Aufgabe der vorliegenden Erfindung ist es, Verbindungen aufzuzeigen, die bei hohen Werten für die eigene oder in Flüssigkristall-Phasen induzierte Polarisation $P_S$ Strukturelemente aufweisen, die sie auch mit anderen Komponenten in Flüssigkristall-Systemen "verträglich" (d.h. mischbar) machen, da u.a. der mesogene Teil der Moleküle oftmals für eine gute "Verträglichkeit" mit den anderen Mischungskomponenten in Flüssigkristall-Systemen verantwortlich ist; dabei müssen diese Verbindungen selbst nicht unbedingt flüssigkristallin sein, insbesondere nicht unbedingt eine $S_C$-Phase aufweisen.

Die vorliegende Erfindung betrifft chirale Oxiranylmethyl-ether der allgemeinen Formel (I),

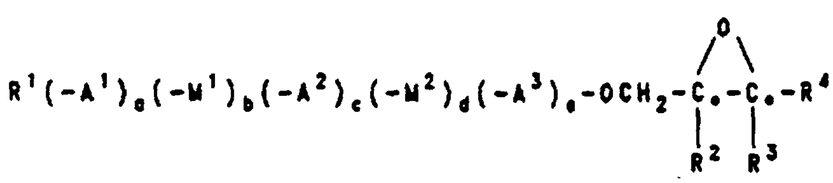

(I)

in der die Symbole und Indices folgende Bedeutung haben:

* ist ein chirales Zentrum

$R^1$ ist ein geradkettiger oder verzweigter Alkylrest mit 1 bis 16 C-Atomen (mit oder ohne asymmetrisches C-Atom), wobei auch eine oder mehrere nicht benachbarte $-CH_2$-Gruppen durch -O-, -S-, -CO-, -CO-O-, -O-CO-, -CO-S-, -S-CO-, -O-CO-O-, -CH=CH-, $-C\equiv C-$, oder $\Delta$ ersetzt sein können und/oder ein oder mehrere H-Atome des Alkylrestes durch F, Cl, Br oder CN substituiert sein können, oder eine der nachfolgenden chiralen Gruppen:

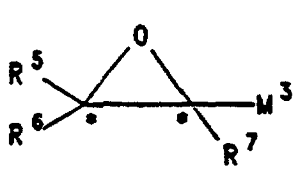

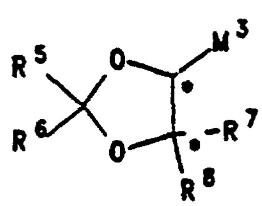

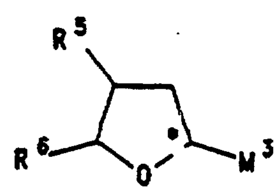

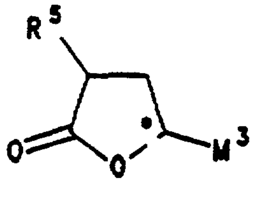
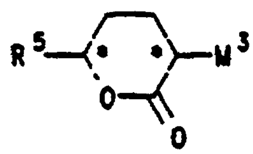
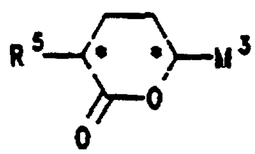

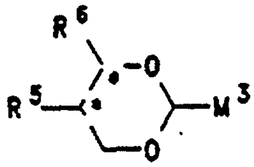
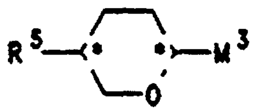

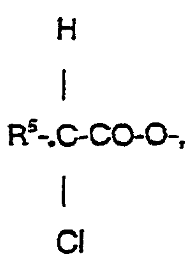
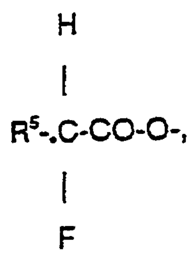
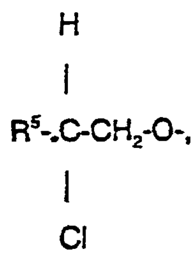
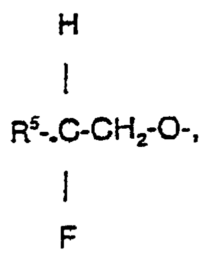

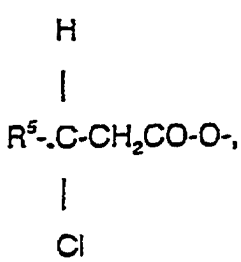
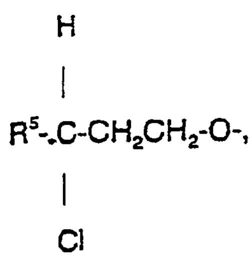

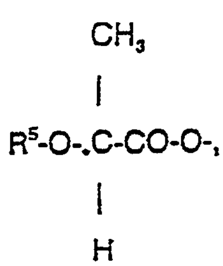
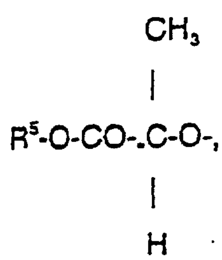

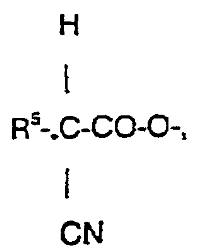 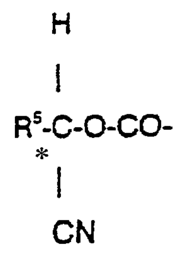

$R^2$, $R^3$, $R^5$, $R^6$, $R^7$, $R^8$ sind unabhängig voneinander H oder ein geradkettiger oder verzweigter Alkylrest mit 1 bis 16 C-Atomen; oder $R^5$ und $R^6$ können zusammen auch $-(CH_2)_4-$ oder $-(CH_2)_5-$ sein, wenn sie als Substituenten an ein Dioxolan-System gebunden sind;

$R^4$ ist ein geradkettiger oder verzweigter Alkylrest mit 1 bis 16 C-Atomen;

$A^1$, $A^2$, $A^3$ sind gleich oder verschieden 1,4-Phenylen, wobei ein oder zwei H-Atome durch F, Cl und/oder CN ersetzt sein können, Pyrazin-2,5-diyl, Pyridazin-3,6-diyl, Pyridin-2,5-diyl, Pyrimidin-2,5-diyl, trans-1,4-Cyclohexylen, bei dem ein oder zwei H-Atome durch CN und/oder $CH_3$ ersetzt sein können, (1,3,4)-Thiadiazol-2,5-diyl, 1,3-Dioxan-2,5-diyl, 1,3-Dithian-2,5-diyl, 1,3-Thiazol-2,4-diyl, 1,3-Thiazol-2,5-diyl, Thiophen-2,4-diyl, Thiophen-2,5-diyl, Piperazin-1,4-diyl, Piperazin-2,5-diyl, Naphthalin-2,6-diyl, Bicyclo[2.2.2]octan-1,4-diyl oder 1,3-Dioxaborinan-2,5-diyl, wobei $A^1$ und $A^2$ und $A^3$ nicht gleichzeitig unsubstituiertes 1,4-Phenylen sein können, wenn $M^1$ oder $M^2$ -COO- ist;

$M^1$, $M^2$ sind gleich oder verschieden, -O-, -S-, -CO-, -CO-O-, -O-CO-, -CO-S-, -S-CO-, -O-CO-O-, $-CH_2-O-$, $-O-CH_2-$, $-CH_2CH_2-$, -CH=CH- oder -C≡C-;

$M^3$ ist $-CH_2-O-$, $-O-CH_2-$, -CO-O-, -O-CO- oder eine Einfachbindung;

a, b, c, d, e sind null oder eins, unter der Bedingung, daß die Summe a+c+e 1, 2 oder 3 ist;

ausgenommen sind die Verbindungen, in denen die Gruppe
$-(A^1)_a(-M^1)_b(-A^2)_c(-M^2)_d(-A^3)_e-$ folgende Bedeutung hat:

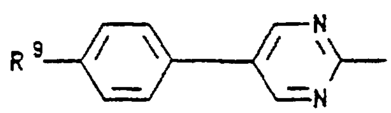 , 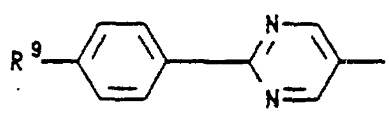 ,

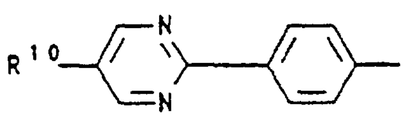 , 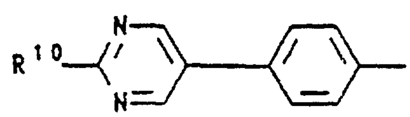 ,

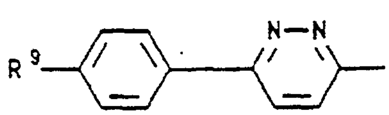 , 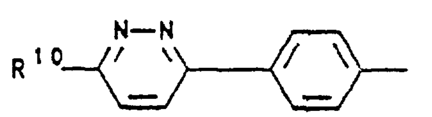 ,

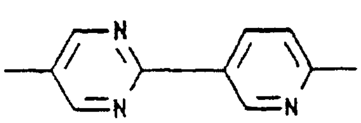 , 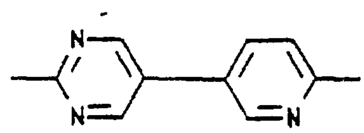 ,

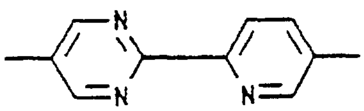 , 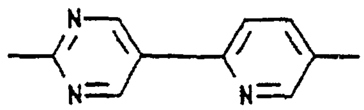 ,

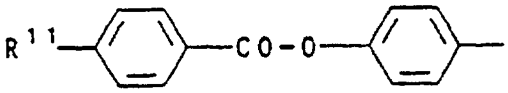

wobei

$R^9$ geradkettiges oder verzweigtes ($C_3$-$C_{12}$)-Alkenyl mit terminaler Doppelbindung bedeutet, in dem eine oder zwei nicht benachbarte $CH_2$-Gruppen durch O- und/oder S-Atome ersetzt sein können,

$R^{10}$ geradkettiges oder verzweigtes Alkyl mit 1-12 C-Atomen bedeutet, wobei eine oder zwei nicht benachbarte $CH_2$-Gruppen durch O und/oder S ersetzt sein können, und

$R^{11}$ eine Alkoxygruppe mit 5-12 C-Atomen darstellt oder

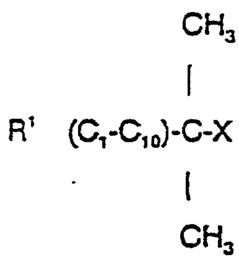

bedeutet, wobei

X geradkettiges oder verzweigtes Alkylen mit 1 bis 16 C-Atomen ist, bei dem auch ein oder zwei nicht-benachbarte $-CH_2$-Gruppen durch -O-, -S-, -O-CO-, -CO-O-, CO oder -O-CO-O- ersetzt sein können.

In der bevorzugten Ausführungsform der Erfindung werden, unter Beibehaltung der oben beschriebenen Ausnahmen, chirale Oxiranylmethyl-ether der allgemeinen Formel (I) eingesetzt, in der die Symbole und Indices folgende Bedeutung haben:

$R^1$ ist ein geradkettiger oder verzweigter Alkylrest mit 1 bis 16 C-Atomen (mit oder ohne asymmetrisches C-Atom), wobei auch eine oder mehrere nicht benachbarte $-CH_2$-Gruppe durch -O-, -S-, -CO-, -CO-O-, -O-CO-, -O-CO-O-,-CH=CH-, -C≡C-, oder △ ersetzt sein können, oder eine der nachfolgenden chiralen Gruppen;

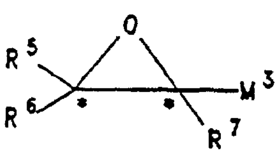 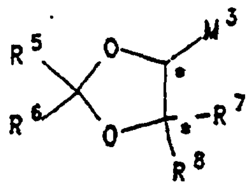 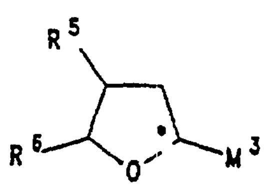

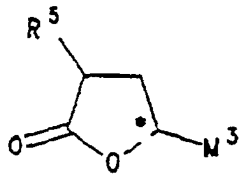

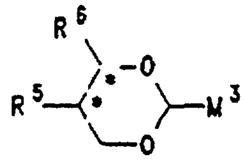

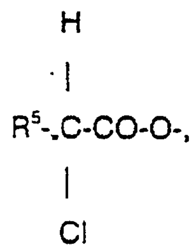

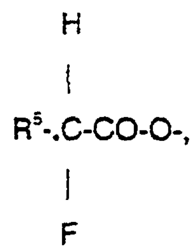

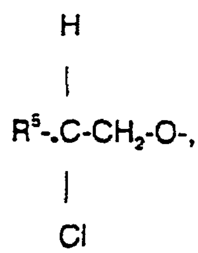

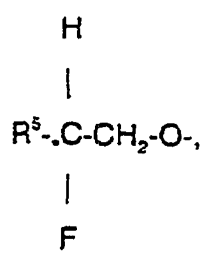

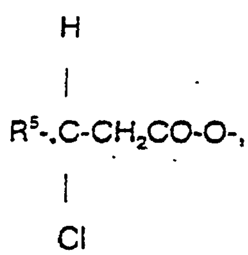

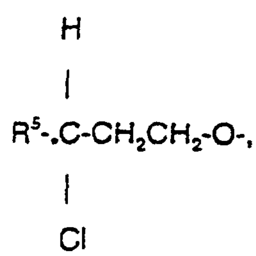

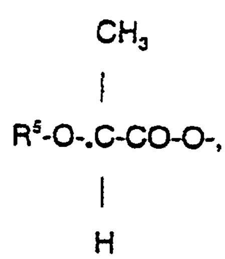

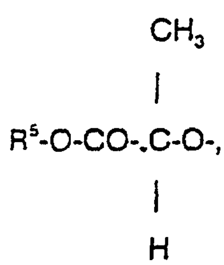

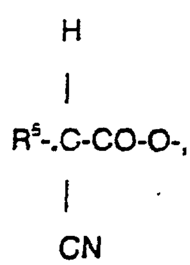

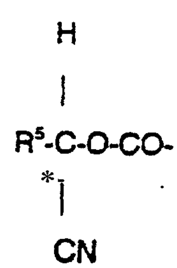

$R^2$, $R^3$, $R^5$, $R^6$, $R^7$, $R^8$ sind unabhängig voneinander H oder ein geradkettiger oder verzweigter Alkylrest mit 1 bis 16 C-Atomen, wobei $R^5$ und $R^6$ zusammen auch $-(CH_2)_4-$ oder $-(CH_2)_5-$ sein können, wenn sie als Substituenten an ein Dioxolan-System gebunden sind,
$R^4$ ist ein geradkettiger oder verzweigter Alkylrest mit 1 bis 16 C-Atomen,
$A^1$, $A^2$, $A^3$ sind gleich oder verschieden 1,4-Phenylen, Pyrazin-2,5-diyl, Pyridazin-3,6-diyl, Pyridin-2,5-diyl, Pyrimidin-2,5-diyl, trans-1,4-Cyclohexylen, (1,3,4)-Thiadiazol-2,5-diyl, 1,3-Dioxan-2,5-diyl, Naphthalin-2,6-diyl oder Bicyclo[2.2.2]octan-1,4-diyl, wobei $A^1$ und $A^2$ und $A^3$ nicht gleichzeitig unsubstituiertes 1,4-Phenylen sein können, wenn $M^1$ oder $M^2$ -COO- ist,
$M^1$, $M^2$ sind gleich oder verschieden -O-, -CO-, -CO-O-, -O-CO-, $-CH_2-O-$, $-O-CH_2-$, $-CH_2-CH_2-$, -CH=CH- oder -C≡C-,
$M^3$ ist $-CH_2-O-$, $-O-CH_2-$, -CO-O-, -O-CO- oder eine Einfachbindung.

In einer besonders bevorzugten Ausführungsform der Erfindung werden, unter Beibehaltung der oben beschriebenen Ausnahmen, chirale Oxiranylmethyl-ether der allgemeinen Formel (I) eingesetzt, in der die Symbole und Indices folgende Bedeutung haben:

$R^1$ ist ein geradkettiger oder verzweigter Alkylrest mit 1 bis 16 C-Atomen (mit oder ohne asymmetrisches C-Atom), wobei auch eine oder zwei nicht benachbarte $-CH_2$-Gruppe durch -O-, -CO-, -CO-O-, -O-CO-, -CH=CH-, oder Δ ersetzt sein können, oder eine der nachfolgenden chiralen Gruppen:

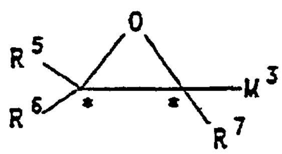
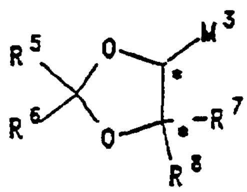
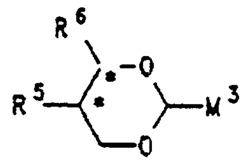

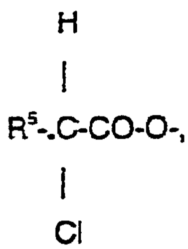
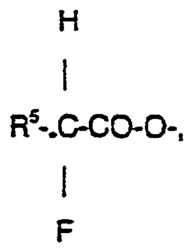

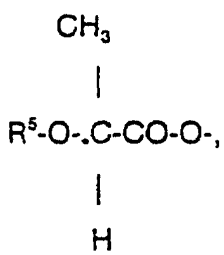
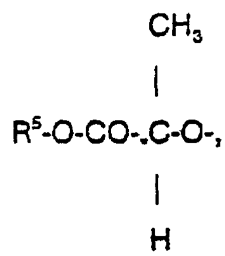

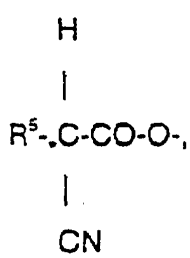

$R^2$, $R^3$, $R^5$, $R^6$, $R^7$, $R^8$ sind unabhängig voneinander H oder ein geradkettiger oder verzweigter Alkylrest mit 1 bis 16 C-Atomen, wobei $R^5$ und $R^6$ zusammen auch $-(CH_2)_4-$ oder $-(CH_2)_5-$ sein können, wenn sie als Substituenten an ein Dioxolan-System gebunden sind,

$R^4$ ist ein geradkettiger oder verzweigter Alkylrest mit 1 bis 16 C-Atomen,

$A^1$, $A^2$, $A^3$ sind gleich oder verschieden 1,4-Phenylen, Pyrazin-2,5-diyl, Pyridin-2,5-diyl, Pyrimidin-2,5-diyl, trans-1,4-Cyclohexylen, (1,3,4)-Thiadiazol-2,5-diyl oder Naphthalin-2,6-diyl, wobei $A^1$ und $A^2$ und $A^3$ nicht gleichzeitig

unsubstituiertes 1,4-Phenylen sein können, wenn $M^1$ oder $M^2$ -COO- ist,
$M^1$, $M^2$ sind gleich oder verschieden -O-, -CO-O-, -O-CO-, $-CH_2-O-$, $-O-OH_2-$. $-CH_2-CH_2-$, -CH=CH- oder -C≡C-,
$M^3$ ist $-CH_2-O-$, $-O-CH_2-$, -CO-O-, -O-CO- oder eine Einfachbindung;

In einer insbesondere bevorzugten Ausführung der Erfindung werden, unter Beibehaltung der oben beschriebenen Ausnahmen, chirale Oxiranylmethyl-ether der allgemeinen Formel (I) eingesetzt in der

$R^1$ ein Alkylrest mit 1 bis 16 C-Atomen ist, wobei eine oder zwei nicht benachbarte $-CH_2$-Gruppe durch -O-, Δ oder -CH =CH- ersetzt sein können, oder die chiralen Gruppen

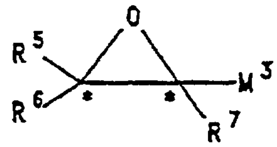

ist,
$R^2$, $R^3$, $R^5$, $R^6$ unabhängig voneinander H oder ein Alkylrest mit 1 bis 16 C-Atomen ist,
$R^4$ ist ein geradkettiger oder verzweigter Alkylrest mit 1 bis 16 C-Atomen,
$M^3$ gleich $-CH_2-O-$ oder -CO-O- ist,

und die Gruppierung $(-A^1)_a(-M^1)_b(-A^2)_c(-M^2)_d(-A^3)_e-$ folgende Bedeutung hat:

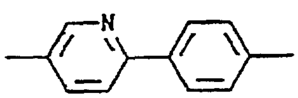 , 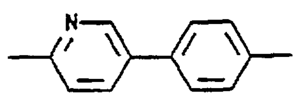 , 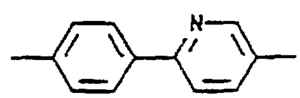 ,

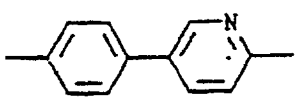 , 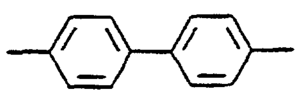 , 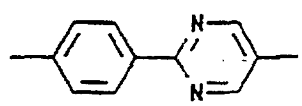 ,

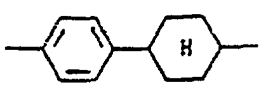 , 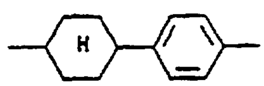 , 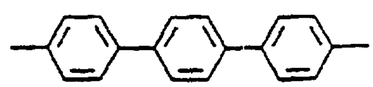 ,

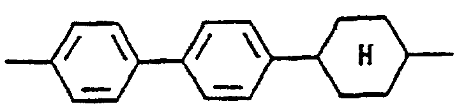 , 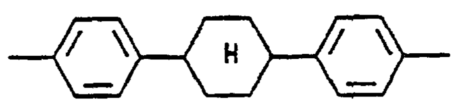 ,

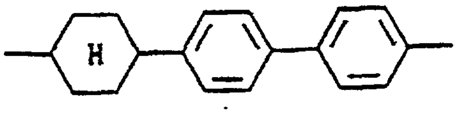 , 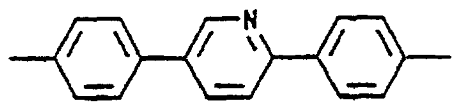 ,

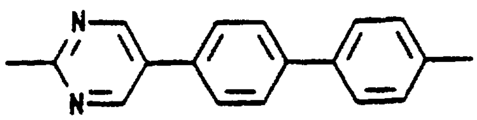 , 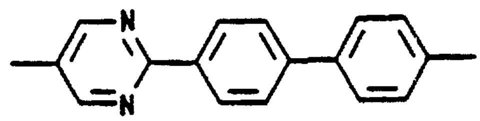 ,

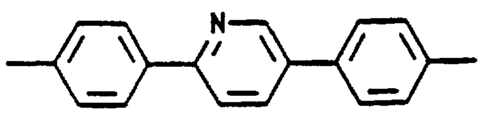 , 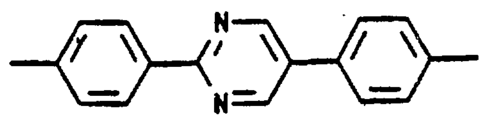 ,

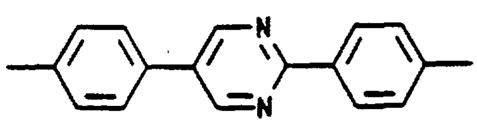 , 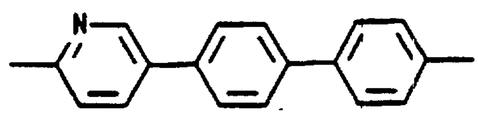 ,

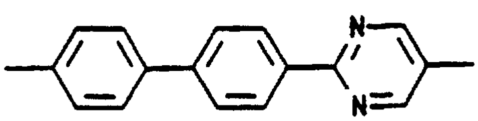 , 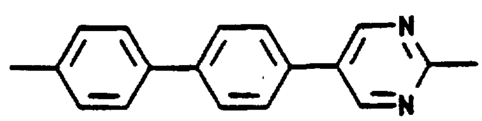 ,

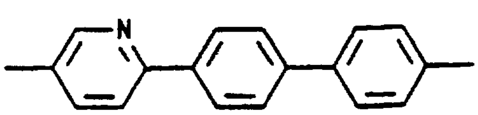 , 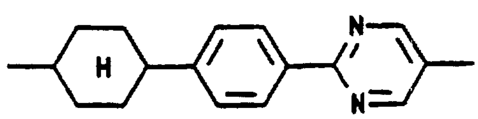 ,

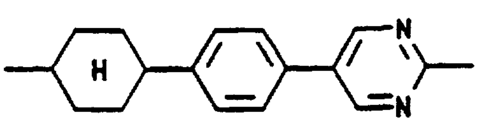 , 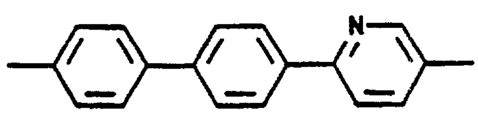 ,

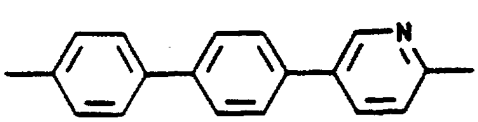 , 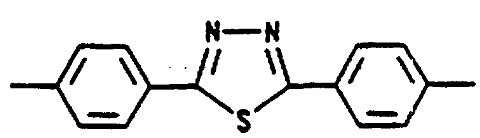 ,

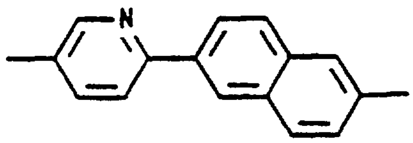 , 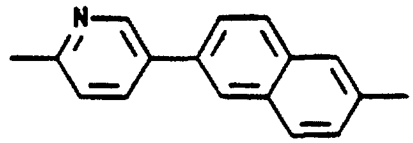 ,

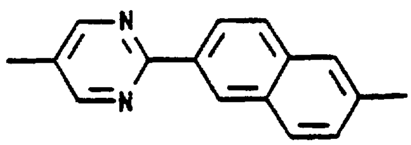 , 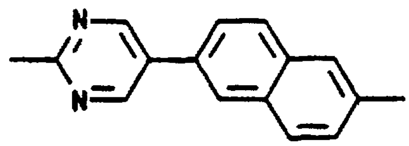 ,

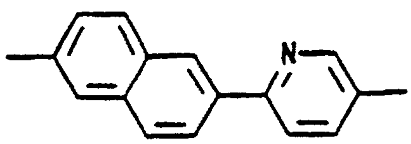 , 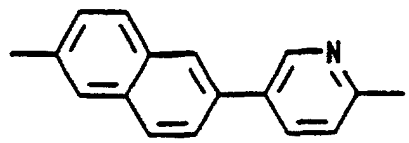 ,

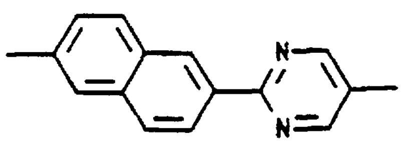 , 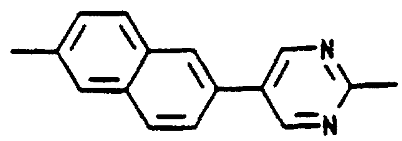 ,

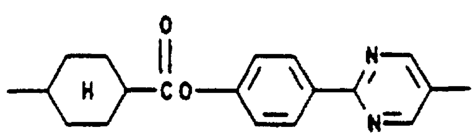 , 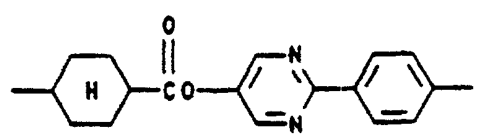 ,

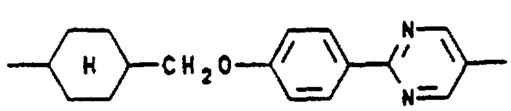 , 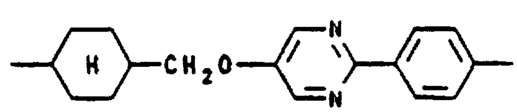

Die Verbindungen der allgemeinen Formel (I) können hergestellt werden durch Umsetzung der Verbindungen der allgemeinen Formel (IX) mit den Oxiranen der allgemeinen Formel (X), worin Z gleich H ist oder für eine nucleofuge Gruppe wie p-Toluolsulfonyl, Methylsulfonyl oder Trifluormethylsulfonyl steht.

$$R^1(-A^1)_a(-M^1)_b(-A^2)_c(-M^2)_d(-A^3)_e\ \text{-OH} \qquad \text{(IX)}$$

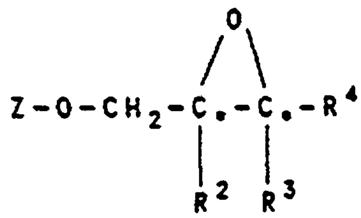

(X)

Diese Umsetzungen können nach an sich literaturbekannten Methoden (siehe z.B. Houben-Weyl, Methoden der Organischen Chemie, Georg Thieme Verlag, Stuttgart; O. Mitsunobu, "The Use of Diethyl Azodicarboxylate and Triphenylphosphine in the Synthesis and Transformation of Natural Products", Synthesis 1981, S. 1 - 28) durchgeführt werden.

Die Verbindungen (IX) und (X) sind aus der Literatur bekannt. So können zum Beispiel die Oxirane (X) mit Z= H aus den entsprechenden Allylalkoholen durch enantioselektive Epoxidierung hergestellt werden (siehe z.B. Pfenniger, "Asymmetric Epoxidation of Allylic Alcohols: The Sharpless Epoxidation, Synthesis 1986, S. 89 - 116). Sie werden dann als solche eingesetzt, oder aber nach Standardmethoden, z.B. durch Umsetzung mit p-Toluolsulfonylchlorid, in die entpsrechenden Verbindungen (X) mit Z gleich $H_3CC_6H_4SO_2$ überführt. Analoges gilt für die anderen genannten nucleofugen Gruppen.

Die genannten Oxiranylmethyl-ether sind als Komponenten für Flüssigkristallmischungen geeignet, wobei $R^4$ dann die gleiche Bedeutung wie$R^2$ haben kann, d.h. auch H bedeutet. Dabei können die LC-Mischungen 0,01 bis 60 Gew.-%, bevorzugt 0,1 bis 20 Gew.-%, besonders bevorzugt 0,1 bis 5 Gew.-% der erfindungsgemäßen Verbindungen enthalten. Die anderen Bestandteile werden vorzugsweise ausgewählt aus den bekannten Verbindungen mit nematischen, cholesterischen und/oder smektischen Phasen; dazu gehören beispielsweise Schiffsche Basen, Biphenyle, Terphenyle, Phenylcyclohexane, Cyclohexylbiphenyle, N, S oder O-haltige Heterocyclen, z.B. Pyrimidine, Zimtsäureester, Cholesterinester oder verschieden überbrückte, terminalpolar mehrkernige Ester von p-Alkylbenzoesäuren. Im allgemeinen liegen die im Handel erhältlichen Flüssigkristallmischungen bereits vor der Zugabe der optisch aktiven Verbindung(en) als Gemische verschiedener Komponenten vor, von denen mindestens eine mesogen ist, d.h. als Verbindung, in derivatisierter Form oder im Gemisch mit anderen Komponenten eine Flüssigkristallphase zeigt, die mindestens eine enantiotrope (Klärtemperatur > Schmelztemperatur) oder monotrope (Klärtemperatur < Schmelztemperatur) Mesophasenbildung erwarten läßt.

Die erfindungsgemäßen Verbindungen sind insbesondere als Dotierstoffe für geneigt-smektische Flüssigkristallphasen geeignet, da sie diese in ferroelektrische Flüssigkristallphasen umwandeln.

Flüssigkristallmischungen, welche die erfindungsgemäßen Verbindungen enthalten können beispielsweise in elektrooptischen oder vollständig optischen Bauelementen eingesetzt werden, z.B. als Anzeigevorrichtung, zur Bildbearbeitung, Informationsverarbeitung, Datenspeicherung oder allgemein im Bereich der nichtlinearen Optik.

Die Elemente enthalten darüber hinaus folgende Komponenten: zwei Elektroden, zwei Trägerplatten sowie mindestens eine Orientierungsschicht. Allgemein wird der Aufbau von FLC-Displays in EP-B 0 032 362 beschrieben.

Die Erfindung wird durch die nachfolgenden Beispiele näher eriäutert:

Für die ferroelektrischen Flüssigkristalimischungen wurden die Werte für die spontane Polarisation $P_s$[nC/cm$^2$], die elektrische Schaltzeit $\tau$[$\mu$s] und die helikale Verdrillungskraft in der nematischen Phase bestimmt, wobei die ersten beiden Messungen bei einer Temperatur von 20°C vorgenommen wurden.

Die $P_s$-Werte wurden nach der Methode von H. Diamant et al. (Rev. Sci. Instr., 28, 30, 1957) gemessen, wobei Meßzellen mit 10 $\mu$m Eiektrodenabstand ohne Orientierungsschicht verwendet wurden.

Zur Messung der elektrischen Schaltzeit wurde eine Rechteckspannung von $\pm$ 100 Volt an die oben beschriebene Meßzelle angelegt und der Polarisationsumkehrstrom gemessen. Die elektrische Schaltzeit $\tau$ ist definiert als Zeitverzögerung zwischen der Spannungsumpolung und dem maximalen Polarisationsstrom.

Die helikale Verdrillung in der nematischen Phase (HTP) wurde nach der Grandjean-Cano Methode mit Hilfe einer Keilzelle bestimmt (F. Grandjean, CR Acad. Sci. (Paris) 172, 71 (1921), R. Cano, Bull. Soc. Franc. Minéral. Crystallogr. XC (333 (1967)).

Die Phasenumwandlungstemperaturen wurden beim Aufheizen mit Hilfe eines Polarisationsmikroskops anhand der Texturänderungen bestimmt. Die Bestimmung des Schmelzpunkts wurde hingegen mit einem DSC-Gerät durchgeführt. Die Angabe der Phasenumwandlungstemperaturen zwischen den Phasen

Nematisch (N bzw. N*)
Smektisch-C ($S_c$ bzw. $S_c$*)
Smektisch-A ($S_A$)
Kristallin (X)

erfolgte in °C und die Werte stehen zwischen den Phasenbezeichnungen in der Phasenfolge.

Beispiel 1

[(2S,3S)-3-Butyloxiran-2-yl]methyl-[4-(5-(4-hexylphenyl)pyrimidin-2-yl)phenyl]ether

Zu 0,01 g (3,84 mmol) Triphenylphosphin in 50 ml THF werden bei 0°C 0,67 g (3,84 mmol) Diethylazodicarboxylat getropft und 0,5 h bei 0°C gerührt. Anschließend werden 1,28 g (3,84 mmol) 5-(4-hexylphenyl)-2-(4-hydroxyphenyl)pyrimidin und 0,50 g (3,84 mmol) (2S,3S)-3-butyloxiran-2-ylmethanol zugegeben und 17 h bei Raumtemperatur gerührt. Nach Einengen des Reaktionsgemisches wird chromatographisch (Kieselgel/Dichlormethan : Essigester = 9 : 1) und durch Umkristallisation aus Acetonitril gereinigt.

Es werden 1,17 g [(2S,3S)-3-Butyloxiran-2-yl]methyl-[4-(5-(4-hexylphenyl)-pyrimidin-2-yl)phenyl]ether mit $[\alpha]_D^{20}$ (c = 2,37 in $CH_2Cl_2$) = -17,00° erhalten.

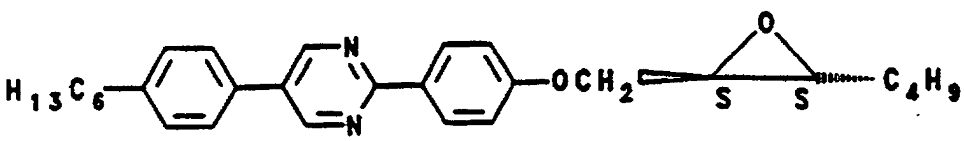

Die Verbindung zeigt folgende Phasenfolge:
X 126 $S^*_C$ 158 - 164 $S_A$ 198 I

Beispiel 2

[(2S,3S)-3-Butyloxiran-2-yl]methyl-[4-(4-(5-octylpyrimidin-2-yl)phenyl)phenyl]ether

Analog Beispiel 1 werden aus 0,76 g (2,91 mmol) Triphenylphpsphin, 0,51 g (2,91 mmol) Diethylazodicarboxylat, 0,70 g (1,94 mmol) 2-(4'-Hydroxy-4-biphenyl)-5-octylpyrimidin und 0,38 g (2,91 mmol) (2S,3S)-3-butyloxiran-2-ylmethanol 0,65 g [(2S,3S)-3-butyloxiran-2-yl]methyl-[4-(4-(5-octylpyrimidin-2-yl)phenyl)phenyl]ether mit $[\alpha]_D^{20}$ (c = 2,68 in $CH_2Cl_2$) = -15,86° erhalten.

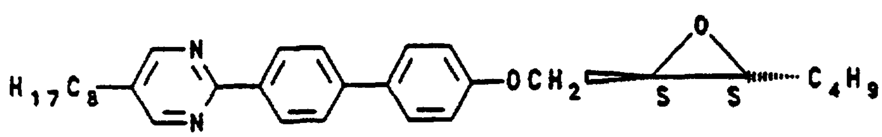

Die Verbindung zeigt folgende Phasenfolge:
X 117 (132) $S_2$ 142 $S^*_C$ 169 N 184 I

Beispiel 3

[(2S,3S)-3-Butyl-oxiran-2-yl]methyl-(4-{5-(2S,3S)-3-butyloxiran-2-yl-methyloxypyrimidin-2-yl}phenyl]ether

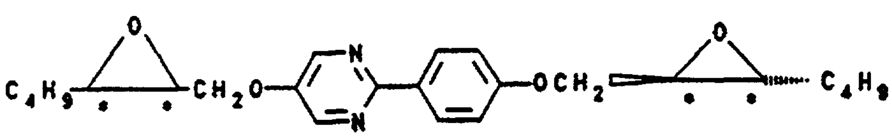

(S), (S)
(S), (S)

wird analog Beispiel 1 erhalten durch Umsetzung von [(2S,3S)-3-Butyl-2-yl]methyl-[4(5-hydroxypyrimidin-2-yl)phenyl]ether - erhalten analog Beispiel 1 aus 5-Benzyloxy-2-(4-hydroxyphenyl)-pyrimidin und (2S,3S)-3-Butyl-oxiran-2-yl-methanol mit nachfolgender hydrogenolytischer Entfernung der Benzylgruppe - mit (2S,3S)-3-Butyl-oxiran-2-yl-methanol.

Die Verbindung zeigt folgende Phasenfolge:

X 89 $S^*_C$ 112,5 I

Beispiel 4

[(2S,3S)-3-Propyloxiran-2-yl]methyl-[2-(4-hexyloxyphenyl)-pyrimidin-5-yl]ether

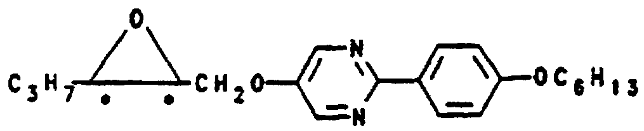

(S), (S)

wird analog Beispiel 1 erhalten aus 2-(4-Hexyloxyphenyl)-5-hydroxy-pyrimidin und (2S,3S)-3-Propyl-oxiran-2-yl-methanol.

Die Verbindung zeigt folgende Phasenfolge:

X 92 $S^*_C$ 98 $S_A$ 107 N* 109 I

Beispiel 5

[(2S,3S)-3-Butyloxiran-2-yl]methyl-[2-(6-octyloxynaphthalin-2-yl)pyrimidin-5-yl]ether

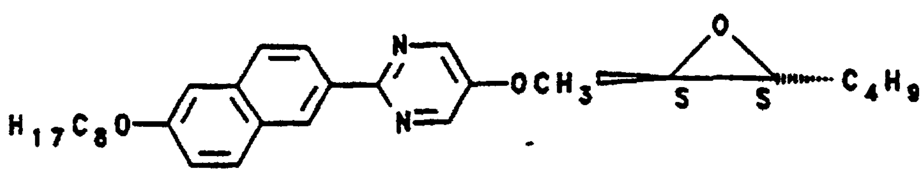

Die Synthese erfolgt analog Beispiel 1 aus 5-Hydroxy-2-(6-octyloxynaphthalin-2-yl)-pyrimidin und (2S,3S)-3-Butyloxiran-2-yl-methanol.

Die Verbindung zeigt die Phasenfolge:

X 85 (55) $S^*_c$ 128,4 $S_A$ 130,6 N* 141 I

$[\alpha]_D^{20}$ = -15,67°

Beispiel 6

[(2S,3R)-3-Propyloxiran-2-yl]methyl-[2-(4-(trans-4-pentylcyclohexyl)phenyl)pyrimidin-5-yl]ether

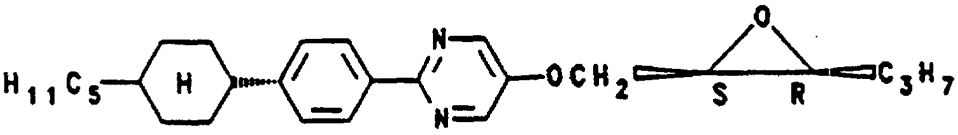

Die Synthese erfolgt analog Beispiel 1 aus 5-Hydroxy 2-[4-(trans-4-pentylcyclohexyl)phenyl]pyrimidin und (2S,3R)-3-Propyloxiran-2-yl-methanol.

Die Verbindung zeigt die Phasenfolge:
X 67 (70) N* 112 I
$[\alpha]_D^{20}$ = -4,01°

Beispiel 7

[(2S,3S)-3-Butyloxiran-2-yl]methyl-[2-(4-trans-4-octylcyclohexyl)phenyl]pyrimidin-5-yl]ether

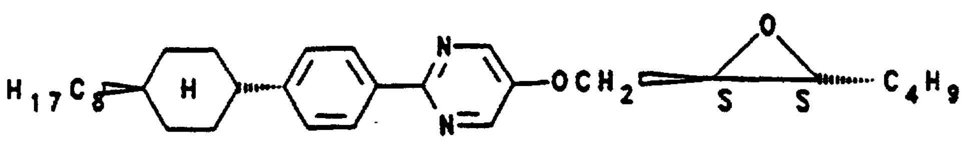

Die Synthese erfolgt analog Beispiel 1 aus 5-Hydroxy-2-[4-(trans-4-octylcyclohexyl)phenyl]pyrimidin und (2S, 3S)-3-Butyloxiran-2-yl-methanol.

Die Verbindung zeigt die Phasenfolge:
X 92 (62) $S_1$ 84 (79) $S_2$ 111 $S_A$ 156 N* 173 I
$[\alpha]_D^{20}$ = -16,65°

Beispiel 8

[(2S,3S)-3-Butyloxiran-2-yl]methyl-[4'-(trans-4-propylcyclohexyl)biphenyl-4-yl]ether

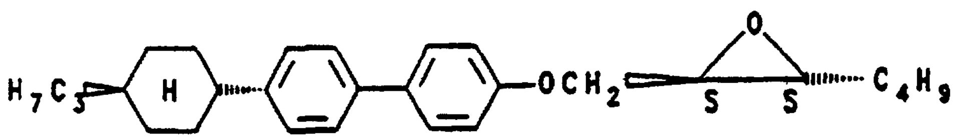

Die Synthese erfolgt analog Beispiel 1 aus 4-Hydroxy-4'-(trans-4-propylcyclohexyl)biphenyl und (2S,3S)-3-Butyloxiran-2-yl-methanol.

Die Verbindung zeigt die Phasenfolge:
X 120 $S_1$ 82 $S_2$ 178 $S_A$ 190 N* 197 I
$[\alpha]_D^{20}$ = -15,35°

Beispiel 9

[(2S,3S)-3-Butyloxiran-2-yl]methyl-[2-(4-(trans-4-pentylcyclohexyl)phenyl)pyrimidin-5-yl]ether

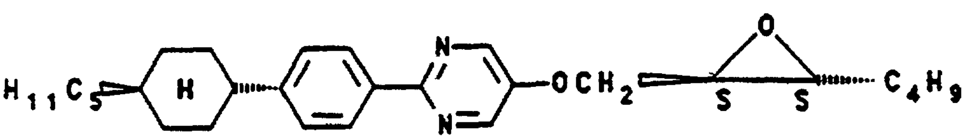

Die Synthese erfolgt analog Beispiel 1 aus 5-Hydroxy-2-[4-(trans-4-pentylcyclohexyl)phenyl]pyrimidin und (2S,3S)-3-Butyloxiran-2-yl-methanol.

Die Verbindung zeigt die Phasenfolge:

X 107 $S^*_C$ 123 N* 186 I
$[\alpha]_D^{20}$ = -16,86°

Beispiel 10

[(2S,3S)-3-Butyloxiran-2-yl]methyl-[4-(5-(4-hexylphenyl)pyridin-2-yl)phenyl]ether

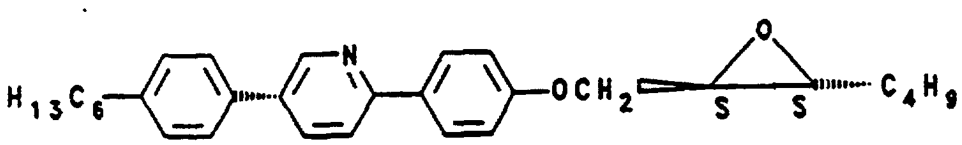

Die Synthese erfolgt analog Beispiel 1 aus Hydroxy-4-[5-(4-hexylphenyl]pyridin-2-yl]benzol und (2S,3S)-3-Butyloxiran-2-yl-methanol.
Die Verbindung zeigt die Phasenfolge:
X 115 $S_1$ 130 $S_2$ 174 $S_3$ 198 $S_C$ 211 $S_A$ 222 I
$[\alpha]_D^{20}$ = -14,76°

Beispiel 11

[(2S,3S)-3-Butyloxiran-2-yl]methyl-[2-(4-(tans-4-propylcyclohexyl)phenyl)pyrimidin-5-yl]ether

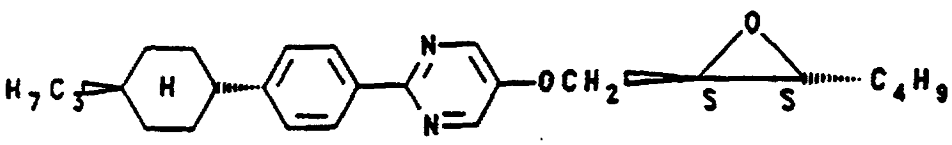

Die Synthese erfolgt analog Beispiel 1 aus 5-Hydroxy-2-[4-(trans-4-propylcyclohexyl)phenyl]pyrimidin und (2S,3S)-3-Butyloxiran-2-yl-methanol.
Die Verbindung zeigt die Phasenfolge:
X 114 $S^*_C$ 105 N* 188 I
$[\alpha]_D^{20}$ = -19,97°

Beispiel 12

[(2S,3S)-3-Butyloxiran-2-yl]methyl-[2-(4-(tans-4-pentylcyclohexylcarbonyloxy)phenyl)pyrimidin-5-yl]ether

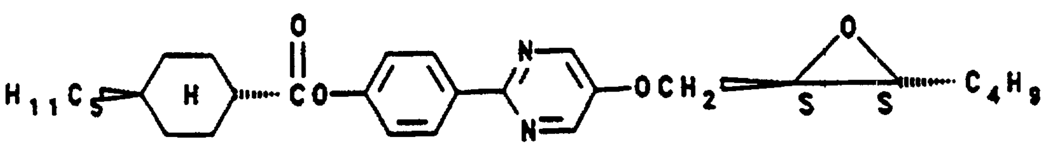

Die Synthese erfolgt analog Beispiel 1 aus 5-Hydroxy-2-[4-(trans-4-pentylcyclohexylcarbonyloxy)phenyl]pyrimidin und (2S,3S)-3-Butyloxiran-2-yl-methanol.
Die Verbindung zeigt die Phasenfolge:
X 103 S, 83 $S^*_C$ 119 N* 203 I

$[\alpha]_D^{20}$ = -16,37°
Spontane Polarisation bei 105°C 170 nC/cm$^2$.

Beispiel 13

[(2S,3S)-3-Butyloxiran-2-yl]methyl-[4-(5-octyloxypyridin-2-yl)phenyl]ether

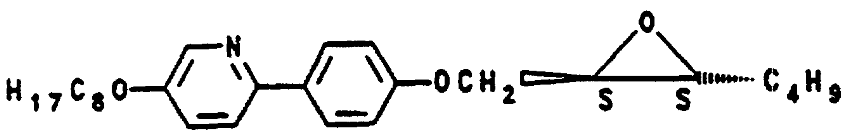

Die Synthese erfolgt analog Beispiel 1 aus 2-(4-Hydroxyphenyl]pyridin-5-octyloxypyridin und (2S,3S)-3-Butyloxiran-2-yl-methanol.
Die Verbindung zeigt die Phasenfolge:
X 87 $S_1$ 86 $S_2$ 103 $S_4$ 111 $S^*_C$ 122 I
$[\alpha]_D^{20}$ = -17,48°

Beispiel 14

[(2S,3S)-3-Butyloxiran-2-yl]methyl-[4-(5-9-decenyloxy)pyridin-2-yl)phenyl]ether

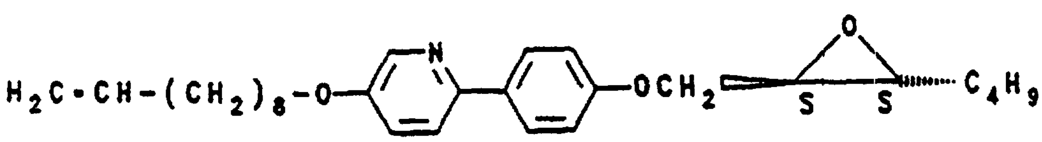

Die Synthese erfolgt analog Beispiel 1 aus 2-(4-Hydroxyphenyl)-5-(7-octenyloxy)pyridin und (2S,3S)-3-Butyloxiran-2-yl-methanol.
Die Verbindung zeigt die Phasenfolge:
X 74 $S_1$ 70 $S_2$ 102 $S^*_C$ 115 I
$[\alpha]_D^{20}$ = -16,95°

Beispiel 15

[(2S,3S)-3-Butyloxiran-2-yl]methyl-[2-(4-(trans-4-pentylcyclohexylmethoxy) phenyl)pyrimidin-5-yl]ether

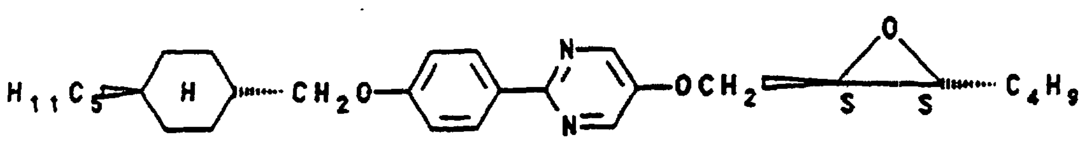

Die Synthese erfolgt analog Beispiel 1 aus 5-Hydroxy-2-[4-(trans-4-pentylcyclohexylmethoxy)phenyl]pyrimidin und (2S,3S)-3-Butyloxiran-2-yl-methanol.
Die Verbindung zeigt die Phasenfolge:
X 13 $S^*_C$ 134 N 172 I

$[\alpha]_D^{20}$ = -18,36°

Beispiel 16

[(2S,3S)-3-Butyloxiran-2-yl]methyl-[4-{5-(2S,3S)-3-propyloxiran-2-yl-methyloxy-pyrimidin-2-yl}phenyl]ether

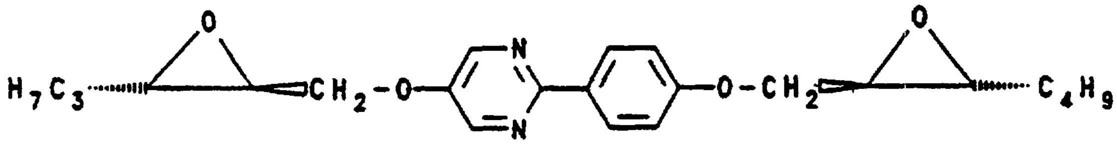

Die Synthese erfolgt analog Beispiel 3, aus [(2S,3S)-3-Butyloxiran-2-yl]methyl-[4-(5-hydroxypyrimidin-2-yl) phenyl]ether und (2S,3S)-3-Propyloxiran-2-yl-methanol.
Die Verbindung zeigt die Phasenfolge:

X 101 $S^*_C$ 108 N* 110 I

$[\alpha]_D^{20}$ = -32,5°

Beispiel 17

[(2S,3S)-3-Butyloxiran-2-yl]methyl-[4-{5-(2S,3R)-3-propyloxiran-2-yl-methyloxypyrimidin-2-yl}phenyl]ether

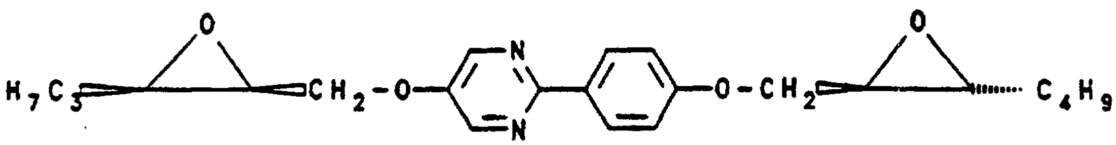

Die Synthese erfolgt analog Beispiel 3, aus [(2S,3S)-3-Butyloxiran-2-yl]methyl-[4-(5-hydroxypyrimidin-2-yl) phenyl]ether und (2R,3R)-3-Propyloxiran-2-yl-methanol.
Die Verbindung zeigt die Phasenfolge:

X 79 I

$[\alpha]_D^{20}$ = -18,0°

Beispiel 18

[(2S,3S)-3-Butyloxiran-2-yl]methyl-[4-{5-(4S)-2,2-dioxolan-4-yl-methyloxy-pyrimidin-2-yl}phenyl]ether

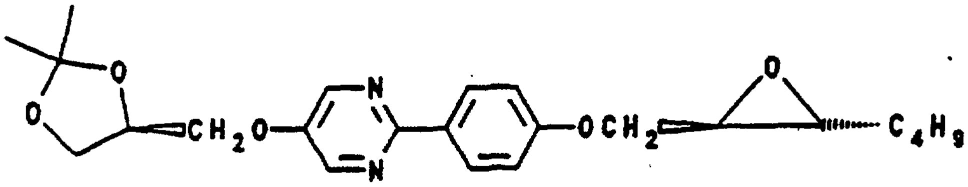

Die Synthese erfolgt analog Beispiel 3, aus [(2S,3S)-3-Butyloxiran-2-yl]methyl-[4-(5 hydroxypyrimidin-2-yl) phenyl]ether und (S)-(+)-2,2-Dimethyl-4-hydroxymethyl-1,3-dioxolan.
Die Verbindung zeigt die Phasenfolge:

X 113 I

$[\alpha]_D^{20}$ = -15,1°

Beispiel 19

[(2S,3S)-3-Propyloxiran-2-yl]methyl-[4-{5-(4S)-2,2-dimethyl-1,3-dioxolan-4-yl-methyloxy-pyrimidin-2-yl}phenyl]ether

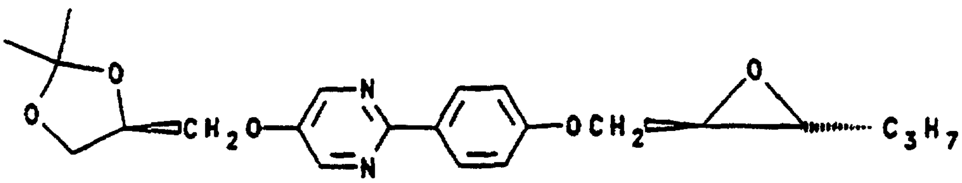

Die Synthese erfolgt analog Beispiel 3, aus [(2S,3S)-3-Propyloxiran-2-yl]methyl-[4-(5-hydroxypyrimidin-2-yl) phenyl]ether und (S)-(+)-2,2-Dimethyl-4-hydroxymethyl-1,3-dioxolan.
Die Verbindung zeigt die Phasenfolge:
X 93-120 I
$[\alpha]_D^{20}$ = -11,7°

Beispiel 20

[(2S,3S)-3-Propyloxiran-2-yl]methyl-[4-{5-(2S,3S)-3-propyloxiran-2-yl-methyloxypyrimidin-2-yl}phenyl]ether

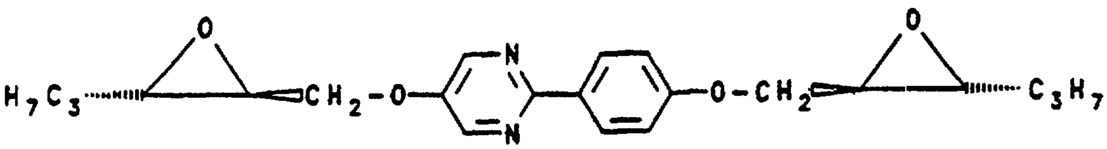

Die Synthese erfolgt analog Beispiel 3, aus [(2S,3S)-3-Propyloxiran-2-yl]methyl-[4-(5-hydroxypyrimidin-2-yl) phenyl]ether und (2S,3S)-3-Propyloxiran-2-yl-methanol.
Die Verbindung zeigt die Phasenfolge:
X 112 (94) $S_A$ 102 N* 111 I
$[\alpha]_D^{20}$ = -37,3°

Beispiel 21

[(2S,3S)-3-Propyloxiran-2-yl]methyl-[4-{5-(2S,3S)-3-butyloxiran-2-yl-methyloxypyrimidin-2-yl}phenyl]ether

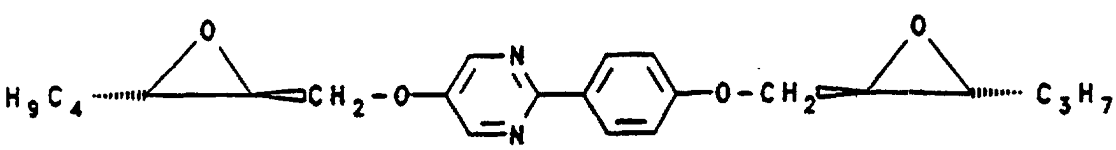

Die Synthese erfolgt analog Beispiel 3, aus [(2S,3S)-3-Propyloxiran-2-yl]methyl-[4-(5-hydroxypyrimidin-2-yl) phenyl]ether und (2S,3S)-3-Butyloxiran-2-yl-methanol.
Die Verbindung zeigt die Phasenfolge:
X 99 $S^*_C$ 110 I

$[\alpha]_D^{20}$ = -34,2°

Beispiel 22

[(2S,3S)-3-Propyloxiran-2-yl]methyl-[4-{5-(trans-4-pentylcyclohexylcarbonyloxy}pyrimidin-2-yl}phenyl]ether

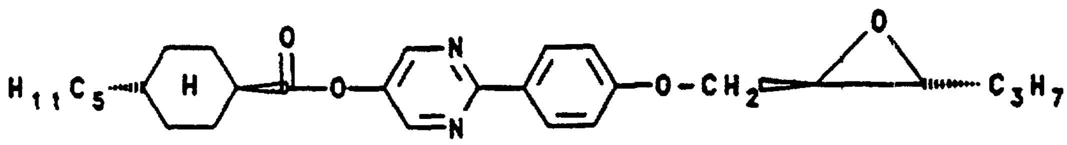

Zu einer Lösung aus 0,5 g (1,75 mmol) [(2S,3S)-3-Propyloxiran-2-yl]methyl-[4-(5-hydroxypyrimidin-2-yl)phenyl]ether, 0,38 g (1,93 mmol) trans-4-Pentylcyclohexylcarbonsäure und 0,023 g (0,193 mmol) 4-Dimethyaminopyridin in 30 ml Dichlormethan fügt man 0,39 g (1,93 mmol) Dicyclohexylcarbodiimid zu und rührt 18 Stunden bei Raumtemperatur. Das Reaktionsgemisch wird abfiltriert und eingeengt. Man reinigt chromatographisch (Kieselgel, $CH_2Cl_2$/ Ethylacetat = 20/1) und durch Umkristallisation aus n-Heptan.

Es werden 0,51 g [(2S,3S)-3-Propyloxiran-2-yl]methyl-[5-{4-trans-4-pentylcyclohexylcarbonyloxy)pyrimidin-2-yl}-phenyl-4-yl]-ether erhalten.

Die Verbindung zeigt die Phasenfolge:

X 105 (68) $S_A$ 181 N* 219 I

$[\alpha]_D^{20}$ = -14,7°

Beispiel 23

[(2S)-2-Methyloxiran-2-yl]methyl-[4-{5-(10-undecenyloxy)pyrimidin-2-yl}phenyl]ether

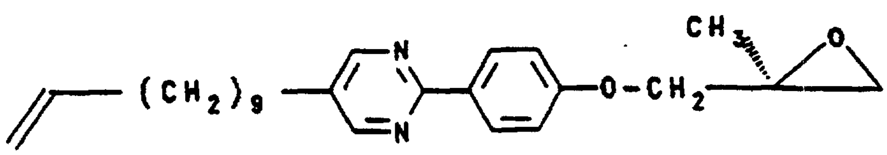

Die Synthese erfolgt analog Beispiel 3, aus [(2S)-2-Methyloxiran-2-yl]methyl-[4-(5-hydroxypyrimidin-2-yl)phenyl]ether und 10-Undecen-1-ol.

Die Verbindung zeigt die Phasenfolge:

X 63 $S^*_C$ 46 $S_A$ 66 I

$[\alpha]_D^{20}$ = +1,78°

Beispiel 24

[(2R)-2-Methyloxiran-2-yl]methyl-[4-{5-(10-undecenyloxy)pyrimidin-2-yl}phenyl]ether

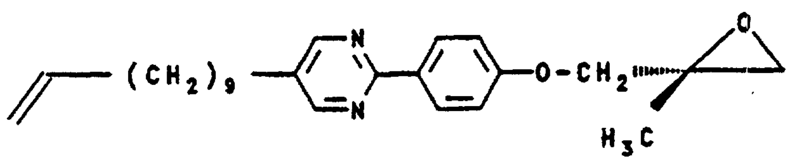

Die Synthese erfolgt analog Beispiel 3, aus [(2R)-2-Methyloxiran-2-yl]methyl-[4-(5-hydroxypyrimidin-2-yl)phenyl]ether und 10-Undecen-1-ol.

Die Verbindung zeigt die Phasenfolge:

X 63 $S^*_C$ 46 $S_A$ 66 I

$[\alpha]_D^{20}$ = -1,63°

Beispiel 25

[(2S)-2-Oxiran-2-yl]methyl-[4-{5-(7-octenyloxy)pyrimidin-2-yl}phenyl]ether

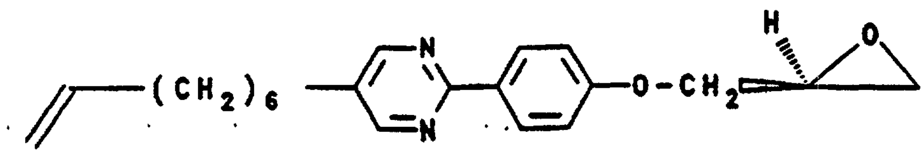

Die Synthese erfolgt analog Beispiel 3, aus [(2S)-2-Oxiran-2-yl]methyl-[4-(5-hydroxypyrimidin-2-yl)phenyl] ether und 7-Undecen-1-ol.

Die Verbindung zeigt die Phasenfolge:

X 62 $S_A$ 69 N* 72 I

$[\alpha]_D^{20}$ = +3,5°

Beispiel 26

[(2S,3S)-3-Butyloxiran-2-yl]methyl-[4-{5-(2R)-2-methyloxiran-2-yl-methyl-oxy-pyrimidin-2-yl}phenyl]ether

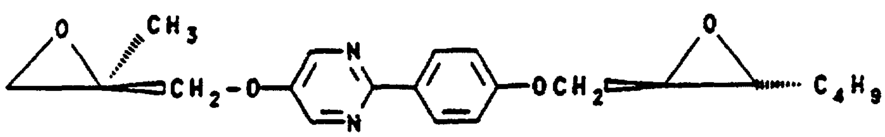

Die Synthese erfolgt analog Beispiel 3, aus [(2S,3S)-3-Butyloxiran-2-yl]methyl-[4-(5-hydroxypyrimidin-2-yl) phenyl]ether und (2S)-2-Methyloxiran-2-yl-methanol.

Die Verbindung zeigt die Phasenfolge:

X 112 I

$[\alpha]_D^{20}$ = -18,5°

Beispiel 27

5-Oxanonan-[4-{5-(2S)-2-methyloxiran-2-methyloxy-pyrimidin-2-yl} phenyl]ether

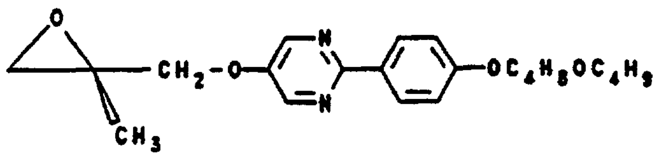

Die Synthese erfolgt analog Beispiel 3, aus 5-Oxanonan-[4-(5-hydroxypyrimidin-2-yl)phenyl]ether und (2R)-2-Methyloxiran-2-yl-methanol.

Die Verbindung zeigt die Phasenfolge:

X 53 I

$[\alpha]_D^{20}$ = -1,7°

Beispiel 28

5-Oxanonan-[4-{5-(2S)-2-methyloxy-pyrimidin-2-yl}phenyl]ether

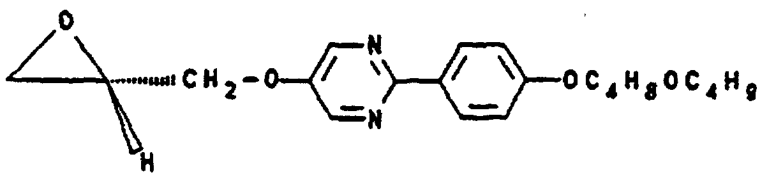

Die Synthese erfolgt analog Beispiel 3, aus 5-Oxanonan-[4-(5-hydroxypyrimidin-2-yl)phenyl]ether und (2R)-2-Oxiranmethanol.

Die Verbindung zeigt die Phasenfolge:

X 62 I

$[\alpha]_D^{20}$ = +4,7°

Beispiel 29

5-Oxanonan-[4-{5-(2S,3S)-3-propyloxiranmethyloxy-pymidin-2-yl}phenyl]ether

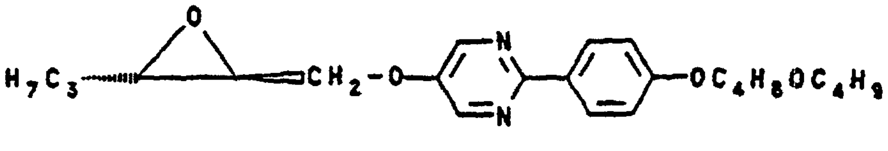

Die Synthese erfolgt analog Beispiel 3, aus 5-Oxanonan-[4-(5-hydroxypyrimidin-2-yl)phenyl]ether und (2S, 3S)-3-Propyloxiran-2-yl-methanol.

Die Verbindung zeigt die Phasenfolge:

X 60 $S^*_C$ 85 $S_A$ 90 I

$[\alpha]_D^{20}$ = -16,6°

Beispiel 30

5-Oxanonan-[4-{5-(2S,3S)-3-butyloxiranmethyloxy-pyrimidin-2-yl}phenyl]ether

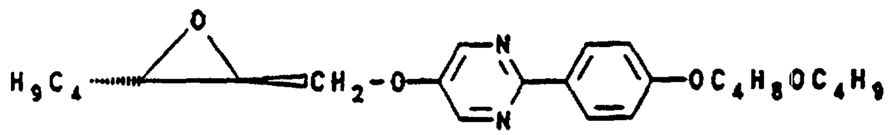

Die Synthese erfolgt analog Beispiel 3, aus 5-Oxanonan-[4-(5-hydroxypyrimidin-2-yl)phenyl]ether und (2S, 3S)-3-Butyloxiran-2-yl-methanol.

Die Verbindung zeigt die Phasenfolge:

X 56 $S^*_C$ 91 I

$[\alpha]_D^{20}$ = -19,9°

Beispiel 31

[(2S,3S)-3-Propyloxiran-2-yl]methyl-[2-{4-(trans-4-pentylcyclohexylcarbonyloxy) pyrimidin-5-yl}phenyl]ether

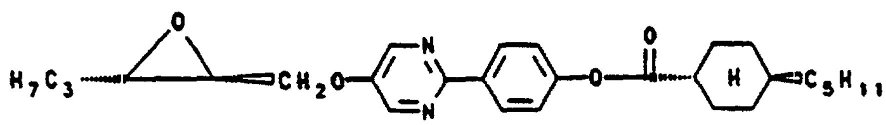

Die Synthese erfolgt analog Beispiel 1, aus 5-Hydroxy-2-[4-(trans-4-pentylcyclohexylcarbonyloxy)phenyl]pyrimidin und (2S,3S)-3-Propyloxiran-2-yl-methanol.

Die Verbindung zeigt die Phasenfolge:

X 129 N* 214 I

$[\alpha]_D^{20}$ = -16,8°

Beispiel 32

[(2R)-2-Methyloxiran-2-yl]methyl-[4-{5-(4-hexylphenyl)pyrimidin-2-yl}phenyl]ether

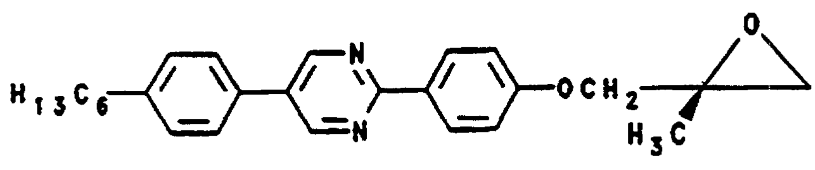

Die Synthese erfolgt analog Beispiel 1, aus 5-(4-hexylphenyl)-2-(4-hydroxyphenyl)pyrimidin und (2S)-2-Methyloxiran-2-yl-methanol.

Die Verbindung zeigt die Phasenfolge:

X 106 $S_3$ 115 $S_2$ 122-126 $S_A$ 180 I

$[\alpha]_D^{20}$ = -1,12°

Beispiel 33

[(2S)-2-Methyloxiran-2-yl]methyl-[4-{5-(4-hexylphenyl)pyrimidin-2-yl)phenyl]ether

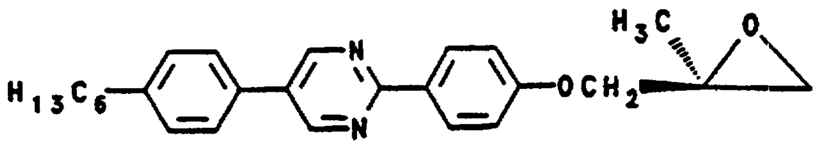

Die Synthese erfolgt analog Beispiel 1, aus 5-(4-hexylphenyl)-2-(4-hydroxyphenyl)pyrimidin und (2R)-2-Methyloxiran-2-yl-methanol,

Die Verbindung zeigt die Phasenfolge:

X 106 $S_3$ 115 $S_2$ 122-126 $S_A$ 180 I

$[\alpha]_D^{20}$ = -1,04°

Beispiel 34

[(2S,3S)-3-Butyloxiran-2-yl]methyl-[4-{5-(2S)-2-methyloxyran-2-yl-carbonyloxypyrimidin-2-yl}phenyl]ether

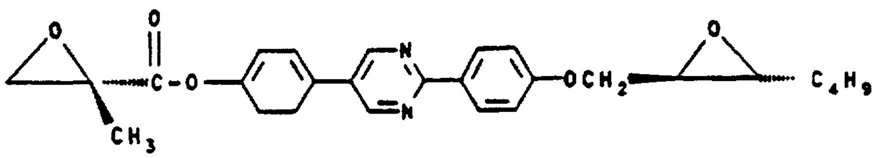

Die Synthese erfolgt analog Beispiel 22, aus [(2S,3S)-3-Butyloxiran-2-yl]methyl-[4-(5-hydroxypyrimidin-2-yl) phenyl]ether und (2S)-2-Methyloxiran-2-yl-carbonsäure.

Die Verbindung zeigt die Phasenfolge:

X 91 N* 65 I

$[\alpha]_D^{20}$ = -1,04°

Beispiel 35

[(2S.3S)-3-Butyloxiran-2-yl]methyl-[4-(2-dodecyloxypyridin-5-yl)phenyl]ether

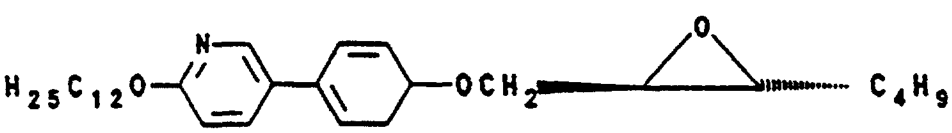

Die Synthese erfolgt analog Beispiel 1 aus 5-(4-Hydroxyphenyl)-2-dodecyloxypyridin und (2S,3S)-3-Butyloxiran-2-yl-methanol

Die Verbindung zeigt folgende Phasenfolge:

X 42 $S^*_C$ 90 $S_A$ 97 I

$[\alpha]_D^{20}$ = -13,6°

Beispiel 36

[(2S,3S)-3-Butyloxiran-2-yl]methyl-[4-(2-hexyloxypyridin-5-yl)phenyl]ether

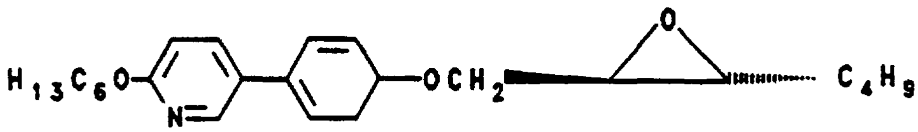

Die Synthese erfolgt analog Beispiel 1 aus 5-(4-Hydroxyphenyl)-2-hexylpyridin und (2S,3S)-3-Butyloxiran-2-yl)-methanol.

Die Verbindung zeigt folgende Phasenfolge

$X_1$ B (-24) $X_2$ 38 $S^*_C$ 92 $S_A$ 101 I

$[\alpha]^{20}_D$ = -18,2°

Anwendungsbeispiel 1

a) Eine ferroelektrische Mischung besteht aus den Komponenten

| | |
|---|---|
| 5-Octyloxy-2-(4-hexyloxyphenyl)pyrimidin | 22,8 Mol-% |
| 5-Octyloxy-2-(4-butyloxyphenyl)pyrimidin | 24 Mol-% |
| 5-Octyloxy-2-(4-decyioxyphenyl)pyrimidin | 19,2 Mol-% |
| 5-Octyloxy-2-(4-octyloxyphenyl)pyrimidin | 10,5 Mol-% |
| trans-4-Pentyl-cyclohexancarbonsäure-(4'-(5-decylpyrimidin-2-yl)phenylester | 13,5 Mol-% |
| ((2S,3S)-3-Butyloxiran-2-yl)methyl-[4-(5-(4-hexylphenyl)pyrimidin-2-yl)phenyl]ether | 10 Mol-% |

zeigt folgende flüssigkristalline Phasenbereiche:

$S^*_C$ 88 $S_A$ 100 N 120 I

und weist bei einer Temperatur von 20°C eine spontane Polarisation von 16,6 nC/cm$^2$ auf und schaltet bei einer Feldstärke von 10 V/µm mit einer Schaltzeit von 180 µs.

Anwendungsbeispiel 2

a) Eine ferroelektrische Mischung besteht aus den Komponenten

| | |
|---|---|
| 5-Octyloxy-2-(4-hexyloxyphenyl)pyrimidin | 24,1 Mol-% |
| 5-Octyloxy-2-(4-butyloxyphenyl)pyrimidin | 25,4 Mol-% |
| 5-Octyloxy-2-(4-decyloxyphenyl)pyrimidin | 20,2 Mol-% |
| 5-Octyloxy-2-(4-octyloxyphenyl)pyrimidin | 11,1 Mol-% |
| trans-4-Pentyl-cyclohexancarbonsäure-(4'-(5-decylpyrimidin-2-yl))phenylester | 14,3 Mol-% |
| ((2S,3S)-3-Butyloxiran-2-yl)methyl-[4-(4-(5-octylpyrimidin-2-yl)phenyl)phenyl] ether | 5 Mol-% |

zeigt folgende flüssigkristalline Phasenbereiche:

$S^*_C$ 87 $S_A$ 94 N 108 I

und weist bei einer Temperatur von 20°C eine spontane Polarisation von 5,0 nC/cm$^2$ auf.

Im Vergleich dazu weist die in DE 38 31 226 beanspruchte flüssigkristalline Mischung, die sich von den obengenannten Mischungen nur dadurch unterscheidet, daß sie keinen Dotierstoff enthält, folgende Phasenbereiche auf.

$S^*_C$ 84 $S_A$ 93 N 105 I

Die Zugabe der Dotierstoffe induziert eine Polarisation und bewirkt außerdem eine Erhöhung aller flüssigkristallinen Phasenübergänge.

Anwendungsbeispiel 3

a) Eine ferroelektrische Mischung bestehend aus den Komponenten

| | |
|---|---|
| 5-Octyl-2-(4-hexyloxyphenyl)pyrimidin | 14,2 Mol-% |
| 5-Octyl-2-(4-decyloxyphenyl)pyrimidin | 9,5 Mol-% |
| 5-Decyl-2-(4-hexyloxyphenyl)pyrimidin | 9,2 Mol-% |
| 5-Octyl-2-(4-(7-cyclopropylheptyloxy)-phenyl)pyridin | 7,7 Mol-% |
| 5-Octyl-2-(4-(6-cyclopropyl)-hexylcarbonyloxyphenyl)pyrimidin | 9,6 Mol-% |
| 5-(8-Cyclopropyloctyloxy)-2-(4-transpentyl-cyclohexyl-4-phenyl)pyrimidin | 11,0 Mol-% |
| trans-4-Pentylcyclohexancarbonsäure-4-(8-Cyclopropyloctyl)pyrimidin-2-yl-phenylester | 6,8 Mol-% |
| 5-(5-Cyclopropylpentyloxy-2-(4-hexyloxyphenyl)pyrimidin | 10,2 Mol-% |
| 2-(4-Hexylphenyl)-5-(4-(4-cyclopropylbutyloxyphenyl)pyrimidin | 6,8 Mol-% |
| ((2S,3S)-3-Butyloxiran-2-yl)methyl-[4-(5-(4-hexylphenyl)pyrimidin-2-yl)phenyl]ether | 15,0 Mol-% |

zeigt folgende flüssigkristalline Phasenbereiche:

$S^*_C$ 78,8 $S_A$ 89,5 N* 108,2 I

Sie weist bei einer Temperatur von 90° eine helikale Verdrillungskraft von 1,47/μm auf. Im Vergleich dazu weist die flüssigkristalline Mischung, die sich von der der obengenannten Mischung nur dadurch unterscheidet, daß sie keinen Oxiran-Dotierstoff enthält, folgende Phasenbereiche auf.

$S_C$ 67,5 $S_A$ 77,3 N 94,8 I

Die Zugabe der erfindungsgemäßen Verbindung führt neben der Ausbildung einer Helix, die in Mischungen zur Kompensation der helikalen Verdrillung genutzt werden kann, zu einer Erhöhung aller flüssigkristallinen Phasenbereiche.

Die folgenden Anwendungsbeispiele 4 bis 29 (Tabelle 1) wurden mit der Basismischung A erstellt.

| Mischung A: | |
|---|---|
| 5-Octyloxy-2-(4-hexyloxyphenyl)pyrimidin | 25,34 Mol-% |
| 5-Octyloxy-2-(4-octyloxyphenyl)pyrimidin | 11,69 Mol-% |
| 5-Octyloxy-2-(4-butyloxyphenyl)pyrimidin | 26,73 Mol-% |
| 5-Octyloxy-2-(4-decyloxyphenyl)pyrimidin | 21,24 Mol-% |
| trans-4-Pentyl-cyclohexancarbonsäure-(4'-(5-decylpyrimidin-2-yl)phenylester | 15 Mol-% |

Tabelle 1 Erfindungsgemäße Dotierstoffe in der Basismischung A

| Anwendungsbeispiel | Substanzbeispiel | Mischungsverhältnis A: Substanzbeispiel | Messtemperatur [°C] | $P_s$ [nC/cm$^2$] | Schaltzeit [$\mu$s] |
|---|---|---|---|---|---|
| 4 | 3 | 90:10 | 20 | 49 | |
| 5 | 4 | 90:10 | 20 | 4 | 370 |
| 6 | 5 | 90:10 | 20 | 15 | |
| 7 | 6 | 90:10 | 20 | 43 | |
| 8 | 8 | 90:10 | 20 | 15 | |
| 9 | 10 | 90:10 | 20 | 11 | |
| 10 | 11 | 90:10 | 25 | 13 | 230 |
| 11 | 12 | 90:10 | 20 | 49 | |
| 12 | 14 | 90:10 | 20 | 8 | 312 |
| 13 | 15 | 90:10 | 20 | 16 | 180 |
| 14 | 16 | 90:10 | 20 | 20 | |
| 15 | 17 | 90:10 | 20 | 74 | |

| Anwendungsbeispiel | Substanzbeispiel | Mischungsverhältnis A: Substanzbeispiel | Messtemperatur [°C] | $P_s$ [nC/cm$^2$] | Schaltzeit [µs] |
|---|---|---|---|---|---|
| 16 | 18 | 90:10 | 40 | 11,5 | |
| 17 | 19 | 90:10 | 20 | 14,5 | |
| 18 | 20 | 90:10 | 20 | 19 | |
| 19 | 21 | 90:10 | 20 | 24 | |
| 20 | 22 | 90:10 | 20 | 7,5 | |
| 21 | 23 | 90:10 | 20 | 5,5 | |
| 22 | 24 | 90:10 | 20 | 4,5 | |
| 23 | 25 | 90:10 | 20 | 4 | |
| 24 | 26 | 90:10 | 40 | 1,2 | |
| 25 | 27 | 90:10 | 20 | 8,5 | 300 |
| 26 | 29 | 90:10 | 20 | 8,4 | |
| 27 | 30 | 90:10 | 20 | 13,5 | |

| Anwendungsbeispiel | Substanzbeispiel | Mischungsverhältnis A: Substanzbeispiel | Messtemperatur [°C] | $P_s$ [nC/cm²] | Schaltzeit [μs] |
|---|---|---|---|---|---|
| 28 | 31 | 90:10 | 20 | 21 | |
| 29 | 33 | 90:10 | 20 | 5,5 | |

Durch Zugabe der erfindungsgemäß beanspruchten Verbindungen zu einer achiralen Basismischung A wird eine hohe spontane Polarisation induziert. Die daraus resultierenden ferroelektrischen Mischungen sind bistabil schaltbar und können somit in geeigneten Kombinationen im Display Anwendung finden.
Weiterhin wurden erfindungsgemäße Dotierstoffe in der Basismischung B getestet (Anwendungsbeispiele 30 bis 52, Tabelle 2).

| Mischung B | |
|---|---|
| 5-Octyloxy-2-(4-hexyloxyphenyl)pyrimidin | 11,06 Mol-% |
| 5-Octyloxy-2-(4-octyloxyphenyl)pyrimidin | 5,11 Mol-% |
| 5-Octyloxy-2-(4-butyloxyphenyl)pyrimidin | 11,67 Mol-% |
| 5-Octyloxy-2-(4-decyloxyphenyl)pyrimidin | 9,28 Mol-% |
| 5-Octyl-2-(4-octyloxyphenyl)pyrimidin | 15,88 Mol-% |
| 5-Octyl-2-(4-hexyloxyphenyl)pyrimidin | 17,7 Mol-% |
| 5-Octyl-2-(4-decyloxyphenyl)pyrimidin | 11,8 Mol-% |
| trans-4-Pentyl-cyclohexancarbonsäure-(4'-(5-dodecylpyrimidin-2-yl)phenylester | 17,5 Mol-% |

Mischung B zeigt folgende flüssigkristalline Phasenbereiche:
$S_C$ 69 $S_A$ 76 N 94 I

Tabelle 2: Erfindungsgemäße Dotierstoffe in der Basismischung B

| Erfindungsbeispiel | Substanzbeispiel | Mischungsverhältnis B: Substanzbeispiel | Messtemperatur [°C] | HTP [$\mu m^{-1}$] | flüssigkristalline Phasen-bereiche der Testmischung |
|---|---|---|---|---|---|
| 30 | 3 | 90:8 | 80 | -2,27 | $S_C^*$ 73 $S_A$ 78 N* 94 I |
| 31 | 4 | 90:10 | 80 | -0,56 | $S_C^*$ 71,50 $S_A$ 78 N* 94,5 I |
| 32 | 5 | 90:10 | 81 | -0,5 | $S_C^*$ 75 $S_A$ 79 N* 98 I |
| 33 | 6 | 95:5 | 74 | -3,75 | $S_C^*$ 65,50 $S_A$ 72 N* 94 I |
| 34 | 7 | 95:5 | 80 | -0,84 | $S_C^*$ 73 $S_A$ 78 N* 94 I |
| 35 | 8 | 95:5 | 82 | -2,3 | $S_C^*$ 69 $S_A$ 80 N* 97 I |
| 36 | 9 | 90:10 | 82 | -0,75 | $S_C^*$ 76 $S_A$ 80 N* 102 I |
| 37 | 10 | 95:5 | 84 | -1,73 | $S_C^*$ 74 $S_A$ 82 N* 99 I |
| 38 | 11 | 90:10 | 81 | -0,6 | $S_C^*$ 75 $S_A$ 79 N* 101 I |
| 39 | 12 | 95:5 | 79 | -1,39 | $S_C^*$ 68 $S_A$ 77 N* 87 I |
| 40 | 15 | 95:5 | 78 | -0,78 | $S_C^*$ 74 $S_A$ 76 N* 98 I |

| Erfindungsbeispiel | Substanzbeispiel | Mischungsverhältnis B: Substanzbeispiel | Messtemperatur [°C] | HTP $[\mu m^{-1}]$ | flüssigkristalline Phasen-bereiche der Testmischung |
|---|---|---|---|---|---|
| 41 | 16 | 90:10 | 80 | -2,47 | $S_C^*$ 73 $S_A$ 78 N* 95 I |
| 42 | 17 | 90:10 | 62 | -8,5 | $S_C^*$ 60 N* 87 I |
| 43 | 18 | 90:10 | 68 | -0,85 | $S_C^*$ 66 N* 90 I |
| 44 | 19 | 95:5 | 72 | -1,4 | $S_C^*$ 67 $S_A$ 70 N* 91 I |
| 45 | 21 | 90:10 | 79 | -1,49 | $S_C^*$ 73 $S_A$ 77 N* 95 I |
| 46 | 22 | 90:10 | 82 | -1,04 | $S_C^*$ 70 $S_A$ 80 N* 104 I |
| 47 | 26 | 92:8 | 73 | -1,33 | $S_C^*$ 66 $S_A$ 71 N* 91 I |
| 48 | 27 | 90:10 | 63 | 0,26 | $S_C^*$ 61 N* 87,5 I |
| 49 | 28 | 90:10 | 75 | 1,1 | $S_C^*$ 64 $S_A$ 73 N* 91 I |
| 50 | 29 | 90:10 | 80 | 0,6 | $S_C^*$ 71 $S_A$ 78 N* 93 I |
| 51 | 31 | 90:10 | 74 | -0,28 | $S_C^*$ 72 N* 105 I |
| 52 | 34 | 95:5 | 69 | -6,32 | $S_C^*$ 67 N* 92 I |

Neben der Induzierung einer spontenen Polarisation ist aus obiger Tabelle erkennbar, daß die erfindungsgemäß beanspruchten Verbindungen den $S^*_C/S^*_A$-Übergang gegenüber der achiralen Mischung B anheben und somit den praxistauglichen Temperaturbereich verbreitern.
Die helikale Verdrillungskraft dieser Verbindungen läßt sich vorteilhaft dafür verwenden, die Verdrillung in der cholesterischen Phase aufzuheben.
Eine genügend große Ganghöhe der Helix in der cholesterischen Phase ist notwendig, um in Displays eine gute homogen Orientierung der Flüssigkristallmischung zu erzielen.
In den Anwendungsbeispielen 53 und 54 wurde die kritische Pulsfläche von ferroelektrischen Mischungen, die die erfindungsgemäßen Dotierstoffe enthalten, bestimmt. Unter der kritischen Pulsfläche versteht man das Produkt aus der kritischen Pulsbreite und der angewendeten Feldstärke.
Die kritische Pulsbreite ist die minimale Pulsbreite, die nötig ist, um den FLC aus einem stabilen Schaltzustand in den anderen zu schalten. Sie wird bestimmt, indem der FLC zuerst durch einen genügend großen bipolaren Rückstellpuls in einen der zwei stabilen Schaltzustände geschaltet wird und dann nach 20 ms ein inverser bipolarer Puls angewendet wird, deren Breite variiert wird, bis ein Schalten erfolgt.
In den beiden folgenden Beispielen wurde die kritische Pulsfläche durch Applikation bipolarer Pulse mit einer Pulsbreite von 30 µs und Ausmessen der Pulsfläche eines Schenkels bestimmt.

Anwendungsbeispiel 53

Eine ferroelektrische Mischung bestehend aus den Komponenten

| | |
|---|---|
| 5-Octyl-2-(4-hexyloxyphenyl)pyrimidin | 16,1 Mol-% |
| 5-Octyl-2-(4-decyloxyphenyl)pyrimidin | 10,7 Mol-% |
| 5-Octyl-2-(4-octyloxyphenyl)pyrimidin | 14,5 Mol-% |
| 5-Octyloxy-2-(4-hexyloxyphenyl)pyrimidin | 10 Mol-% |
| 5-Octyloxy-2-(4-octyloxyphenyl)pyrimidin | 4,6 Mol-% |
| 5-Octyloxy-2-(4-butyloxyphenyl)pyrimidin | 10,5 Mol-% |
| 5-Octyloxy-2-(4-decyloxyphenyl)pyrimidin | 8,4 Mol-% |
| (2R,3R)-3-Propyloxiran-2-carbonsäure[4-(5-{10-undecenyloxy})pyrimidin-2-yl]phenyl-ester | 6 Mol-% |
| [(2S,3S)-3-Butyloxiran-2-yl]methyl-[2-(4-(trans-4-phtylcyclohexylcarbonyloxy)phenyl) pyrimidin-5-yl]ether | 9,6 Mol-% |
| [(2S,3S)-3-Butyloxiran-2-yl]methyl-[4-(5-(4-hexylphenyl)pyrimidin-2-yl)phenyl]ether | 9,6 Mol-% |

zeigt folgende flüssigkristalline Phasenbereiche:

$S^*_C$ 74 $S_A$ 87 N* 98 I

und weist bei einer Temperatur von 25°C eine spontane Polarisation von 53 $nC/cm^2$ auf.
Die Mischung schaltet bei 25°C mit einer kritischen Pulsfläche von 630 Vs/m.
Die helikale Verdrillung ist im gesamten Temperaturbereich der cholesterischen Phase weitgehend aufgehoben.
Die gleiche Mischung ohne die erfindungsgemäß beanspruchten Verbindungen zeigt folgende flüssigkristalline Phasenbereiche

$S^*_C$ 56 $S_A$ 77 N* 79 I

und weist bei 25°C eine spontane Polarisation von 12 $nC/cm^2$ auf. Die helikale Verdrillungskraft beträgt bei 18°C +4,9 $\mu m^{-1}$.
Aus obigem Vergleich ist klar ersichtlich, daß die hier beanspruchten Verbindungen nicht nur den praxistauglichen Phasenbereich vergrößern und die helikale Verdrillung weitgehend aufheben sondern auch die spontane Polarisation stark erhöhen und somit die Schaltzeiten verkleinern.

Anwendungsbeispiel 54

Eine ferroelektrische Mischung bestehend aus den Komponenten

| | |
|---|---|
| 5-Octyl-2-(4-hexyloxyphenyl)pyrimidin | 17,1 Mol-% |
| 5-Octyl-2-(4-decyloxyphenyl)pyrimidin | 11,4 Mol-% |
| 5-Octyl-2-(4-octyloxyphenyl)pyrimidin | 15,3 Mol-% |
| 5-Octyloxy-2-(4-hexyloxyphenyl)pyrimidin | 10,7 Mol-% |

(fortgesetzt)

| | |
|---|---|
| 5-Octyloxy-2-(4-octyloxyphenyl)pyrimidin | 4,9 Mol-% |
| 5-Octyloxy-2-(4-butyloxyphenyl)pyrimidin | 11,2 Mol-% |
| 5-Octyloxy-2-(4-decyloxyphenyl)pyrimidin | 8,9 Mol-% |
| (2R,3R)-3-Propyloxiran-2-carbonsäure[4-(5-{10-undecenyloxy})pyrimidin-2-yl]phenylester | 5,5 Mol-% |
| [(2S,3S)-3-Butyloxiran-2-yl]methyl-[2-(4-(trans-4-pentylcyclohexylcarbonyloxy)phenyl) pyrimidin-5-yl]ether | 7 Mol-% |
| [(2S,3S)-3-Butyloxiran-2-yl]methyl-[4-{5-(2S,3S)-3-butyloxiran-2-yl-methyloxy-pyrimidin-2-yl}) phenyl]ether | 8 Mol-% |

zeigt folgende flüssigkristalline Phasenbereiche:

$S^*_C$ 69 $S_A$ 80 N* 87 I

und weist bei einer Temperatur von 25°C eine spontane Polarisation von 47 $nC/cm^2$ auf.

Die Mischung schaltet bei 25°C mit einer kritischen Pulsfläche von 670 Vs/m.

Die helikale Verdrillung ist im gesamten Temperaturbereich der cholesterischen Phase weitgehend aufgehoben.

## Patentansprüche

**1.** Chirale Oxiranylmethyl-ether der allgemeinen Formel (I),

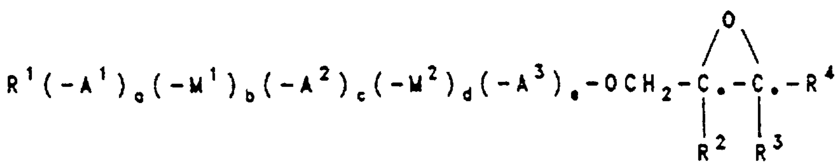

(I)

in der die Symbole und Indices folgende Bedeutung haben:

* ist ein chirales Zentrum

$R^1$ ist ein geradkettiger oder verzweigter Alkylrest mit 1 bis 16 C-Atomen (mit oder ohne asymmetrisches C-Atom), wobei auch eine oder mehrere nicht benachbarte $-CH_2$-Gruppen durch -O-, -S-, -CO-, -CO-O-, -O-CO-, -CO-S-, -S-CO-, -O-CO-O-, -CH = CH-, -C ≡ C-, oder Δ ersetzt sein können und/oder ein oder mehrere H-Atome des Alkylrestes durch F, Cl, Br oder CN substituiert sein können, oder eine der nachfolgenden chiralen Gruppen:

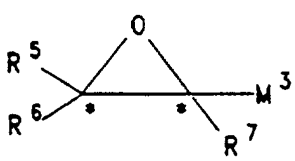

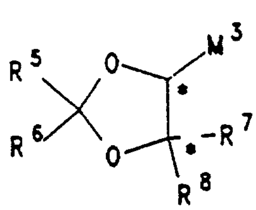

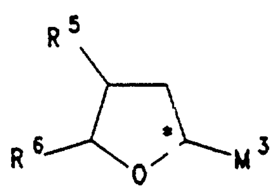

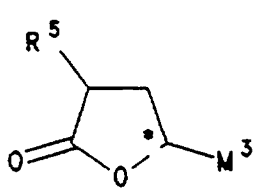

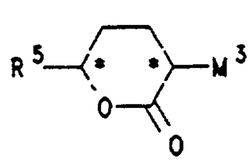

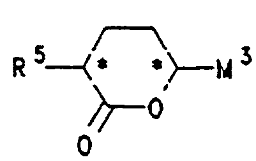

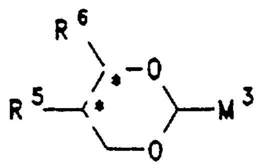 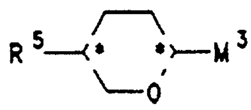

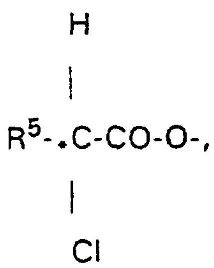 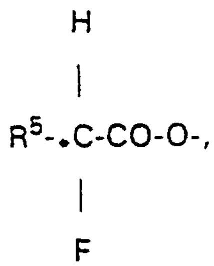 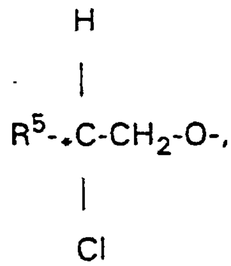 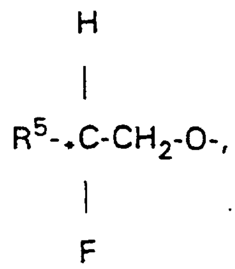

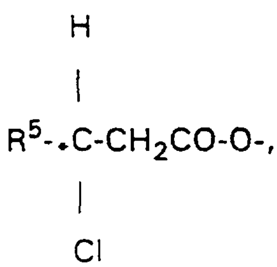 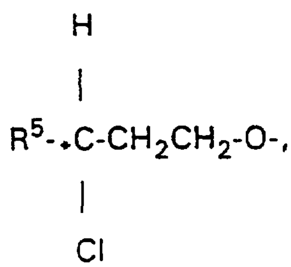

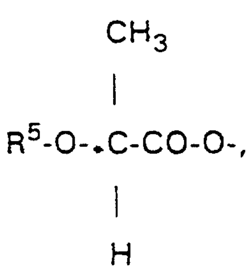 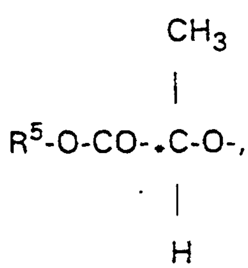

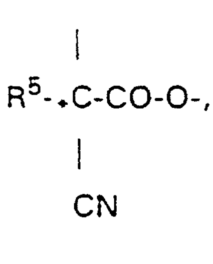 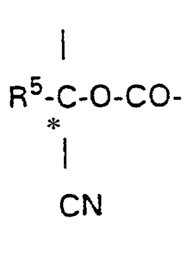

$R^2$, $R^3$, $R^5$, $R^6$, $R^7$, $R^8$ sind unabhängig voneinander H oder ein geradkettiger oder verzweigter Alkylrest mit

1 bis 16 C-Atomen, oder $R^5$ und $R^6$ können zusammen auch $-(CH_2)_4-$ oder $-(CH_2)_5-$ sein, wenn sie als Substituenten an ein Dioxolan-System gebunden sind,

$R^4$ ist ein geradkettiger oder verzweigter Alkylrest mit 1 bis 16 C-Atomen,

$A^1$, $A^2$, $A^3$ sind gleich oder verschieden 1,4-Phenylen, wobei ein oder zwei H-Atome durch F, Cl und/oder CN ersetzt sein können, Pyrazin-2,5-diyl, Pyridazin-3,6-diyl, Pyridin-2,5-diyl, Pyrimidin-2,5-diyl, trans-1,4-Cyclohexylen, bei dem ein oder zwei H-Atome durch CN und/oder $CH_3$ ersetzt sein können, (1,3,4)-Thiadiazol-2,5-diyl, 1,3-Dioxan-2,5-diyl, 1,3-Dithian-2,5-diyl, 1,3-Thiazol-2,4-diyl, 1,3-Thiazol-2,5-diyl, Thiophen-2,4-diyl, Thiophen-2,5-diyl, Piperazin-1,4-diyl, Piperazin-2,5-diyl, Naphthalin-2,6-diyl, Bicyclo[2.2.2]octan-1,4-diyl oder 1,3-Dioxaborinan-2,5-diyl, wobei $A^1$ und $A^2$ und $A^3$ nicht gleichzeitig unsubstituiertes 1,4-Phenylen sein können, wenn $M^1$ oder $M^2$ -COO- ist,

$M^1$, $M^2$ sind gleich oder verschieden, -O-, -S-, -CO-, -CO-O-, -O-CO-, -CO-S-, -S-CO-, -O-CO-O-, $-CH_2-O-$, $-O-CH_2-$, $-CH_2CH_2-$, -CH = CH- oder -C ≡ C-,

$M^3$ ist $-CH_2-O-$, $-O-CH_2-$, -CO-O-, -O-CO- oder eine Einfachbindung,

a, b, c, d, e sind null oder eins, unter der Bedingung, daß die Summe a+c+e 1, 2 oder 3 ist,

ausgenommen sind die Verbindungen, in denen die Gruppe $(-A^1)_a(-M^1)_b(-A^2)_c(-M^2)_d(-A^3)_e-$ folgende Bedeutung hat:

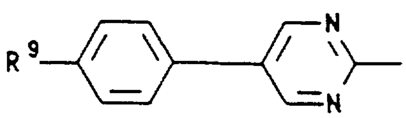 , 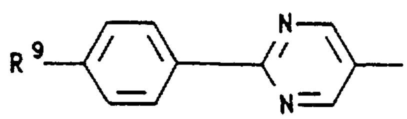 ,

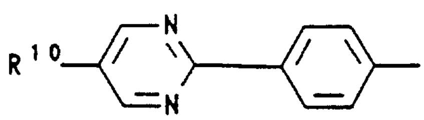 , 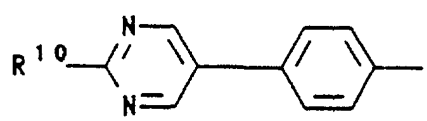 ,

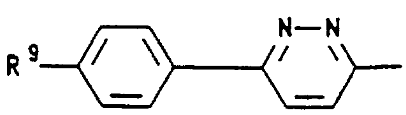 , 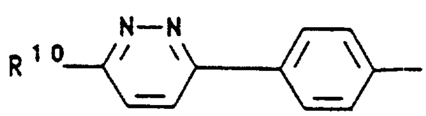 ,

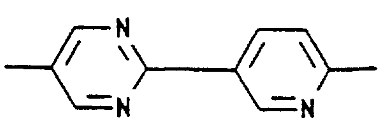 , 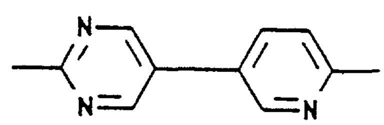 ,

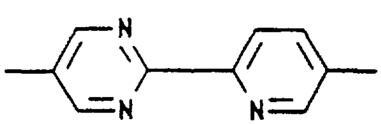 , 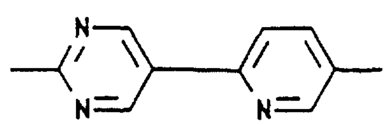 ,

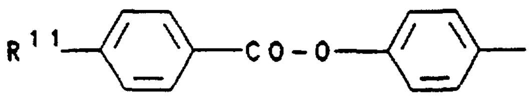

wobei

$R^9$ geradkettiges oder verzweigtes ($C_3$-$C_{12}$)-Alkenyl mit terminaler Doppelbindung bedeutet, in dem eine oder zwei nicht benachbarte $CH_2$-Gruppen durch O- und/oder S-Atome ersetzt sein können,

$R^{10}$ geradkettiges oder verzweigtes Alkyl mit 1-12 C-Atomen bedeutet, wobei eine oder zwei nicht benachbarte $CH_2$-Gruppen durch O und/oder S ersetzt sein können, und

$R^{11}$ eine Alkoxygruppe mit 5-12 C-Atomen darstellt

oder

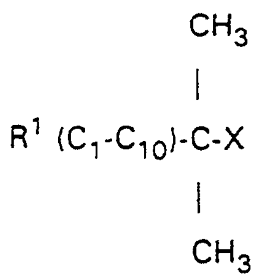

bedeutet, wobei

X geradkettiges oder verzweigtes Alkylen mit 1 bis 16 C-Atomen, bei dem auch ein oder zwei nicht-benachbarte $-CH_2$-Gruppen durch -O-, -S-, -O-CO-, -CO-O-, CO oder -O-CO-O- ersetzt sein können.

**2.** Chirale Oxiranylmethyl-ether nach Anspruch 1, **dadurch gekennzeichnet, daß** die Symbole und Indices der allgemeinen Formel (I) folgende Bedeutung haben:

$R^1$ ist ein geradkettiger oder verzweigter Alkylrest mit 1 bis 16 C-Atomen (mit oder ohne asymmetrisches C-Atom), wobei auch eine oder mehrere $-CH_2$-Gruppe durch -O-, -S-, -CO-, -CO-O-, -O-CO-, -O-CO-O-, -CH=CH-, -C≡C-, oder Δ ersetzt sein können, oder eine der nachfolgenden chiralen Gruppen:

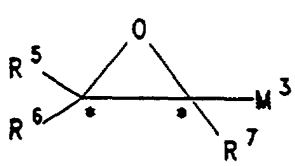

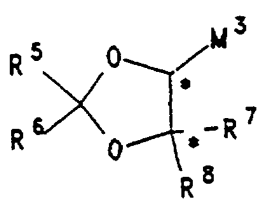

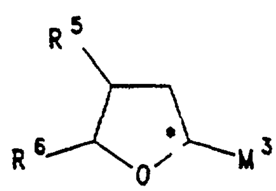

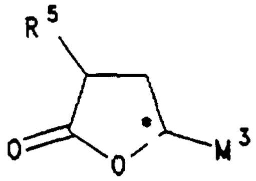

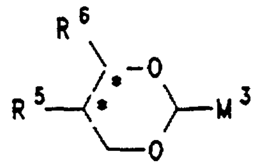

H
|
$R^5$-*C-CO-O-,
|
Cl

H
|
$R^5$-*C-CO-O-,
|
F

H
|
$R^5$-*C-$CH_2$-O-,
|
Cl

H
|
$R^5$-*C-$CH_2$-O-,
|
F

H
|
$R^5$-*C-$CH_2$CO-O-,
|
Cl

H
|
$R^5$-*C-$CH_2CH_2$-O-,
|
Cl

$CH_3$
|
$R^5$-O-*C-CO-O-,
|
H

$CH_3$
|
$R^5$-O-CO-*C-O-,
|
H

H
|
$R^5$-*C-CO-O-,
|
CN

H
|
$R^5$-C-O-CO-
*|
CN

$R^2$, $R^3$, $R^5$, $R^6$, $R^7$, $R^8$ sind unabhängig voneinander H oder ein geradkettiger oder verzweigter Alkylrest mit 1 bis 16 C-Atomen, wobei $R^5$ und $R^6$ zusammen auch $-(CH_2)_4-$ oder $-(CH_2)_5-$ sein können, wenn sie als Substituenten an ein Dioxolan-System gebunden sind,

$R^4$ ist ein geradkettiger oder verzweigter Alkylrest mit 1 bis 16 C-Atomen,

$A^1$, $A^2$, $A^3$ sind gleich oder verschieden 1,4-Phenylen, Pyrazin-2,5-diyl, Pyridazin-3,6-diyl, Pyridin-2,5-diyl, Pyrimidin-2,5-diyl, trans-1,4-Cyclohexylen, (1,3,4)-Thiadiazol-2,5-diyl, 1,3-Dioxan-2,5-diyl, Naphthalin-2,6-diyl oder Bicyclo[2.2.2]octan-1,4-diyle, wobei $A^1$ und $A^2$ und $A^3$ nicht gleichzeitig unsubstituiertes 1,4-Phenylen sein können, wenn $M^1$ oder $M^2$ -COO- ist,

$M^1$, $M^2$ sind gleich oder verschieden -O-, -CO-, -CO-O-, -O-CO-, $-CH_2-O-$, $-O-CH_2-$, $-CH_2-CH_2-$, -CH=CH- oder -C≡C-,

$M^3$ ist $-CH_2-O-$, $-O-CH_2-$, -CO-O-, -O-CO- oder eine Einfachbindung.

**3.** Chirale Oxiranylmethyl-ether nach Anspruch 1, **dadurch gekennzeichnet, daß** die Symbole und Indices der allgemeinen Formel (I) folgende Bedeutung haben:

$R^1$ ist ein geradkettiger oder verzweigter Alkylrest mit 1 bis 16 C-Atomen (mit oder ohne asymmetrisches C-Atom), wobei auch eine oder zwei $-CH_2$-Gruppe durch -O-, -CO-, -CO-O-, -O-CO-, -CH = CH-, oder Δ ersetzt sein können, oder eine der nachfolgenden chiralen Gruppen:

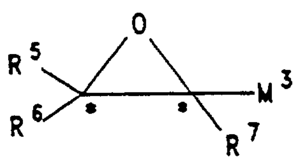

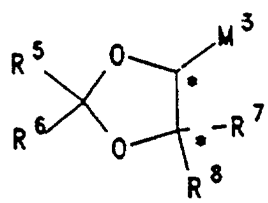

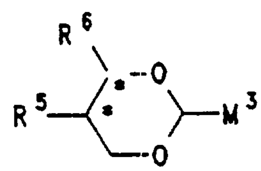

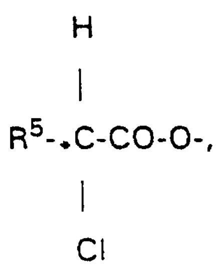

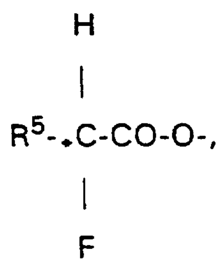

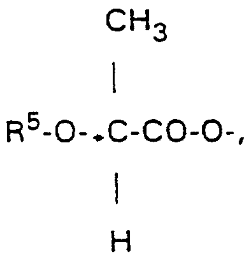

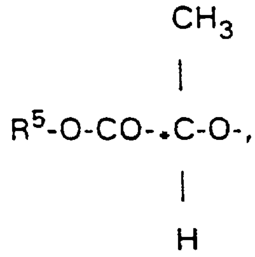

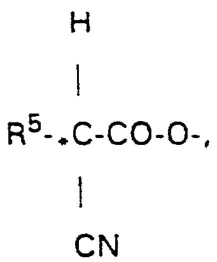

$R^2$, $R^3$, $R^5$, $R^6$, $R^7$, $R^8$ sind unabhängig voneinander H oder ein geradkettiger oder verzweigter Alkylrest mit 1 bis 16 C-Atomen, wobei $R^5$ und $R^6$ zusammen auch $-(CH_2)_4-$ oder $-(CH_2)_5-$ sein können, wenn sie als Substituenten an ein Dioxolan-System gebunden sind,

$R^4$ ist ein geradkettiger oder verzweigter Alkylrest mit 1 bis 16 C-Atomen,

$A^1$, $A^2$, $A^3$ sind gleich oder verschieden 1,4-Phenylen, Pyrazin-2,5-diyl, Pyridin-2, 5-diyl, Pyrimidin-2,5-diyl, trans-1,4-Cyclohexylen, (1,3,4)-Thiadiazol-2,5-diyl oder Naphthalin-2,6-diyl, wobei $A^1$ und $A^2$ und $A^3$ nicht gleichzeitig unsubstituiertes 1,4-Phenylen sein können, wenn $M^1$ oder $M^2$ -COO- ist,

$M^1$, $M^2$ sind gleich oder verschieden -O-, -CO-O-, -O-CO-, $-CH_2-O-$, $-O-CH_2-$, $-CH_2-CH_2-$, -CH = CH- oder -C ≡ C-,

$M^3$ ist $-CH_2-O-$, $-O-CH_2-$, -CO-O-, -O-CO- oder eine Einfachbindung;

4. Chirale Oxiranylmethyl-ether nach Anspruch 1, **dadurch gekennzeichnet, daß**

$R^1$ ein Alkylrest mit 1 bis 16 C-Atomen ist, wobei eine oder zwei nicht benachbarte $-CH_2$-Gruppe durch -O-, Δ oder -CH=CH- ersetzt sein können, oder die chiralen Grupp

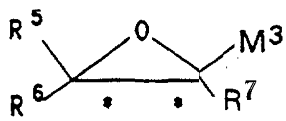

ist,

$R^2$, $R^3$, $R^5$, $R^6$, $R^7$ unabhängig voneinander H oder ein Alkylrest mit 1 bis 16 C-Atomen ist,

$R^4$ ist ein geradkettiger oder verzweigter Alkylrest mit 1 bis 16 C-Atomen

$M^3$ gleich $-CH_2-O-$ oder -CO-O- ist,

und die Gruppierung $(-A^1)_a(-M^1)_b(-A^2)_c(-M^2)_d(-A^3)_e$- folgende Bedeutung hat:

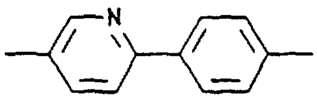 , 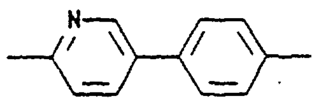 , 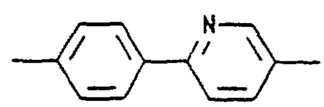 ,

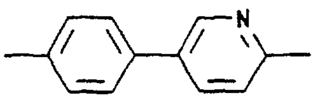 , 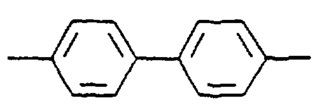 , 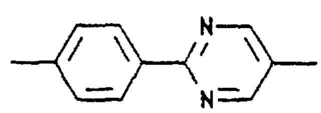 ,

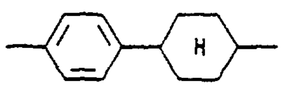 , 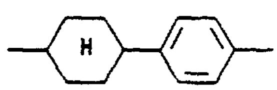 , 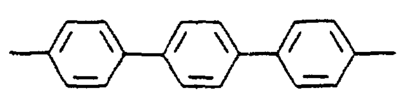 ,

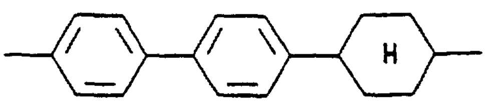 , 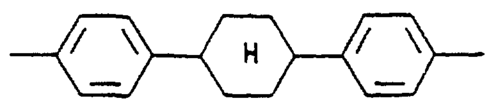 ,

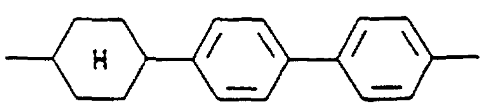 , 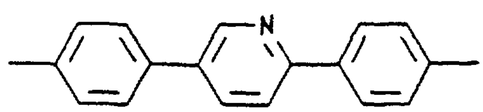 ,

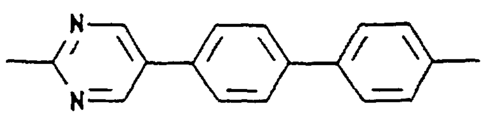 , 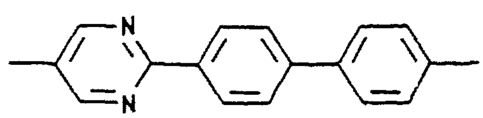 ,

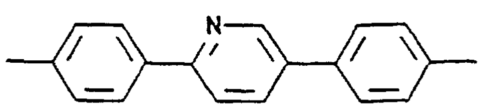 , 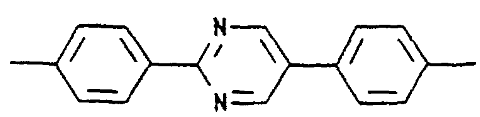 ,

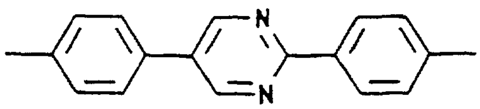 , 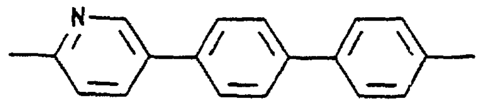 ,

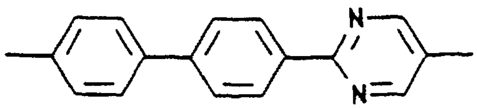 , 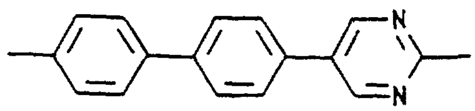 ,

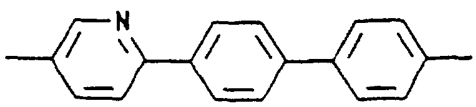 , 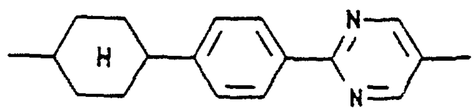 ,

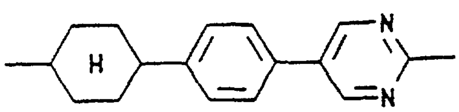 , 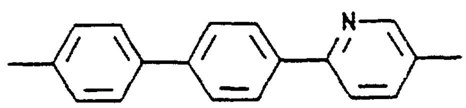 ,

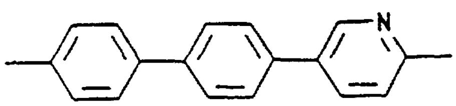 , 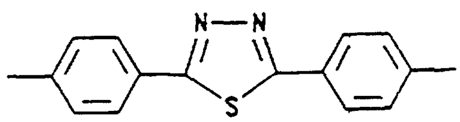 ,

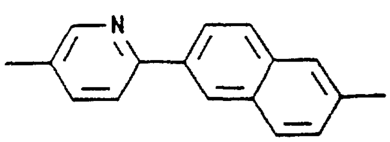 , 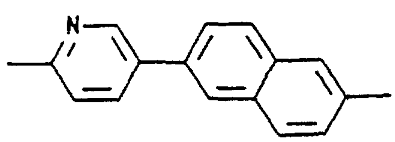 ,

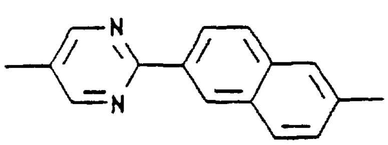 , 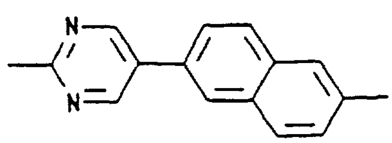 ,

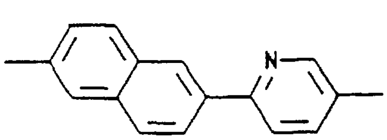 , 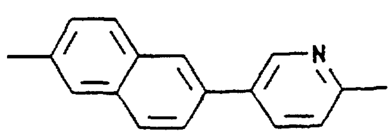 ,

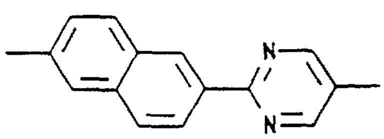 , 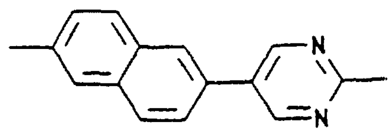 ,

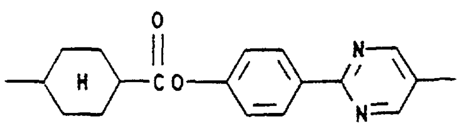 , 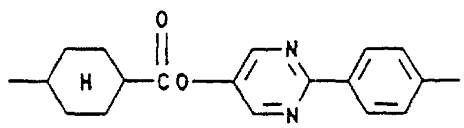 ,

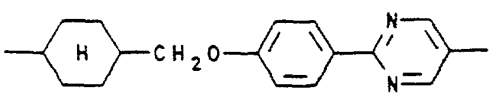 , 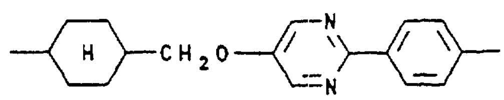

5. Flüssigkristallmischung, **gekennzeichnet durch** einen Gehalt von 0,01 bis 60 Gew.-% an mindestens einem Oxiranmethyl-ether der Formel (I),

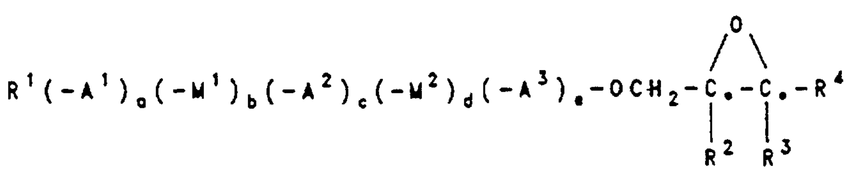 (I)

in der die Symbole und Indices folgende Bedeutung haben:

* ist ein chirales Zentrum

$R^1$ ist ein geradkettiger oder verzweigter Alkylrest mit 1 bis 16 C-Atomen (mit oder ohne asymmetrisches C-Atom), wobei auch eine oder mehrere nicht benachbarte $-CH_2$-Gruppen **durch** -O-, -S-, -CO-, -CO-O-, -O-CO-, -CO-S-, -S-CO-, -O-CO-O-, -CH = CH-, -C ≡ C-, oder Δ ersetzt sein können und/oder ein oder mehrere H-Atome des Alkylrestes **durch** F, Cl, Br oder CN substituiert sein können, oder eine der nachfolgenden chiralen Gruppen:

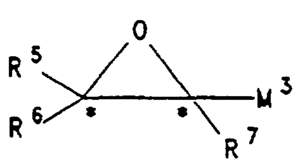

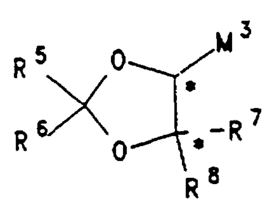

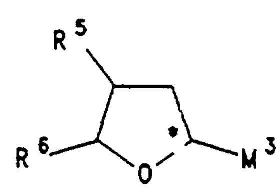

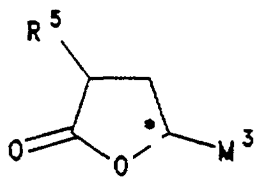

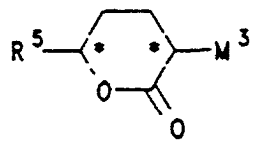

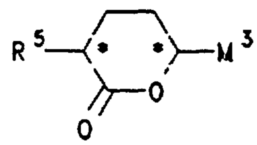

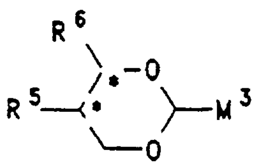

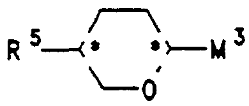

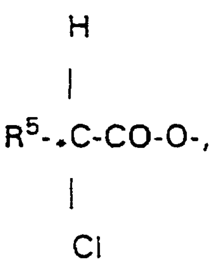

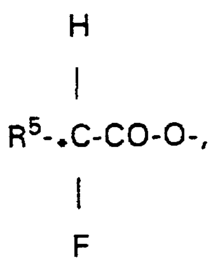

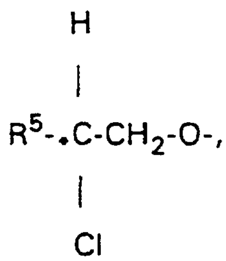

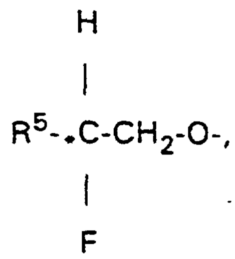

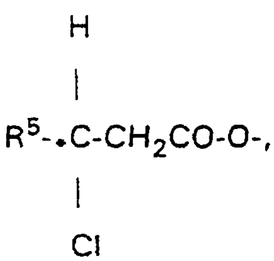

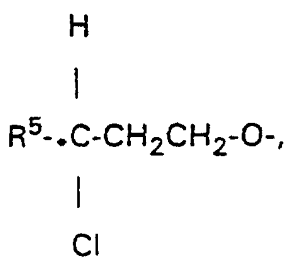

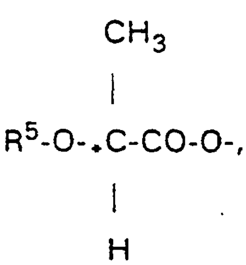

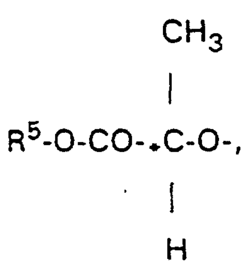

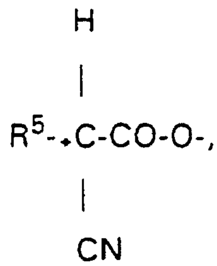,
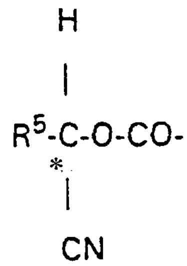

$R^2$, $R^3$, $R^4$, $R^5$, $R^6$, $R^7$, $R^8$ sind unabhängig voneinander H oder ein geradkettiger oder verzweigter Alkylrest mit 1 bis 16 C-Atomen, oder $R^5$ und $R^6$ können zusammen auch $-(CH_2)_4-$ oder $-(CH_2)_5-$ sein, wenn sie als Substituenten an ein Dioxolan-System gebunden sind,

$A^1$, $A^2$, $A^3$ sind gleich oder verschieden 1,4-Phenylen, wobei ein oder zwei H-Atome **durch** F, Cl und/oder CN ersetzt sein können, Pyrazin-2,5-diyl, Pyridazin-3,6-diyl, Pyridin-2,5-diyl, Pyrimidin-2,5-diyl, trans-1,4-Cyclohexylen, bei dem ein oder zwei H-Atome **durch** CN und/oder $CH_3$ ersetzt sein können, (1,3,4)-Thiadiazol-2,5-diyl, 1,3-Dioxan-2,5-diyl, 1,3-Dithian-2,5-diyl, 1,3-Thiazol-2,4-diyl, 1,3-Thiazol-2,5-diyl, Thiophen-2,4-diyl, Thiophen-2,5-diyl, Piperazin-1,4-diyl, Piperazin-2,5-diyl, Naphthalin-2,6-diyl, Bicyclo[2.2.2]octan-1,4-diyl oder 1,3-Dioxaborinan-2,5-diyl, wobei $A^1$ und $A^2$ und $A^3$ nicht gleichzeitig unsubstituiertes 1,4-Phenylen sein können, wenn $M^1$ oder $M^2$ -COO- ist,

$M^1$, $M^2$ sind gleich oder verschieden, -O-, -S-, -CO-, -CO-O-, -O-CO-, -CO-S-, -S-CO-, -O-CO-O-, $-CH_2-O-$, $-O-CH_2-$, $-CH_2CH_2-$, -CH=CH- oder -C≡C-,

$M^3$ ist $-CH_2-O-$, $-O-CH_2-$, -CO-O-, -O-CO- oder eine Einfachbindung,

a, b, c, d, e sind null oder eins, unter der Bedingung, daß die Summe a + c + e 1, 2 oder 3 ist,

ausgenommen sind die Verbindungen, in denen die Gruppe $(-A^1)_a(-M^1)_b(-A^2)_c(-M^2)_d(-A^3)_e-$ folgende Bedeutung hat:

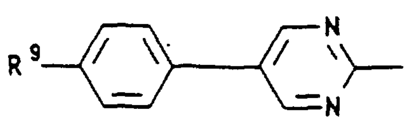,
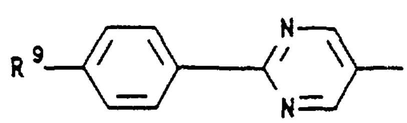,

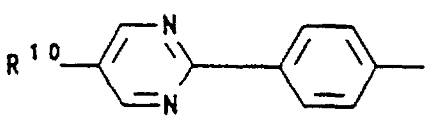,
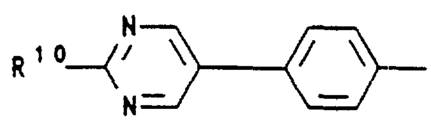,

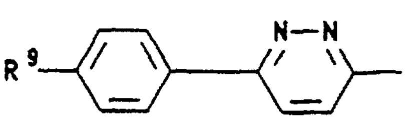,
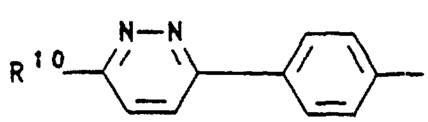,

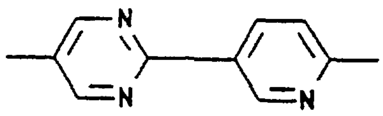 , 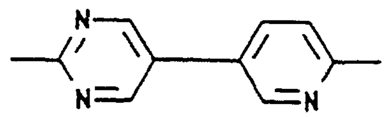 ,

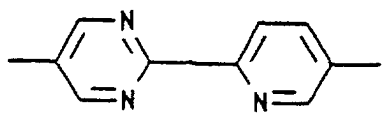 , 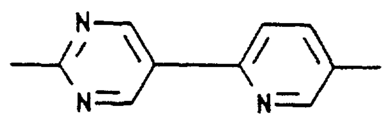 ,

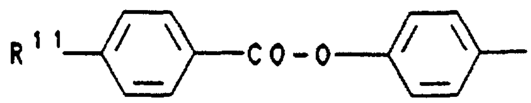

wobei

$R^9$ geradkettiges oder verzweigtes ($C_3$-$C_{12}$)-Alkenyl mit terminaler Doppelbindung bedeutet, in dem eine oder zwei nicht benachbarte $CH_2$-Gruppen **durch** O- und/oder S-Atome ersetzt sein können,

$R^{10}$ geradkettiges oder verzweigtes Alkyl mit 1-12 C-Atomen bedeutet, wobei eine oder zwei nicht benachbarte $CH_2$-Gruppen **durch** O und/oder S ersetzt sein können, und

$R^{11}$ eine Alkoxygruppe mit 5-12 C-Atomen darstellt

oder

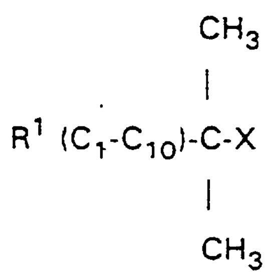

bedeutet, wobei

X geradkettiges oder verzweigtes Alkylen mit 1 bis 16 C-Atomen, bei dem auch ein oder zwei nicht-benachbarte -$CH_2$-Gruppen **durch** -O-, -S-, -O-CO-, -CO-O-, CO oder -O-CO-O- ersetzt sein können.

**6.** Flüssigkristallmischungen, **gekennzeichnet durch** einen Gehalt von 0,01 bis 60 Gew.-% an mindestens einem Oxiranylmethyl-ether nach Anspruch 1.

**7.** Ferroelektrische Flüssigkristallmischung, **gekennzeichnet durch** einen Gehalt von 0,01 bis 60 Gew.-% an mindestens einem Oxiranylmethyl-ether nach Anspruch 2.

**8.** Ferroelektrische Flüssigkristallmischung, **gekennzeichnet durch** einen Gehalt von 0,01 bis 60 Gew.-% an mindestens einem Oxiranylmethyl-ether nach Anspruch 3.

**9.** Ferroelektrische Flüssigkristallmischung, **gekennzeichnet durch** einen Gehalt von 0,01 bis 60 Gew.-% an mindestens einem Oxiranylmethyl-ether nach Anspruch 4.

**10.** Elektrooptische oder vollständig optische Bauelemente, die eine Flüssigkristallmischung nach Anspruch 5, 6, 7 oder 8 enthalten.

## Claims

**1.** Chiral oxiranylmethyl ethers of the general formula (I)

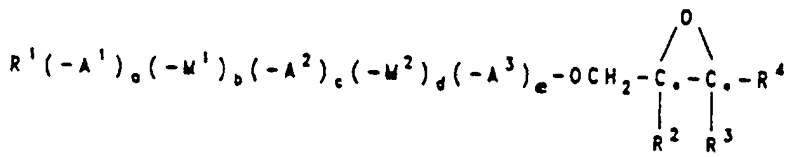

(I)

in which the symbols and indices have the following meanings:

* is the center of chirality,

$R^1$ is a straight-chain or branched alkyl radical having 1 to 16 carbon atoms (with or without an asymmetric carbon atom), it also being possible for one or more non-adjacent $-CH_2-$ groups to be replaced by -O-, -S-, -CO-, -CO-O-, -O-CO-, -CO-S-, -S-CO-, -O-CO-O-, -CH=CH-, -C≡C- or △, and/or for one or more H atoms of the alkyl radical to be substituted by F, Cl, Br or CN, or is one of the following chiral groups:

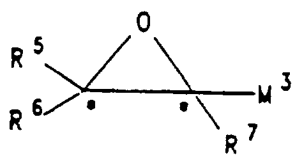
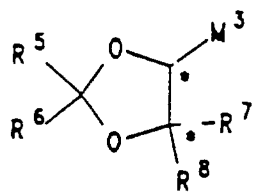
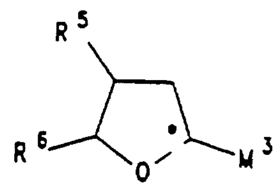

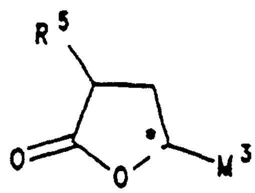
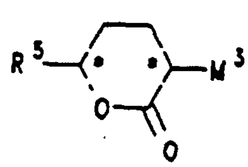
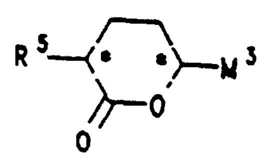

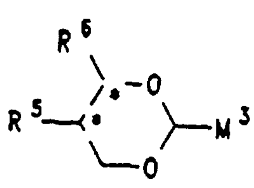
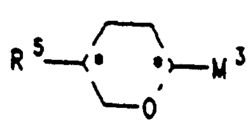

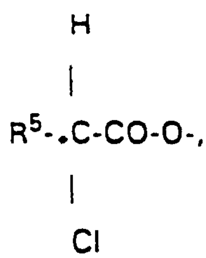 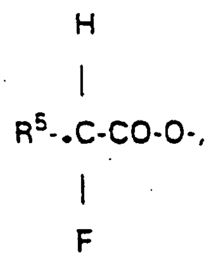 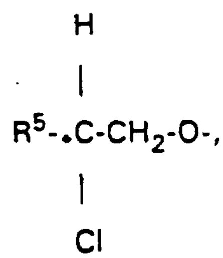 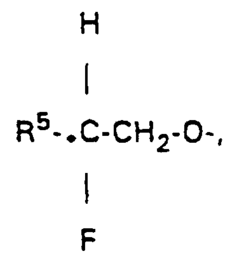

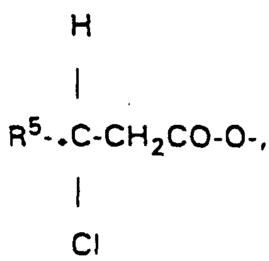 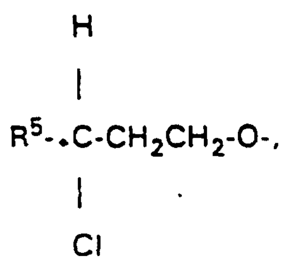

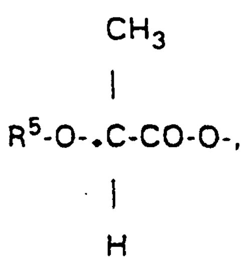 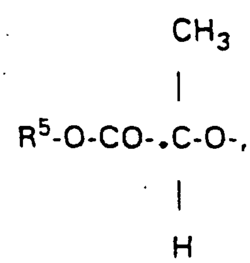

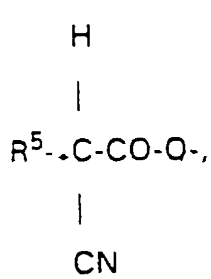 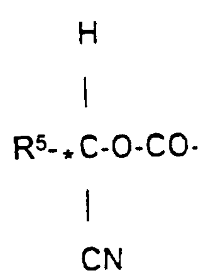

$R^2$, $R^3$, $R^5$, $R^6$, $R^7$ and $R^8$, independently of one another, are H or a straight-chain or branched alkyl radical

having 1 to 16 carbon atoms, or $R^5$ and $R^6$ together may alternatively be $-(CH_2)_4-$ or $-(CH_2)_5-$ if they are bonded as substituents to a dioxolane system,

$R^4$ is a straight-chain or branched alkyl radical having 1 to 16 carbon atoms,

$A^1$, $A^2$ and $A^3$ are identical or different and are 1,4-phenylene, in which one or two H atoms may be replaced by F, Cl and/or CN, pyrazine-2,5-diyl, pyridazine-3,6-diyl, pyridine-2,5-diyl, pyrimidine-2,5-diyl, trans-1,4-cyclohexylene, in which one or two H atoms may be replaced by CN and/or $CH_3$, 1,3,4-thiadiazole-2,5-diyl, 1,3-dioxane-2,5-diyl, 1,3-dithiane-2,5-diyl, 1,3-thiazole-2,4-diyl, 1,3-thiazole-2,5-diyl, thiophene-2,4-diyl, thiophene-2,5-diyl, piperazine-1,4-diyl, piperazine-2,5-diyl, naphthalene-2,6-diyl, bicyclo[2.2.2]octane-1,4-diyl or 1,3-dioxaborinane-2,5-diyl, where $A^1$ and $A^2$ and $A^3$ cannot simultaneously be unsubstituted 1,4-phenylene if $M^1$ or $M^2$ is -COO-,

$M^1$ and $M^2$ are identical or different and are -O-, -S-, -CO-, -CO-O-, -O-CO-, -CO-S-, -S-CO-, -O-CO-O-, $-CH_2-O-$, $-O-CH_2-$, $-CH_2CH_2-$, -CH=CH- or -C≡C-,

$M^3$ is $-CH_2-O-$, $-O-CH_2-$, -CO-O-, -O-CO- or a single bond,

a, b, c, d and e are zero or one, with the proviso that the sum a+c+e is 1,2 or 3,

with the exception of the compounds in which the $(-A^1)_a(-M^1)_b(-A^2)_c(-M^2)_d(-A^3)_e-$ group has the following meanings:

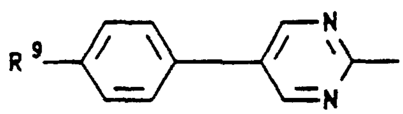 , 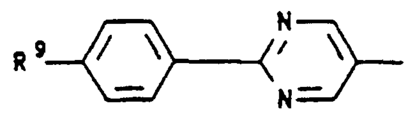 ,

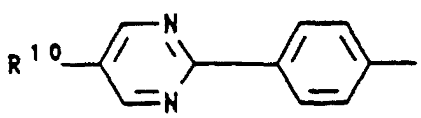 , 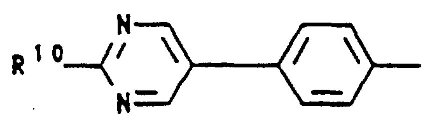 ,

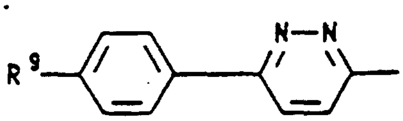 , 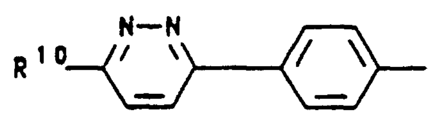 ,

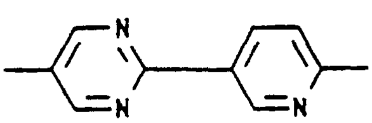 , 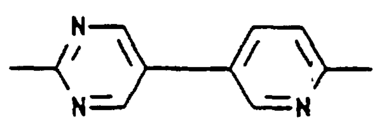 ,

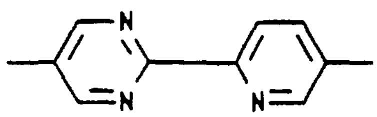 , 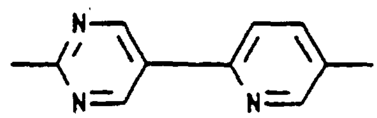 ,

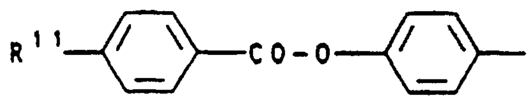

where

$R^9$ is straight-chain or branched ($C_3$-$C_{12}$)-alkenyl containing a terminal double bond, in which one or two non-adjacent $CH_2$ groups may be replaced by O and/or S atoms,

$R^{10}$ is straight-chain or branched alkyl having 1-12 carbon atoms, in which one or two non-adjacent $CH_2$ groups may be replaced by O and/or S, and

$R^{11}$ is an alkoxy group having 5-12 carbon atoms,

or

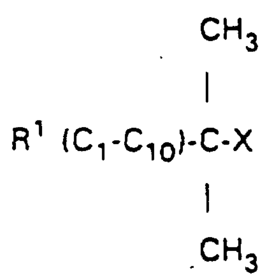 ,

where

X is straight-chain or branched alkylene having 1 to 16 carbon atoms, in which one or two non-adjacent -$CH_2$- groups may also be replaced by -O-, -S-, -O-CO-, -CO-O-, -CO- or -O-CO-O-.

**2.** Chiral oxiranylmethyl ethers as claimed in claim 1, wherein the symbols and indices in the general formula (I) have the following meanings:

$R^1$ is a straight-chain or branched alkyl radical having 1 to 16 carbon atoms (with or without an asymmetric carbon atom), it also being possible for one or more -$CH_2$- groups to be replaced by -O-, -S-, -CO-, -CO-O-, -O-CO-, -O-CO-O-, -CH=CH-, -C≡C- or Δ, or is one of the following chiral groups:

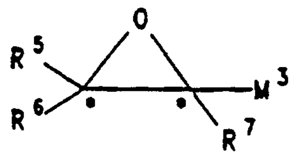
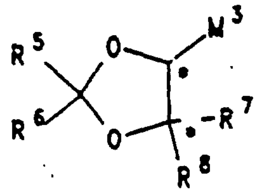
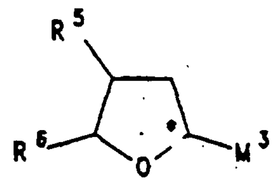

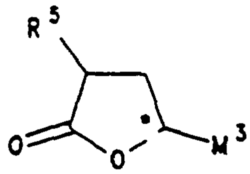
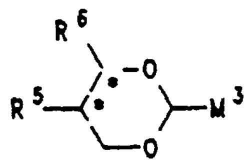

| | | | |
|---|---|---|---|
| H | H | H | H |
| \| | \| | \| | \| |
| $R^5$-*C-CO-O-, | $R^5$-*C-CO-O-, | $R^5$-*C-$CH_2$-O-, | $R^5$-*C-$CH_2$-O-, |
| \| | \| | \| | \| |
| Cl | F | Cl | F |

| | |
|---|---|
| H | H |
| \| | \| |
| $R^5$-*C-$CH_2$CO-O-, | $R^5$-*C-$CH_2CH_2$-O-, |
| \| | \| |
| Cl | Cl |

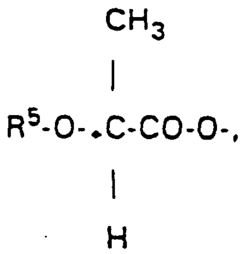, 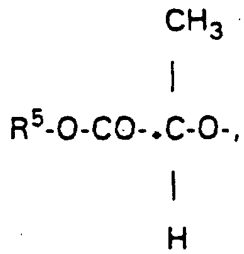,

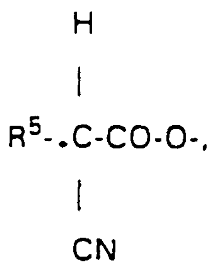, 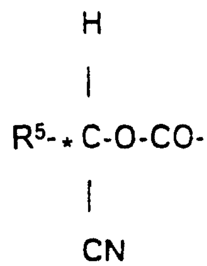

$R^2$, $R^3$, $R^5$, $R^6$, $R^7$ and $R^8$, independently of one another, are H or a straight-chain or branched alkyl radical having 1 to 16 carbon atoms, where $R^5$ and $R^6$ together may alternatively be $-(CH_2)_4-$ or $-(CH_2)_5-$ if they are bonded as substituents to a dioxolane system,

$R^4$ is a straight-chain or branched alkyl radical having 1 to 16 carbon atoms,

$A^1$, $A^2$ and $A^3$ are identical or different and are 1,4-phenylene, pyrazine-2,5-diyl, pyridazine-3,6-diyl, pyridine-2,5-diyl, pyrimidine-2,5-diyl, trans-1,4-cyclohexylene, 1,3,4-thiadiazole-2,5-diyl, 1,3-dioxane-2,5-diyl, naphthalene-2,6-diyl or bicyclo-[2.2.2]octane-1,4-diyl, where $A^1$ and $A^2$ and $A^3$ cannot simultaneously be unsubstituted 1,4-phenylene if $M^1$ or $M^2$ is -COO-,

$M^1$ and $M^2$ are identical or different and are -O-, -CO-, -CO-O-, -O-CO-, $-CH_2-O-$, $-O-CH_2-$, $-CH_2-CH_2-$, -CH=CH- or -C≡C-, and

$M^3$ is $-CH_2-O-$, $-O-CH_2-$, -CO-O-, -O-CO- or a single bond.

3. Chiral oxiranylmethyl ethers as claimed in claim 1, wherein the symbols and indices in the general formula (I) have the following meanings:

$R^1$ is a straight-chain or branched alkyl radical having 1 to 16 carbon atoms (with or without an asymmetric carbon atom), it also being possible for one or two $-CH_2-$ groups to be replaced by -O-, -CO-, -CO-O-, -O-CO-, -CH=CH-, or Δ, or is one of the following chiral groups:

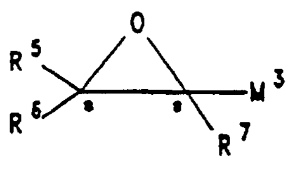
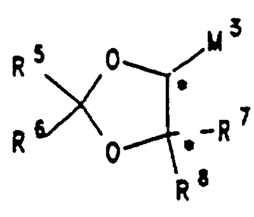
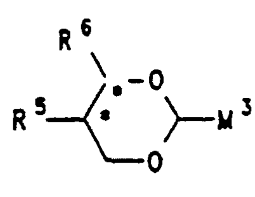

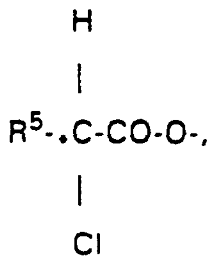

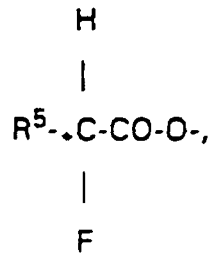

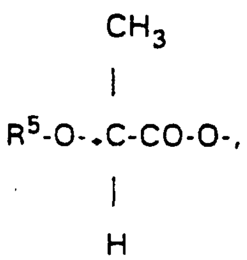

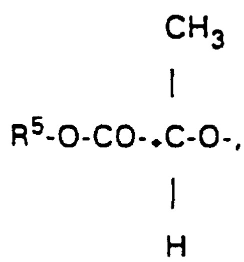

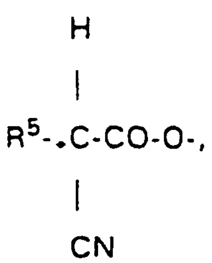

$R^2$, $R^3$, $R^5$, $R^6$, $R^7$ and $R^8$, independently of one another, are H or a straight-chain or branched alkyl radical having 1 to 16 carbon atoms, where $R^5$ and $R^6$ together may alternatively be $-(CH_2)_4-$ or $-(CH_2)_5-$ if they are bonded as substituents to a dioxolane system,

$R^4$ is a straight-chain or branched alkyl radical having 1 to 16 carbon atoms,

$A^1$, $A^2$ and $A^3$ are identical or different and are 1,4-phenylene, pyrazine-2,5-diyl, pyridine-2,5-diyl, pyrimidine-2,5-diyl, trans-1,4-cyclohexylene, 1,3,4-thiadiazole-2,5-diyl naphthalene-2,6-diyl, where $A^1$ and $A^2$ and $A^3$ cannot simultaneously be unsubstituted 1,4-phenylene if $M^1$ or $M^2$ is -COO-,

$M^1$ and $M^2$ are identical or different and are -O-, -CO-O-, -O-CO-, $-CH_2-O-$, $-O-CH_2-$, $-CH_2-CH_2-$, -CH=CH- or $-C\equiv C-$, and

$M^3$ is $-CH_2-O-$, $-O-CH_2-$, -CO-O-, -O-CO- or a single bond.

**4.** Chiral oxiranylmethyl ethers as claimed in claim 1, wherein

$R^1$ is an alkyl radical having 1 to 16 carbon atoms, it being possible for one or two non-adjacent $-CH_2-$ groups to be replaced by -O-, Δ or -CH=CH-, or is the chiral group

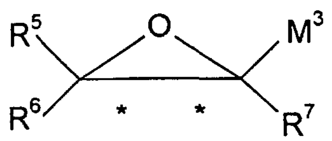

$R^2$, $R^3$, $R^5$, $R^6$ and $R^7$, independently of one another, are H or an alkyl radical having 1 to 16 carbon atoms,

$R^4$ is a straight-chain or branched alkyl radical having 1 to 16 carbon atoms,

$M^3$ is $-CH_2-O-$ or -CO-O-,

and the $(-A^1)_a(-M^1)_b(-A^2)_c(-M^2)_d(-A^3)_e-$ group has the following meanings:

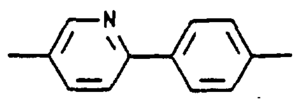 , 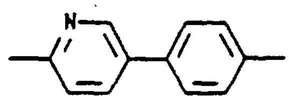 , 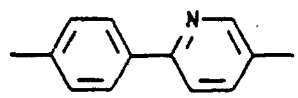 ,

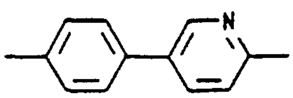 , 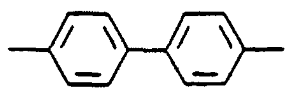 , 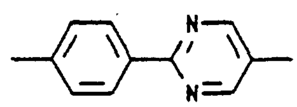 ,

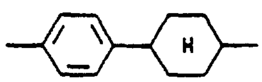 , 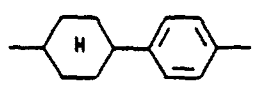 , 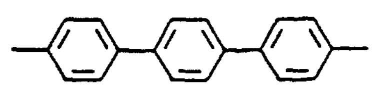 ,

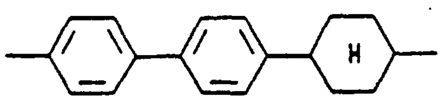 , 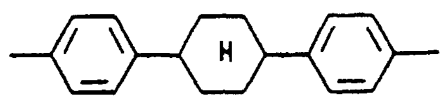 ,

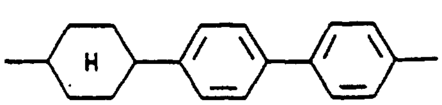 , 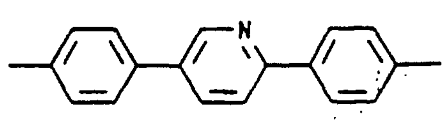 ,

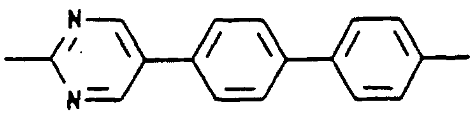 , 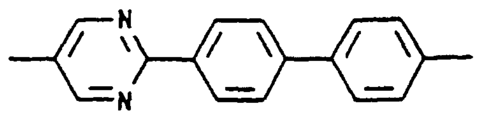 ,

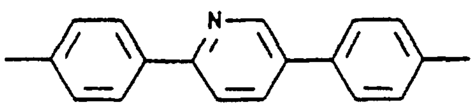 , 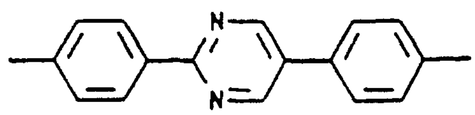 ,

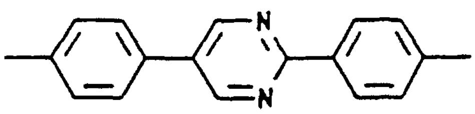 , 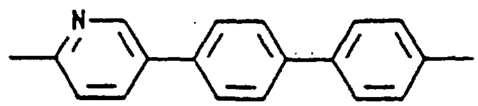 ,

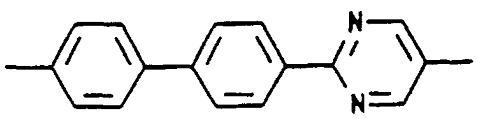 , 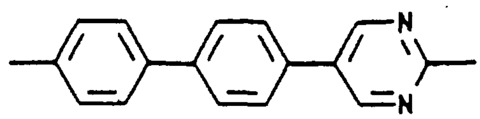 ,

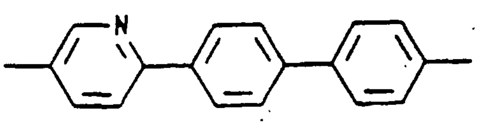 , 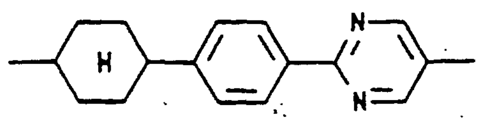 ,

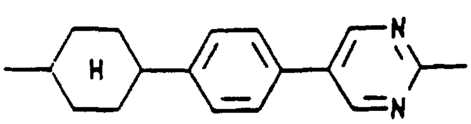 , 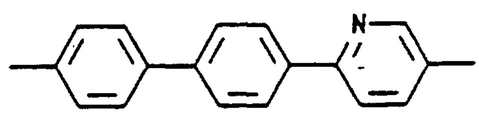 ,

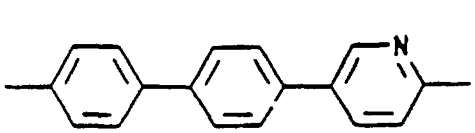 , 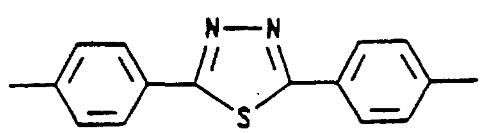 ,

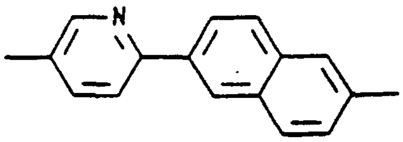 , 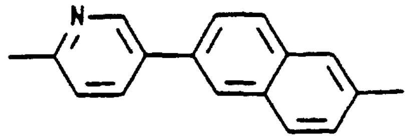 ,

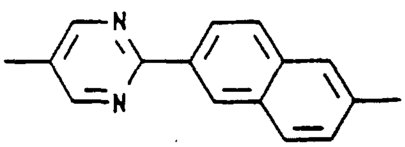 , 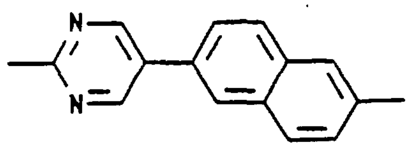 ,

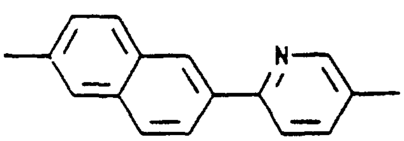 , 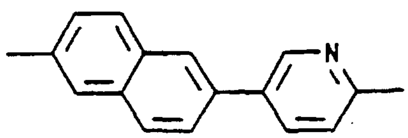 ,

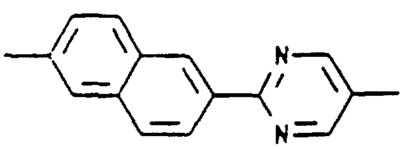 , 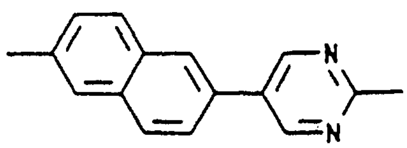 ,

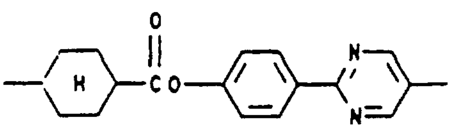 , 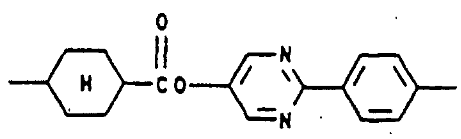 ,

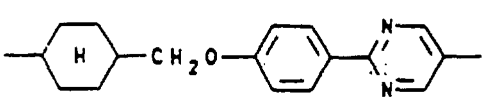 , 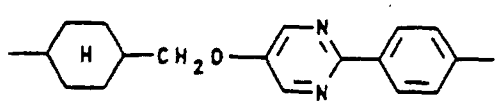

**5.** A liquid-crystal mixture which contains from 0.01 to 60% by weight of at least one oxiranylmethyl ether of the formula (I)

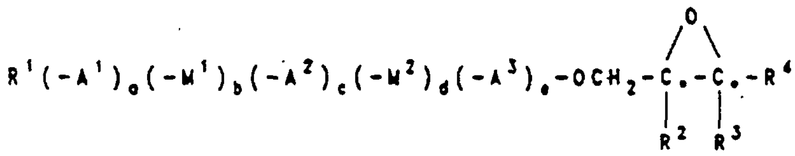 (I)

in which the symbols and indices have the following meanings:

- * is a center of chirality,
- $R^1$ is a straight-chain or branched alkyl radical having 1 to 16 carbon atoms (with or without an asymmetric carbon atom), it also being possible for one or more non-adjacent $-CH_2-$ groups to be replaced by -O-, -S-, -CO-, -CO-O-, -O-CO-, -CO-S-, -S-CO-, -O-CO-O-, -CH=CH-, -C≡C- or Δ, and/or for one or more H atoms of the alkyl radical to be substituted by F, Cl, Br or CN, or is one of the following chiral groups:

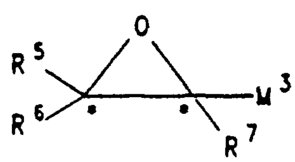
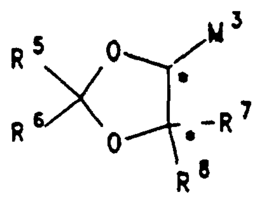
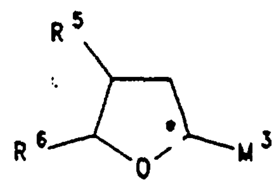

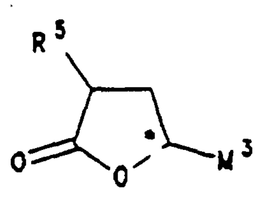
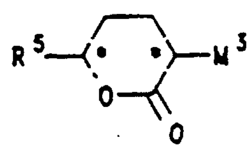
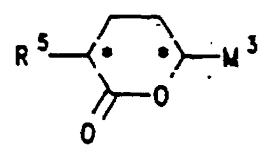

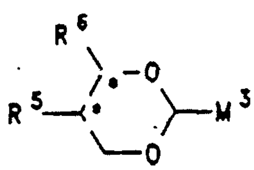
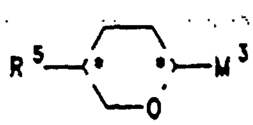

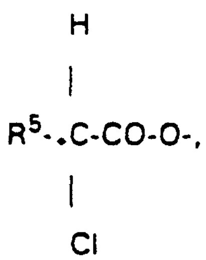 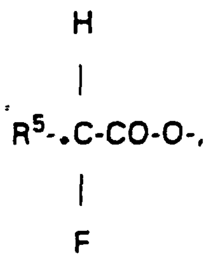 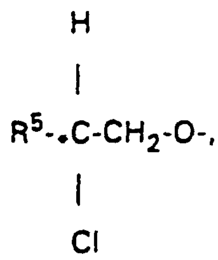 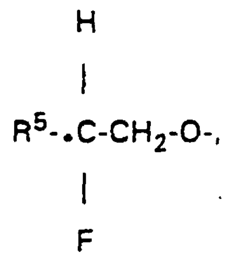

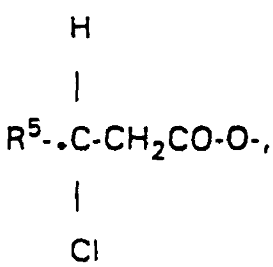 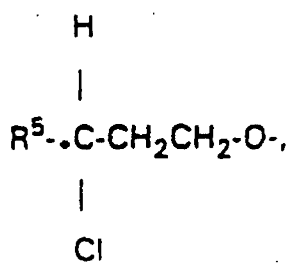

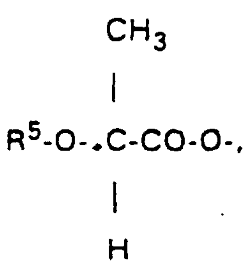 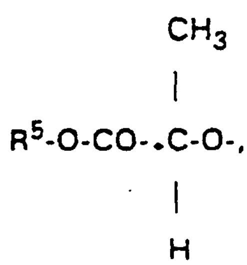

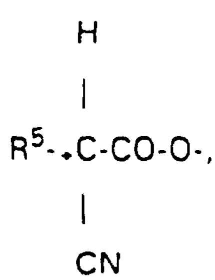 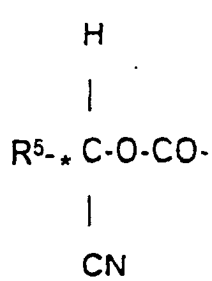

$R^2$, $R^3$, $R^4$, $R^5$, $R^6$, $R^7$ and $R^8$, independently of one another, are H or a straight-chain or branched alkyl radical having 1 to 16 carbon atoms, or $R^5$ and $R^6$ together may alternatively be $-(CH_2)_4-$ or $-(CH_2)_5-$ if they are bonded

as substituents to a dioxolane system,

$A^1$, $A^2$ and $A^3$ are identical or different and are 1,4-phenylene, in which one or two H atoms may be replaced by F, Cl and/or CN, pyrazine-2,5-diyl, pyridazine-3,6-diyl, pyridine-2,5-diyl, pyrimidine-2,5-diyl, trans-1,4-cyclohexylene, in which one or two H atoms may be replaced by CN and/or $CH_3$, 1,3,4-thiadiazole-2,5-diyl, 1,3-dioxane-2,5-diyl, 1,3-dithiane-2,5-diyl, 1,3-thiazole-2,4-diyl, 1,3-thiazole-2,5-diyl, thiophene-2,4-diyl, thiophene-2,5-diyl,piperazine-1,4-diyl, piperazine-2,5-diyl, naphthalene-2,6-diyl, bicycle[2.2.2]-octane-1,4-diyl or 1,3-dioxaborinane-2,5-diyl, where $A^1$ and $A^2$ and $A^3$ cannot simultaneously be unsubstituted 1,4-phenylene if $M^1$ or $M^2$ is -COO-,

$M^1$ and $M^2$ are identical or different and are -O-, -S-, -CO-, -CO-O-, -O-CO-, -CO-S-, -S-CO-, -O-CO-O-, $-CH_2-O-$, $-O-CH_2-$, $-CH_2-CH_2-$, -CH=CH- or -C≡C-,

$M^3$ is $-CH_2-O-$, $-O-CH_2-$, -CO-O-, -O-CO- or a single bond,

a, b, c, d and e are zero or one, with the proviso that the sum a+c+e is 1, 2 or 3,

with the exception of the compounds in which the $(-A^1)_a(-M^1)_b(-A^2)_c(-M^2)_d(-A^3)_e$- group has the following meanings:

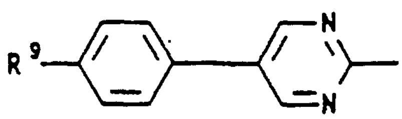 , 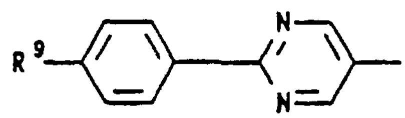 ,

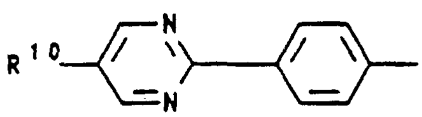 , 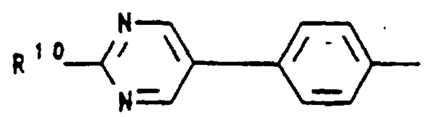 ,

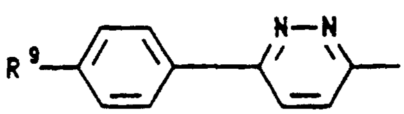 , 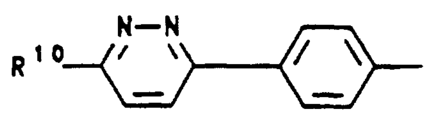 ,

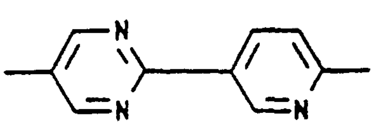 , 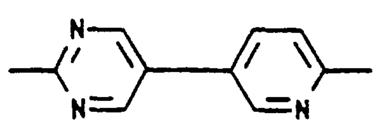 ,

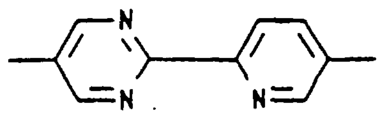 , 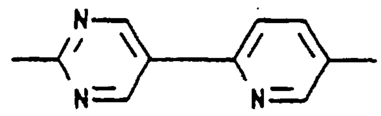 ,

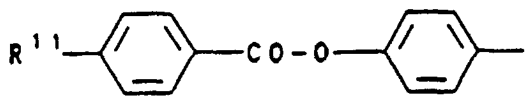

where

$R^9$ is straight-chain or branched ($C_3$-$C_{12}$)-alkenyl containing a terminal double bond, in which one or two non-adjacent $CH_2$ groups may be replaced by O and/or S atoms,

$R^{10}$ is straight-chain or branched alkyl having 1-12 carbon atoms, in which one or two non-adjacent $CH_2$ groups may be replaced by O and/or S, and

$R^{11}$ is an alkoxy group having 5-12 carbon atoms,

or

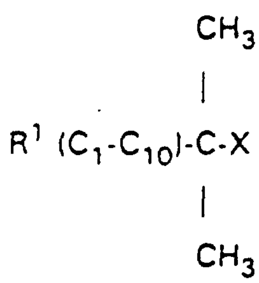 ,

where

X is straight-chain or branched alkylene having 1 to 16 carbon atoms, in which one or two non-adjacent -$CH_2$- groups may also be replaced by -O-, -S-, -O-CO-, -CO-O-, -CO- or -O-CO-O-.

**6.** Liquid-crystal mixtures which contain from 0.01 to 60% by weight of at least one oxiranylmethyl ether as claimed in claim 1.

**7.** A ferroelectric liquid-crystal mixture which contains from 0.01 to 60% by weight of at least one oxiranylmethyl ether as claimed in claim 2.

**8.** A ferroelectric liquid-crystal mixture which contains from 0.01 to 60% by weight of at least one oxiranylmethyl ether as claimed in claim 3.

**9.** A ferroelectric liquid-crystal mixture which contains from 0.01 to 60% by weight of at least one oxiranylmethyl ether as claimed in claim 4.

**10.** Electro-optical or fully optical components containing a liquid-crystal mixture as claimed in claim 5, 6, 7 or 8.

## Revendications

**1.** Oxyde d'oxiranylméthyle chiral de formule générale (I),

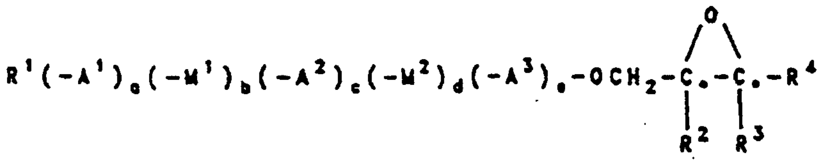 (I)

dans laquelle les symboles et indices ont la signification suivante :

| | |
|---|---|
| * | est un centre chiral |
| $R^1$ | est un groupe alkyle à chaîne linéaire ou ramifiée ayant de 1 à 16 atomes de carbone (avec ou sans atome de carbone asymétrique), dans lequel un ou plusieurs groupes $-CH_2-$ non voisins peuvent aussi être remplacés par -O-, -S-, -CO-, -CO-O-, -O-CO-, -CO-S-, -S-CO-, -O-CO-O-, -CH=CH-, -C≡C- ou Δ et/ou un ou plusieurs atomes d'hydrogène du groupe alkyle peuvent être substitués par F, Cl, Br ou CN, ou un des groupes chiraux suivants : |

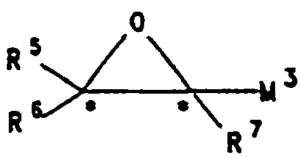
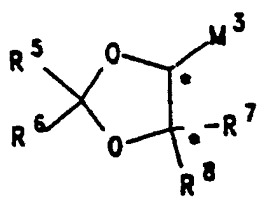
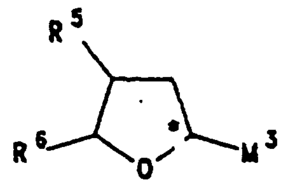

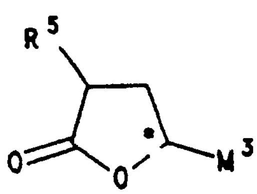
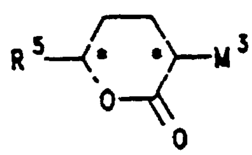
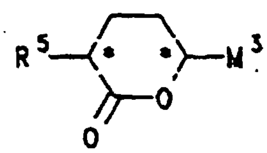

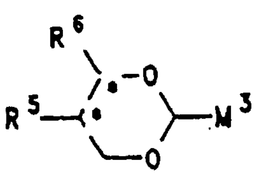
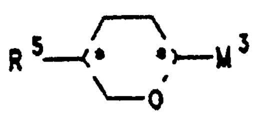

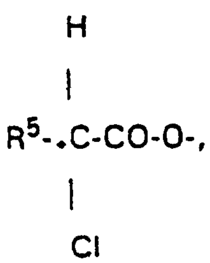
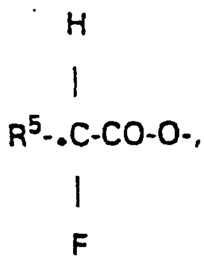
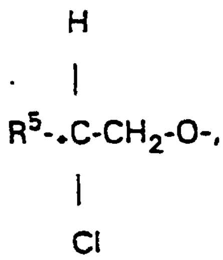
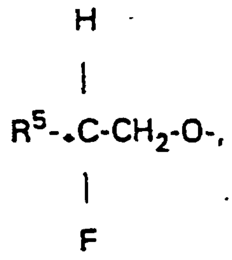

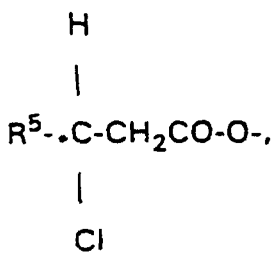
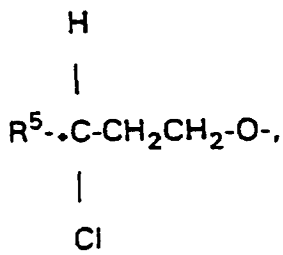

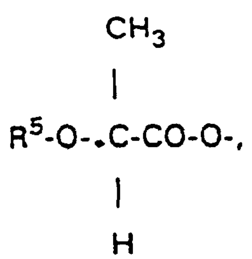
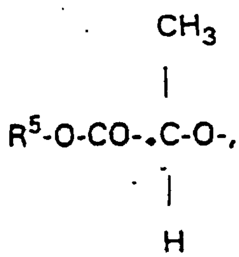

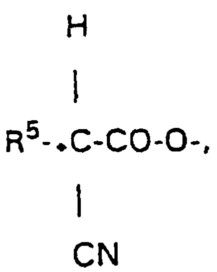
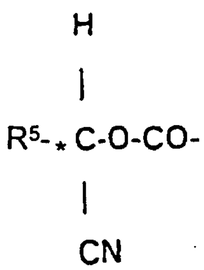

$R^2$, $R^3$, $R^5$, $R^6$, $R^7$, $R^8$ sont indépendamment les uns des autres H ou un groupe alkyle à chaîne linéaire ou ramifiée ayant de 1 à 16 atomes de carbone, ou $R^5$ et $R^6$ peuvent être ensemble aussi $-(CH_2)_4-$ ou $-(CH_2)_5-$ lorsqu'ils sont liés à un système dioxolane comme substituants,

$R^4$ est un groupe alkyle à chaîne linéaire ou ramifiée ayant de 1 à 16 atomes de carbone,

$A^1$, $A^2$, $A^3$ sont, identiques ou différents, un groupe 1,4-phénylène, dans lequel un ou deux atomes H peuvent être remplacés par F, Cl et/ou CN, pyrazin-2,5-diyle, pyridazin-3,6-diyle, pyridin-2,5-diyle, pyrimidin-2,5-diyle, trans-1,4-cyclohexylène, dans lequel un ou deux atomes H peuvent être remplacés par CN et/ou $CH_3$, (1,3,4)-thiadiazol-2,5-diyle, 1,3-dioxan-2,5-diyle, 1,3-dithian-2,5-diyle, 1,3-thiazol-2,4-diyle, 1,3-thia-

| | |
|---|---|
| | zol-2,5-diyle, thiophèn-2,4-diyle, thiophèn-2,5-diyle, pipérazin-1,4-diyle, pipérazin-2,5-diyle, naphtalèn-2,6-diyle, bicyclo-[2.2.2]octan-1,4-diyle ou 1,3-dioxa-borinan-2,5-diyle, $A^1$ et $A^2$ et $A^3$ ne pouvant pas être en même temps un groupe 1,4-phénylène non substitué, lorsque $M^1$ ou $M^2$ est -COO-, |
| $M^1$, $M^2$ | sont, identiques ou différents, -O-, -S-, -CO-, -CO-O-, -O-CO-, -CO-S-, -S-CO-, -O-CO-O-, $-CH_2-O-$, $-O-CH_2-$, $-CH_2-CH_2-$, -CH=CH- ou -C≡C-, |
| $M^3$ | est $-CH_2-O-$, $-O-CH_2-$, -CO-O-, -O-CO- ou une liaison simple, |
| a, b, c, d, e | sont zéro ou un, à la condition que la somme a + b + e soit 1, 2 ou 3, |

à l'exception des composés, dans lesquels le groupe $(-A^1)_a(-M^1)_b(-A^2)_c(-M^2)_d(-A^3)_e-$ a la signification suivante :

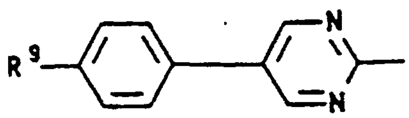 , 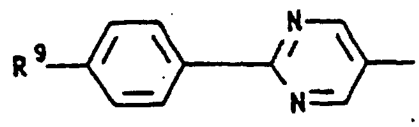 ,

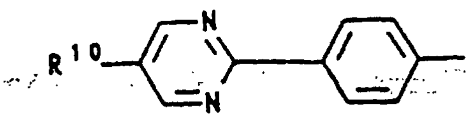 , 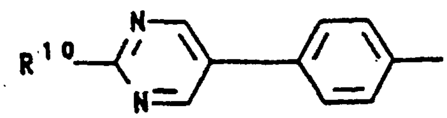 ,

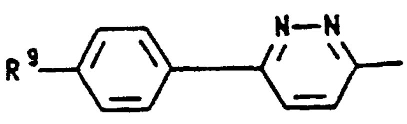 , 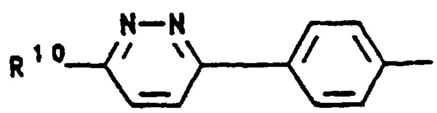 ,

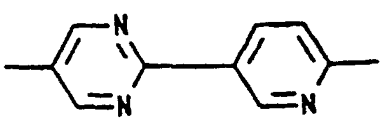 , 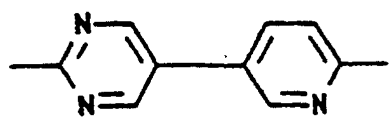 ,

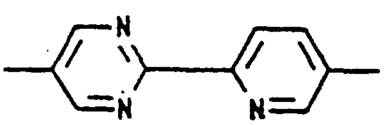 , 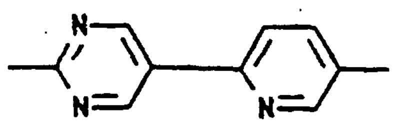 ,

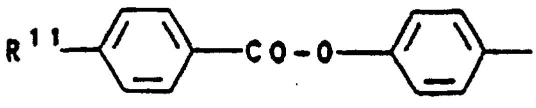

dans lesquelles

$R^9$ représente un groupe alcényle en $C_3$ à $C_{12}$ à chaîne linéaire ou ramifiée ayant une liaison double terminale, dans lequel un ou deux groupes $CH_2$ non voisins peuvent être remplacés par des atomes O et/ou S,

$R^{10}$ représente un groupe alkyle à chaîne linéaire ou ramifiée ayant de 1 à 12 atomes de carbone, dans lequel un ou deux groupes $CH_2$ non voisins peuvent être remplacés par O et/ou S, et

$R^{11}$ représente un groupe alcoxy ayant de 5 à 12 atomes de carbone

ou $R^1$ représente

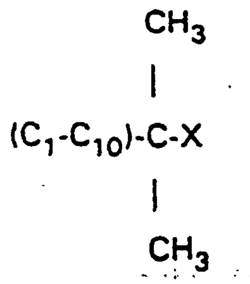

dans laquelle

X représente un groupe alkylène à chaîne linéaire ou ramifiée ayant de 1 à 16 atomes de carbone, dans lequel un ou deux groupes -CH2- non voisins peuvent aussi être remplacés par -O-, -S-, -O-CO-, -CO-O-, CO ou -O-CO-O-.

**2.** Oxyde d'oxiranylméthyle chiral selon la revendication 1, **caractérisé en ce que** les symboles et indices de la formule générale (I) ont la signification suivante :

$R^1$ est un groupe alkyle à chaîne linéaire ou ramifiée ayant de 1 à 16 atomes de carbone (avec ou sans atome de carbone asymétrique), dans lequel également un ou plusieurs groupes $-CH_2-$ peuvent être remplacés par -O-, -S-, -CO-, -CO-O-, -O-CO-, -O-CO-O-, -CH=CH-, -C≡C-, ou Δ, ou un des groupes chiraux suivants :

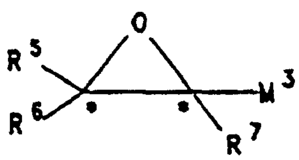

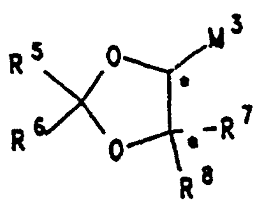

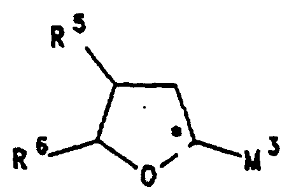

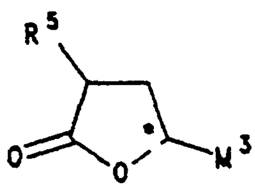

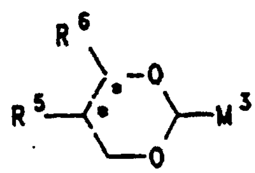

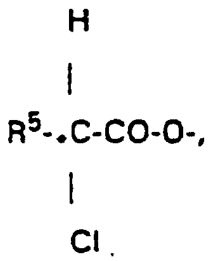 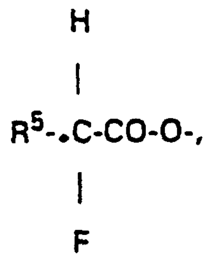 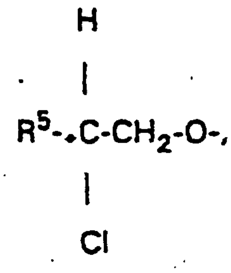 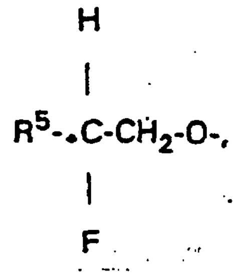

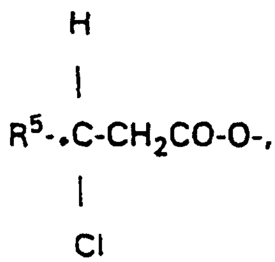 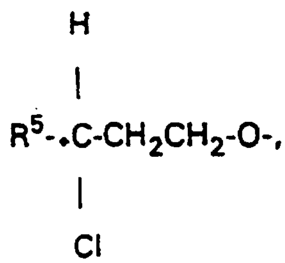

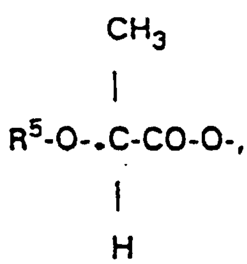 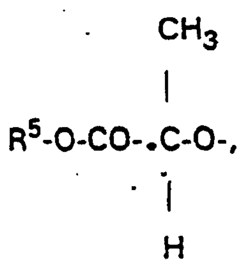

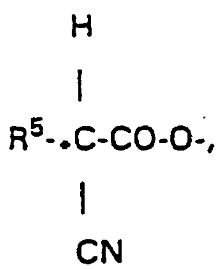 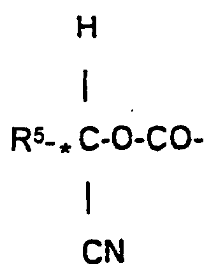

$R^2$, $R^3$, $R^5$, $R^6$, $R^7$, $R^8$ sont indépendamment les uns des autres H ou un groupe alkyle à chaîne linéaire ou ramifiée ayant de 1 à 16 atomes de carbone, $R^5$ et $R^6$ pouvant être ensemble aussi $-(CH_2)_4-$ ou $-(CH_2)_5-$ lorsqu'ils sont liés à un système dioxolane comme substituants,

$R^4$ est un groupe alkyle à chaîne linéaire ou ramifiée ayant de 1 à 16 atomes de carbone,

$A^1$, $A^2$, $A^3$ sont, identiques ou différents, un groupe 1,4-phénylène, pyrazin-2,5-diyle, pyridazin-3,6-diyle, pyridin-2,5-diyle, pyrimidin-2,5-diyle, trans-1,4-cyclohexylène, (1,3,4)-thiadiazol-2,5-diyle, 1,3-dioxan-2,5-diyle, naphtalèn-2,6-diyle, bicyclo[2.2.2]octan-1,4-diyle, $A^1$ et $A^2$ et $A^3$ ne pouvant pas être en même temps un groupe 1,4-phénylène non substitué, lorsque $M^1$ ou $M^2$ est -COO-,

| | |
|---|---|
| $M^1$, $M^2$ | sont, identiques ou différents, -O-, -CO-, -CO-O-, -O-CO-, $-CH_2$-O-, -O-$CH_2$-, $-CH_2$-$CH_2$-, -CH=CH- ou -C≡C-, |
| $M^3$ | est $-CH_2$-O-, -O-$CH_2$-, -CO-O-, -O-CO- ou une liaison simple. |

**3.** Oxyde d'oxiranylméthyle chiral selon la revendication 1, **caractérisé en ce que** les symboles et indices de la formule générale (I) ont la signification suivante :

| | |
|---|---|
| $R^1$ | est un groupe alkyle à chaîne linéaire ou ramifiée ayant de 1 à 16 atomes de carbone (avec ou sans atome de carbone asymétrique), dans lequel un ou deux groupes $-CH_2$- peuvent aussi être remplacés par -O-, -CO-, -CO-O-, -O-CO-, -CH=CH-, ou Δ, ou un des groupes chiraux suivants : |

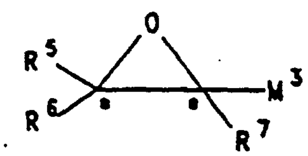
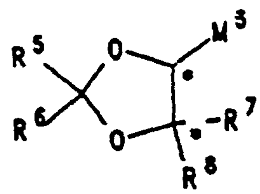
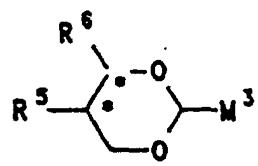

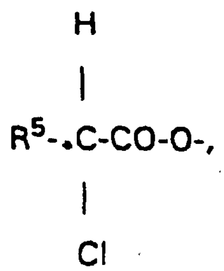
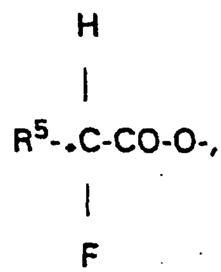

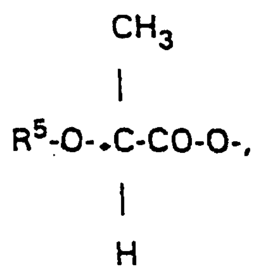
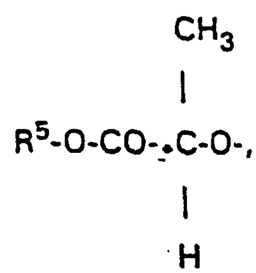

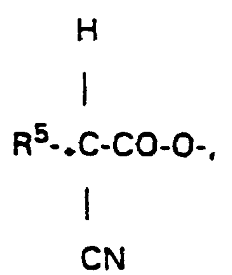

$R^2$, $R^3$, $R^5$, $R^6$, $R^7$, $R^8$ sont indépendamment les uns des autres H ou un groupe alkyle à chaîne linéaire ou ramifiée ayant de 1 à 16 atomes de carbone, $R^5$ et $R^6$ pouvant être ensemble également $-(CH_2)_4-$ ou $-(CH_2)_5-$ lorsqu'ils sont liés à un système dioxolane comme substituants,

$R^4$ est un groupe alkyle à chaîne linéaire ou ramifiée ayant de 1 à 16 atomes de carbone,

$A^1$, $A^2$, $A^3$ sont, identiques ou différents, un groupe 1,4-phénylène, pyrazin-2,5-diyle, pyridazin-3,6-diyle, pyridin-2,5-diyle, pyrimidin-2,5-diyle, trans-1,4-cyclohexylène, (1,3,4)-thiadiazol-2,5-diyle ou naphtalèn-2,6-diyle, $A^1$ et $A^2$ et $A^3$ ne pouvant pas être en même temps un groupe 1,4-phénylène non substitué, lorsque $M^1$ ou $M^2$ est -COO-,

$M^1$, $M^2$ sont, identiques ou différents, -O-, -CO-O-, -O-CO-, $-CH_2-O-$, $-O-CH_2-$, $-CH_2-CH_2-$, -CH=CH- ou -C≡C-,

$M^3$ est $-CH_2-O-$, $-O-CH_2-$, -CO-O-, -O-CO- ou une liaison simple.

**4.** Oxyde d'oxiranylméthyle selon la revendication 1, **caractérisé en ce que**,

$R^1$ est un groupe alkyle ayant de 1 à 16 atomes de carbone, dans lequel un ou deux groupes $-CH_2-$ non voisins peuvent être remplacés par -O-, △ ou -CH=CH-, ou est le groupe chiral

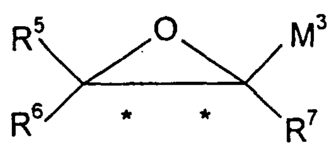,

$R^2$, $R^3$, $R^5$,$R^6$,$R^7$ sont indépendamment les uns des autres H ou un groupe alkyle ayant de 1 à 16 atomes de carbone,

$R^4$ est un groupe alkyle à chaîne linéaire ou ramifiée ayant de 1 à 16 atomes de carbone,

$M^3$ est égal à $-CH_2-O-$ ou -CO-O-,

et le groupement $-(A^1)_a(-M^1)_b(-A^2)_c(-M^2)_d(-A^3)_e-$ a la signification suivante :

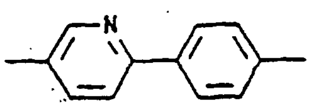 , 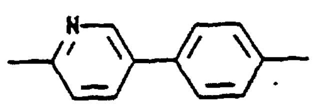 , 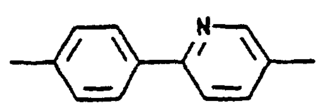 ,

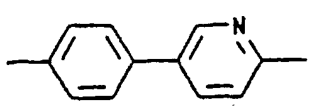 , 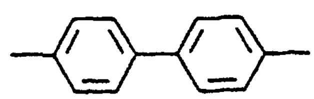 , 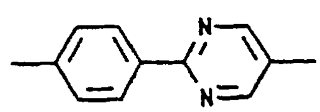 ,

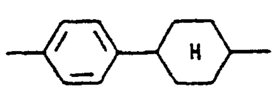 , 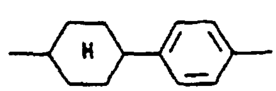 , 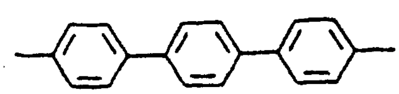 .

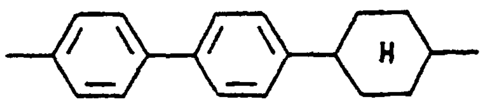 , 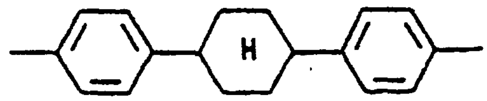 ,

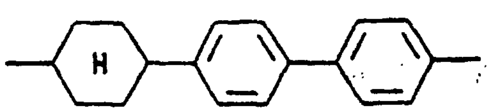 , 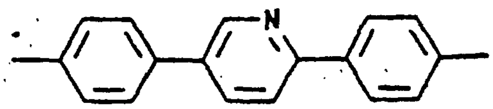 ,

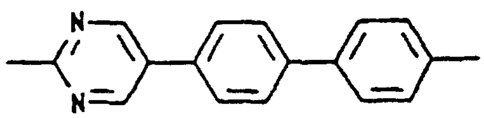 , 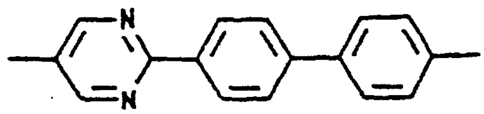 ,

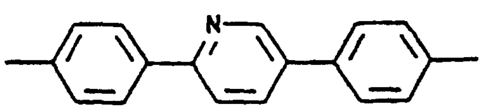 , 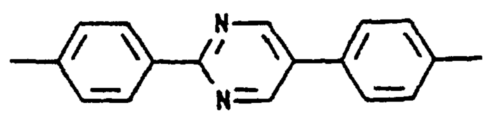 ,

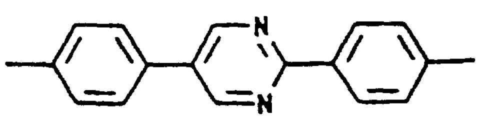 , 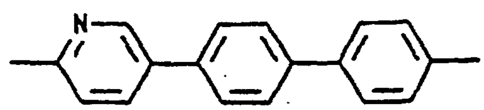 ,

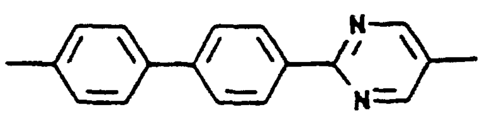 , 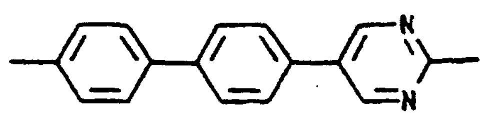 ,

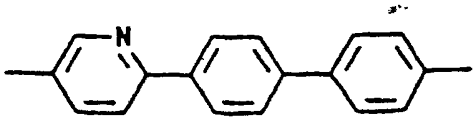 , 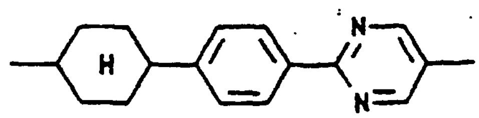 ,

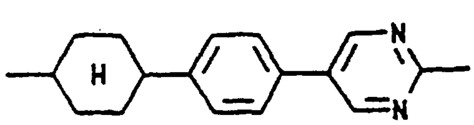 , 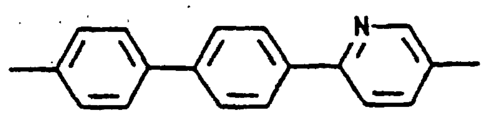 ,

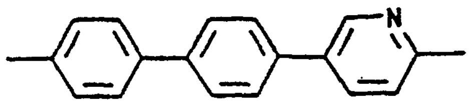 , 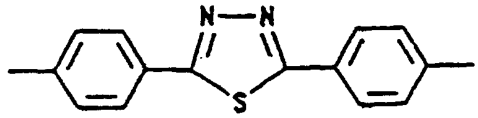 ,

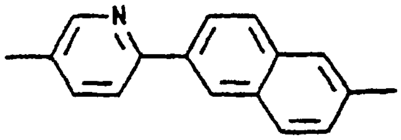 , 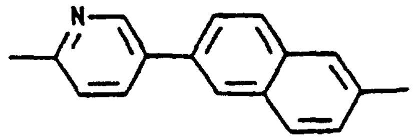 ,

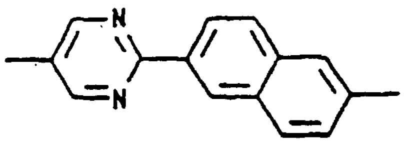 , 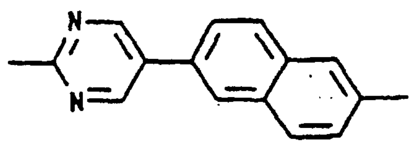 ,

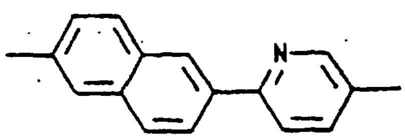 , 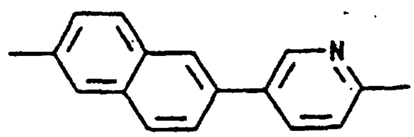 ,

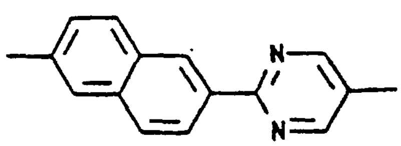 , 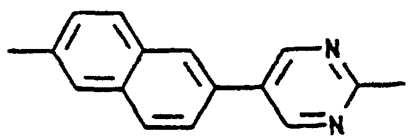 ,

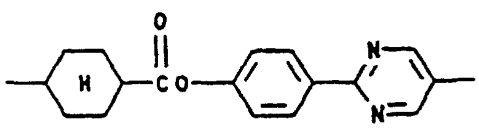 , 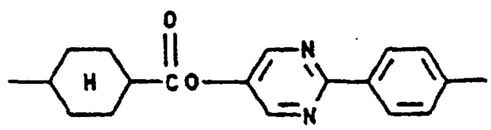 ,

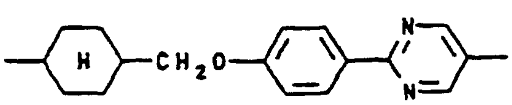 , 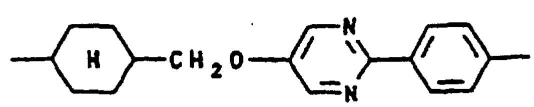

**5.** Mélange de cristaux liquides **caractérisé par** une concentration de 0,01 à 60% en poids d'au moins un oxyde d'oxiraneméthyle de formule (I),

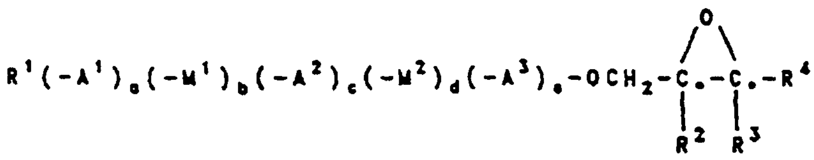
(I)

dans laquelle les symboles et indices ont la signification suivante :

| | |
|---|---|
| * | est un centre chiral |
| $R^1$ | est un groupe alkyle à chaîne linéaire ou ramifiée ayant de 1 à 16 atomes de carbone (avec ou sans atome de carbone asymétrique), dans lequel un ou plusieurs groupes $-CH_2-$ non voisins peuvent aussi être remplacés par -O-, -S-, -CO-, -CO-O-, -O-CO-, -CO-S-, -S-CO-, -O-CO-O-, -CH=CH-, -C≡C-, ou Δ et/ ou un ou plusieurs atomes d'hydrogène du groupe alkyle peuvent être substitués par F, Cl, Br ou CN, ou un des groupes chiraux suivants : |

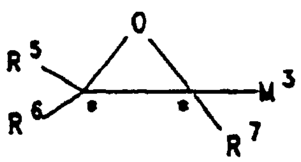

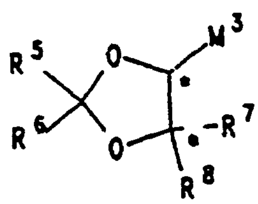

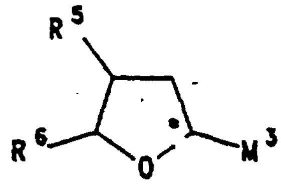

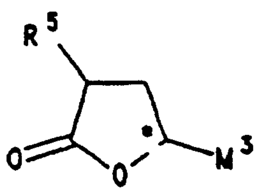

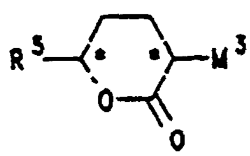

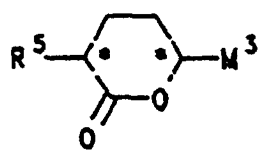

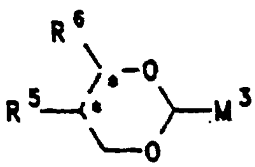

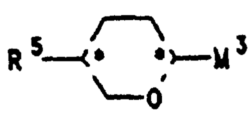

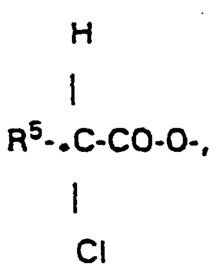

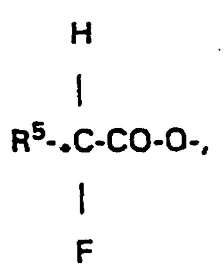

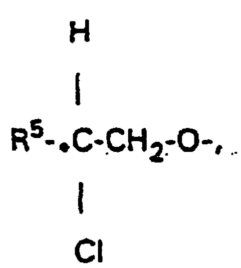

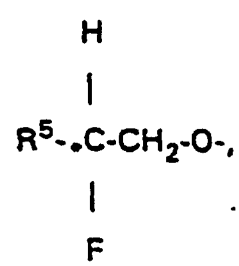

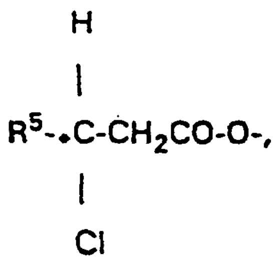 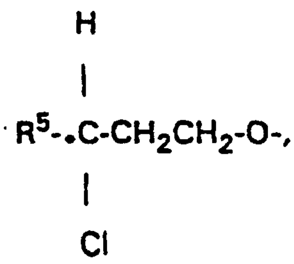

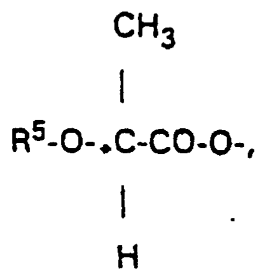 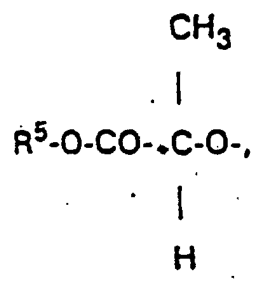

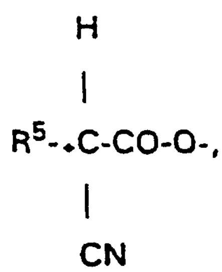 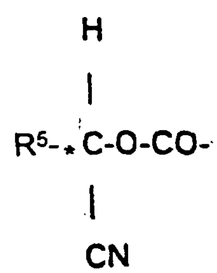

| | |
|---|---|
| $R^2$, $R^3$, $R^4$, $R^5$, $R^6$, $R^7$, $R^8$ | sont indépendamment les uns des autres H ou un groupe alkyle à chaîne linéaire ou ramifiée ayant de 1 à 16 atomes de carbone, ou $R^5$ et $R^6$ peuvent être ensemble aussi $-(CH_2)_4-$ ou $-(CH_2)_5-$ lorsqu'ils sont liés à un système dioxolane comme substituants, |
| $A^1$, $A^2$, $A^3$ | sont, identiques ou différents, un groupe 1,4-phénylène, dans lequel un ou deux atomes H peuvent être remplacés par F, Cl et/ou CN, pyrazin-2,5-diyle, pyridazin-3,6-diyle, pyridin-2,5-diyle, pyrimidin-2,5-diyle, trans-1,4-cyclohexylène, dans lequel un ou deux atomes H peuvent être remplacés par CN et/ou $CH_3$, (1,3,4)-thiadiazol-2,5-diyle, 1,3-dioxan-2,5-diyle, 1,3-dithian-2,5-diyle, 1,3-thiazol-2,4-diyle, 1,3-thiazol-2,5-diyle, thiophèn-2,4-diyle, thiophèn-2,5-diyle, pipérazin-1,4-diyle, pipérazin-2,5-diyle, naphtalèn-2,6-diyle, bicyclo-[2.2.2]octan-1,4-diyle ou 1,3-dioxaborinan-2,5-diyle, $A^1$ et $A^2$ et $A^3$ ne pouvant pas être en même temps un groupe 1,4-phénylène non substitué, lorsque $M^1$ ou $M^2$ est -COO-, |
| $M^1$, $M^2$ | sont, identiques ou différents, -O-, -S-, -CO-, -CO-O-, -O-CO-, -CO-S-, -S-CO-, -O-CO-O-, $-CH_2-O-$, $-O-CH_2-$, $-CH_2-CH_2-$, -CH=CH- ou $-C\equiv C-$, |
| $M^3$ | est $-CH_2-O-$, $-O-CH_2-$, -CO-O-, -O-CO- ou une liaison simple, |
| a, b, c, d, e | sont zéro ou un, à la condition que la somme a + b + e soit 1, 2 ou 3, |

à l'exception des composés, dans lesquels le groupe $(-A^1)_a(-M^1)_b(-A^2)_c(-M^2)_d(-A^3)_e-$ a la signification suivante :

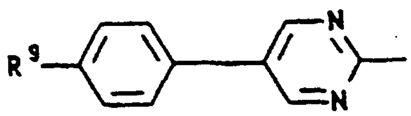 ,

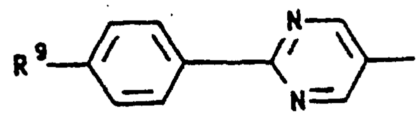 ,

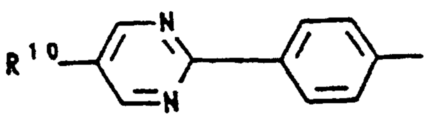 ,

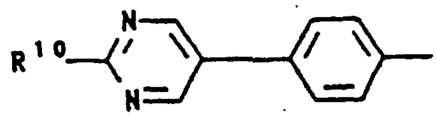 ,

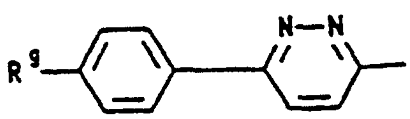 ,

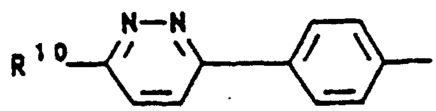 ,

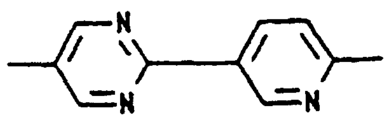 ,

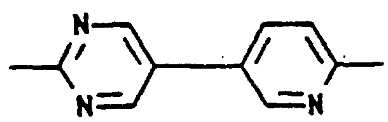 ,

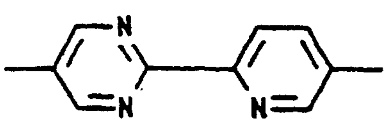 ,

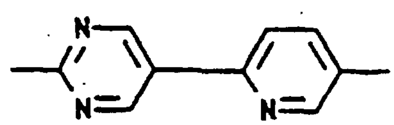 ,

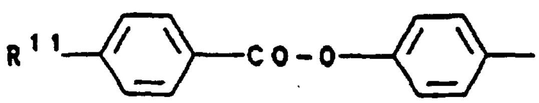

dans lesquelles

$R^9$ représente un groupe alcényle en $C_3$ à $C_{12}$ à chaîne linéaire ou ramifiée ayant une liaison double terminale, dans lequel un ou deux groupes $CH_2$ non voisins peuvent être remplacés par des atomes O et/ou S,

$R^{10}$ représente un groupe alkyle à chaîne linéaire ou ramifiée ayant de 1 à 12 atomes de carbone, dans lequel un ou deux groupes $CH_2$ non voisins peuvent être remplacés par O et/ou S, et

$R^{11}$ représente un groupe alcoxy ayant de 5 à 12 atomes de carbone

ou $R^1$ représente

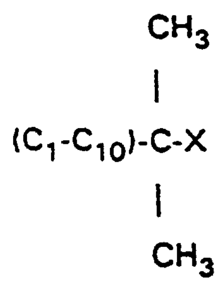

dans laquelle

X représente un groupe alkylène à chaîne linéaire ou ramifiée ayant de 1 à 16 atomes de carbone, dans lequel un ou deux groupes $-CH_2-$ non voisins peuvent aussi être remplacés par -O-, -S-, -O-CO-, -CO-O-, CO ou -O-CO-O-.

**6.** Mélanges de cristaux liquides, **caractérisés par** une concentration de 0,01 à 60% en poids d'au moins un oxyde d'oxiranylméthyle selon la revendication 1.

**7.** Mélange ferroélectrique de cristaux liquides, **caractérisé par** une concentration comprise entre 0,01 et 60% en poids d'au moins un oxyde d'oxiranylméthyle selon la revendication 2.

**8.** Mélange ferroélectrique de cristaux liquides, **caractérisé par** une concentration comprise entre 0,01 et 60% en poids d'au moins un oxyde d'oxiranylméthyle selon la revendication 3.

**9.** Mélange ferroélectrique de cristaux liquides, **caractérisé par** une concentration comprise entre 0,01 et 60% en poids d'au moins un oxyde d'oxiranylméthyle selon la revendication 4.

**10.** Composant électrooptiques ou entièrement optiques, qui contiennent un mélange de cristaux liquides selon la revendication 5, 6, 7 ou 8.